# EUROPEAN PATENT SPECIFICATION

(11) **EP 1 723 140 B1**
(45) Date of publication and mention of the grant of the patent: **04.11.2009**
(21) Application number: 05779890.2
(22) Date of filing: 18.02.2005
(51) Int. Cl.: C07D 405/10, C07D 211/02, C07D 211/26, C07F 7/10, C07D 213/38, C07D 213/53

(54) **PROCESS FOR THE PREPARATION OF TRYPTASE INHIBITORS**
VERFAHREN ZUR HERSTELLUNG VON TRYPTASE-INHIBITOREN
PROCESSUS POUR LA PREPARATION D'INHIBITEURS DE TRYPTASE

(30) Priority: 02.03.2004 EP 04004799; 06.07.2004 US 585745 P
(43) Date of publication of application: 22.11.2006
(73) Proprietor: Aventis Pharmaceuticals Inc., Bridgewater, New Jersey 08807 (US)
(72) Inventor: GRAF, Claus-Dieter, 65926 Frankfurt am Main (DE); TAPPERTZHOFEN, Christoph, D-65926 Frankfurt am Main (DE); SLEDESKI, Adam, W., Belle Mead, NJ 08502 (US)
(74) Representative: Fischer, Hans-Jürgen
(86) International application number: PCT/EP2005/001676
(87) International publication number: WO 2005/095385

(56) References cited:
- EP-A- 0 401 166
- EP-A- 0 540 356
- WO-A-01/90101
- WO-A-99/18053
- WO-A-2004/035744
- WO-A-2004/060884
- DJURIC, S.; VENIT, J.; MAGNUS, P.: "Silicon In Synthesis: Stable Adducts-A New Primary Amine Protecting Group: Alkylation of Ethyl Glycinate" TETRAHEDRON LETTERS, vol. 22, no. 19, 1981, pages 1787-1790, XP002331270

## Description

This invention is directed to processes for the preparation of compounds which are useful as tryptase inhibitors, to intermediates useful in the preparation of such compounds, to processes for the preparation of such intermediates, and to the use of such intermediates for the preparation of such compounds.

### Background of the invention

Mast cell mediated inflammatory conditions, in particular asthma, are a growing public health concern. Asthma is frequently characterized by progressive development of hyper-responsiveness of the trachea and bronchi to both immunospecific allergens and generalized chemical or physical stimuli, which lead to the onset of chronic inflammation. Leukocytes containing IgE receptors, notably mast cells and basophils, are present in the epithelium and underlying smooth muscle tissues of bronchi. These leukocytes initially become activated by the binding of specific inhaled antigens to the IgE receptors and then release a number of chemical mediators. For example, degranulation of mast cells leads to the release of proteoglycans, peroxidase, arylsulfatase B, chymase, and tryptase, which results in bronchiole constriction.

Tryptase is stored in the mast cell secretory granules and is the major secretory protease of human mast cells. Tryptase has been implicated in a variety of biological processes, including degradation of vasodilating and bronchorelaxing neuropeptides (Caughey et al., J. Pharmacol. Exp. Ther., 1988, 244, pages 133-137; Franconi et al., J. Pharmacol. Exp. Ther., 1988, 248, pages 947-951; and Tam et al., Am. J. Respir. Cell Mol. Biol., 1990, 3, pages 27-32) and modulation of bronchial responsiveness to histamine (Sekizawa et al., J. Clin. Invest., 1989, 83, pages 175-179).

As a result, tryptase inhibitors are regarded as useful as anti-inflammatory agents (K. Rice, P.A. Sprengler, Current Opinion in Drug Discovery and Development, 1999, 2, pages 463-474) particularly in the treatment of chronic asthma (M.Q. Zhang, H. Timmerman, Mediators Inflamm., 1997, 112, pages 311-317), and may also be useful in treating or preventing allergic rhinitis (S. J. Wilson et al., Clin. Exp. Allergy, 1998, 28, pages 220-227), inflammatory bowel disease (S.C. Bischoff et al., Histopathology, 1996, 28, pages 1-13), psoriasis (A. Naukkarinen et al., Arch. Dermatol. Res., 1993, 285, pages 341-346), conjunctivitis (A.A.Irani et al., J. Allergy Clin. Immunol., 1990, 86, pages 34-40), atopic dermatitis (A. Jarvikallio et al., Br. J. Dermatol., 1997, 136, pages 871-877), rheumatoid arthritis (L.C Tetlow et al., Ann. Rheum. Dis., 1998, 54, pages 549-555), osteoarthritis (M.G. Buckley et al., J. Pathol., 1998, 186, pages 67-74), gouty arthritis, rheumatoid spondylitis, and diseases of joint cartilage destruction.

In addition, tryptase has been shown to be a potent mitogen for fibroblasts, suggesting its involvement in the pulmonary fibrosis in asthma and interstitial lung diseases (Ruoss et al., J. Clin. Invest., 1991, 88, pages 493-499).

Therefore, tryptase inhibitors are regarded as useful in treating or preventing fibrotic conditions (J.A. Cairns, A.F. Walls, J. Clin. Invest., 1997, 99, pages 1313-1321) for example, fibrosis, scleroderma, pulmonary fibrosis, liver cirrhosis, myocardial fibrosis, neurofibromas and hypertrophic scars.

Additionally, tryptase inhibitors are regarded as useful in treating or preventing myocardial infarction, stroke, angina and other consequences of atherosclerotic plaque rupture (M. Jeziorska et al., J. Pathol., 1997, 182, pages 115-122).

Tryptase has also been discovered to activate prostromelysin that in turn activates collagenase, thereby initiating the destruction of cartilage and periodontal connective tissue, respectively.

Therefore, tryptase inhibitors are regarded as useful in the treatment or prevention of arthritis, periodontal disease, diabetic retinopathy, and tumor growth (W.J. Beil et al., Exp. Hematol., 1998, 26, pages 158-169). Also, tryptase inhibitors are regarded as useful in the treatment of anaphylaxis (L.B. Schwarz et al., J. Clin. Invest., 1995, 96, pages 2702-2710), multiple sclerosis (M. Steinhoff et al., Nat. Med. (N. Y.), 2000, 6, pages 151-158), peptic ulcers and syncytial viral infections. 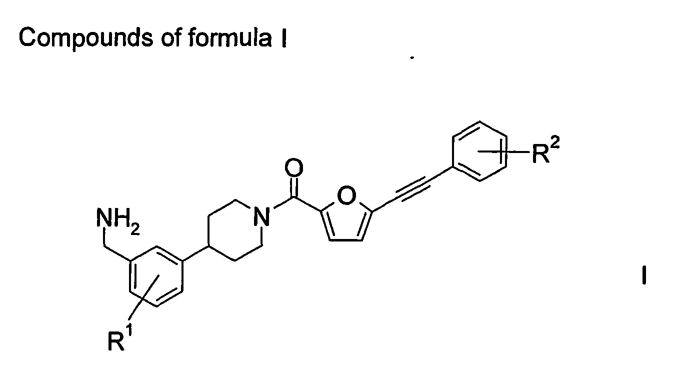 have been disclosed as being useful as tryptase inhibitors, for example, in WO 01/90101 and WO 2004/060884 (international patent application PCT/US2003/040653). Processes for preparing compounds of formula I are also disclosed therein, however, they suffer from substantial drawbacks and hazards, in particular when it is desired to prepare a compound of the formula I on a large scale. The present invention provides advantageous processes which are useful in the preparation of compounds of the formula I and allow to overcome the drawbacks and avoid the hazards of the prior art processes.

### Summary of the invention

This invention is directed to processes for the preparation of compounds of formula I, 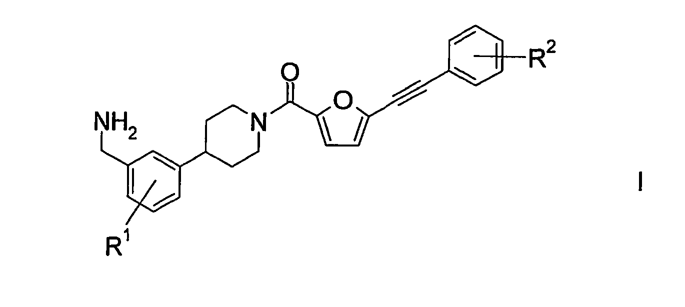 wherein
R¹ is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, (C₁-C₈)-alkoxy, (C₃-C₁₀)-cycloalkoxy, (C₆-C₁₄)-aryloxy, di((C₁-C₈)-alkyl)amino, di((C₃-C₁₀)-cycloalkyl)amino or di((C₆-C₁₄)-aryl)amino; and
R² is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, 5-membered to 10-membered heteroaryl comprising 1, 2 or 3 identical or different ring heteroatoms chosen from nitrogen, oxygen and sulfur, (C₁-C₈)-alkoxy, (C₃-C₁₀)-cycloalkoxy, (C₆-C₁₄)-aryloxy, 5-membered to 10-membered heteroaryloxy comprising 1, 2, or 3 identical or different ring heteroatoms chosen from nitrogen, oxygen and sulfur, di((C₁-C₈)-alkyl)amino, di-((C₃-C₁₀)-cydoalkyl)amino or di((C₆-C₁₄)-aryl)amino;
and their salts.

This invention also relates to intermediates useful in the preparation of compounds of formula I, to processes for the preparation of such intermediates, and to the use of such intermediates for the preparation of such compounds.

### Detailed description of the invention

In a first aspect, the present invention provides a process for the preparation of a compound of the formula I, 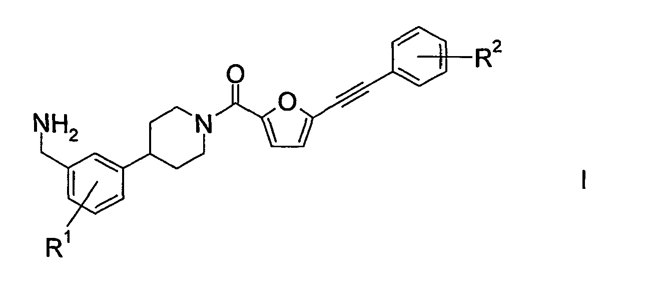 wherein
R¹ is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, (C₁-C₈)-alkoxy, (C₃-C₁₀)-cycloalkoxy, (C₆-C₁₄)-aryloxy, di((C₁-C₈)-alkyl)amino, di((C₃-C₁₀)-cycloalkyl)amino or di((C₆-C₁₄)-aryl)amino; and
R² is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, 5-membered to 10-membered heteroaryl comprising 1, 2 or 3 identical or different ring heteroatoms chosen from nitrogen, oxygen and sulfur, (C₁-C₈)-alkoxy, (C₃-C₁₀)-cycloalkoxy, (C₆-C₁₄)-aryloxy, 5-membered to 10-membered heteroaryloxy comprising 1, 2, or 3 identical or different ring heteroatoms chosen from nitrogen, oxygen and sulfur, di((C₁-C₈)-alkyl)amino, di-((C₃-C₁₀)-cycloalkyl)amino or di((C₆-C₁₄)-aryl)amino;
or its salt,
comprising the steps of
a) treating a phenylethynylsilane compound of the formula II, 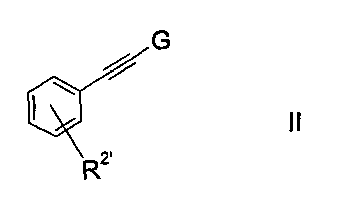 wherein G is trimethylsilyl, triethylsilyl, tri-isopropylsilyl, or dimethyl-tert-butylsilyl, and
   R^{2'} is R² as defined above or is a protected derivative thereof or precursor moiety thereto, in a solvent, such as an aliphatic alcohol or a mixture of an aliphatic alcohol and an ether, for example methanol or a mixture of methanol and tetrahydrofuran, with an alkali metal hydroxide, carbonate or alcoholate or an alkaline earth metal alcoholate, to form a solution of a phenylethyne of the formula III; 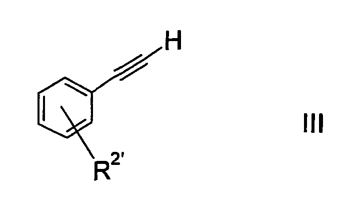
   and
b) combining the resulting solution of the phenylethyne with a compound of the formula IV, 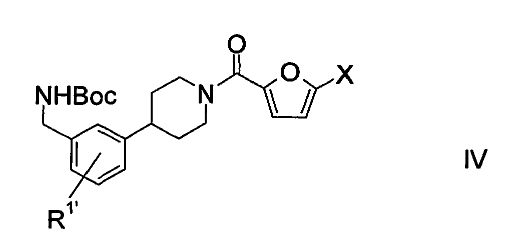 wherein X is bromo or iodo, R^{1'} is R¹ as defined above or is a protected derivative thereof or precursor moiety thereto, and Boc is tert-butoxycarbonyl, in the presence of a homogeneous palladium catalyst, a cuprous salt, and a hindered amine in a solvent, such as an ether or a mixture of an ether and an aliphatic alcohol, for example tetrahydrofuran or a mixture of tetrahydrofuran and methanol, to form a protected compound of the formula V, 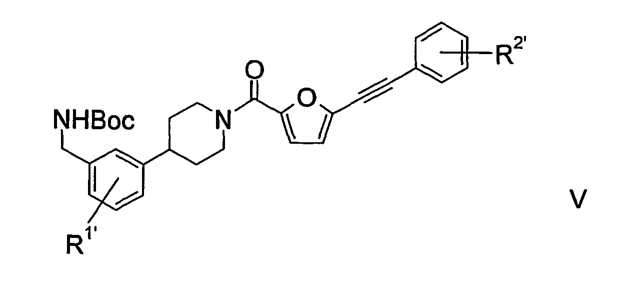 wherein R^{1'} and R^{2'} are R¹ and R², respectively, as defined above or are a protected derivative thereof or precursor moiety thereto, and Boc is tert-butoxycarbonyl, or its salt.

Particular embodiments of this first aspect of the present invention are those wherein G is trimethylsilyl, and/or X is bromo, and/or the homogeneous palladium catalyst is oalladium(II)bis(triphenylphosphine) chloride, and/or the cuprous salt is copper(I)iodide, and/or the hindered amine is triethylamine.

A special embodiment of this first aspect provides for further removal of the tert-butoxycarbonyl group from the protected compound of the formula V and, if applicable, removal of any other protective groups and/or conversion of precursor moieties that may be present in R^{1'} and/or R^{2'} in a compound of the formula V by standard procedures, in a solvent, for example an aliphatic alcohol or an ether, to provide a compound of the formula I or its salt. Another special embodiment of this first aspect provides for removal of the tert-butoxycarbonyl group from the protected compound of the formula V, in particular from a compound in which R^{1'} and R^{2'} are R¹ and R², respectively, in the presence of an acid, in particular a physiologically acceptable acid, for example methanesulfonic acid, in a solvent, for example an aliphatic alcohol or an ether such as isopropanol, to provide a compound of the formula I or its salt.

A second aspect of the present invention provides a process for the preparation of 4-[3-(aminomethyl)phenyl]-1-[5-(2-fluorophenylethynyl)-2-furanoyl]piperidine, i.e., the compound of the formula la, 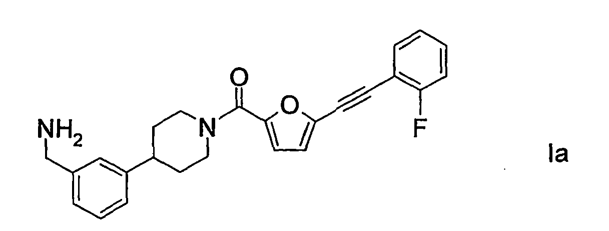 or its salt,
comprising the steps of
a) treating a 2-fluorophenylethynylsilyl compound of the formula IIa, 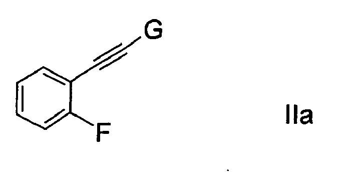 wherein G is trimethylsilyl, triethylsilyl, tri-isopropylsilyl, or dimethyl-tert-butylsilyl, in a solvent, such as an aliphatic alcohol or a mixture of an aliphatic alcohol and an ether, for example methanol or a mixture of methanol and tetrahydrofuran, with an alkali metal hydroxide, carbonate or alcoholate or an alkaline earth metal alcoholate, for example with potassium carbonate in methanol, to form a solution of 2-fluorophenylethyne; and
b) combining the resulting solution of 2-fluorophenylethyne with a compound of the formula IVa, 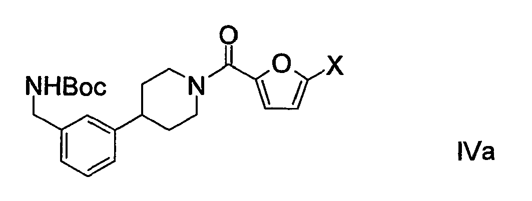 wherein X is bromo or iodo, and Boc is tert-butoxycarbonyl, in the presence of a homogeneous palladium catalyst, a cuprous salt, and a hindered amine in a solvent, such as an ether or a mixture of an ether and an aliphatic alcohol, for example tetrahydrofuran or a mixture of tetrahydrofuran and methanol, to form 4-[3-(tert-butoxycarbonylaminomethyl)phenyl]-1-[5-(2-fluorophenylethynyl)-2-furanoyl]piperidine, i.e., the compound of the formula Va, 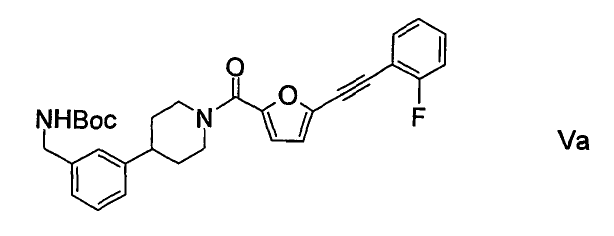 wherein Boc is tert-butoxycarbonyl.

Particular embodiments of this second aspect of the present invention are those wherein G is trimethylsilyl, and/or X is bromo, and/or the homogeneous palladium catalyst is palladium(II)bis(triphenylphosphine) chloride, and/or the cuprous salt is copper(I) iodide, and/or the hindered amine is triethylamine.

A special embodiment of this second aspect provides for further removal of the tert-butoxycarbonyl group from 4-[3-(tert-butoxycarbonylaminomethyl)phenyl]-1-[5-(2-fluorophenylethynyl)-2-furanoyl]piperidine of the formula Va in a solvent to provide the compound of the formula la or its salt. Another special embodiment of this second aspect provides for removal of the tert-butoxycarbonyl group from 4-[3-(tert-butoxycarbonylaminomethyl)phenyl]-1-[5-(2-fluorophenylethynyl)-2-furanoyl]piperidine of the formula Va in the presence of an acid, in particular a physiologically acceptable acid, for example methanesulfonic acid, in a solvent, for example an aliphatic alcohol or an ether such as isopropanol, to provide the compound of the formula la or its salt, for example the methanesulfonic acid salt of 4-[3-(aminomethyl)phenyl]-1-[5-(2-fluorophenylethynyl)-2-furanoyl]piperidine.

A third aspect of the present invention provides a process for the preparation of a compound of the formula IV, 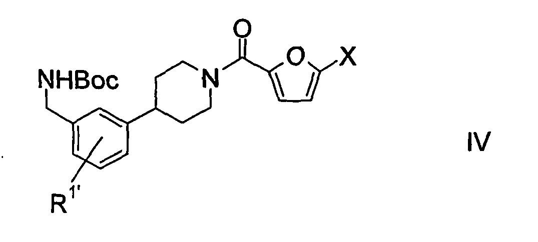 wherein
R^{1'} is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, (C₁-C₈)-alkoxy, (C₃-C₁₀)-cycloalkoxy, (C₆-C₁₄)-aryloxy, di((C₁-C₈)-alkyl)amino, di((C₃-C₁₀)-cycloalkyl)amino or di((C₆-C₁₄)-aryl)amino, or a protected derivative thereof or precursor moiety thereto; and
X is bromo or iodo,
comprising the steps of
a) activating 5-bromo-2-furoic acid or 5-iodo-2-furoic acid, for example by converting it into an activated acid derivative which may be prepared in situ or may be isolated, such as an acid halide, in particular an acid chloride, an active ester or an anhydride or mixed anhydride, for example by treatment with a halogenating agent such as thionyl chloride or oxalyl chloride in a solvent, such as a hydrocarbon or a chlorinated hydrocarbon, for example toluene, at a temperature of from about 0°C to about 120°C, for example at reflux temperature of the solvent, or by treatment with an alkyl chlorocarbonate such as ethyl chlorocarbonate or isobutyl chlorocarbonate and a base such as, for example, triethylamine in a solvent, for example a halogenated hydrocarbon or an ether such as tetrahydrofuran, at a temperature of from about -10° to about 30°C, or by treating it with a condensing agent or coupling agent as is customarily used for the formation of amide bonds in peptide chemistry, for example, such as a carbodiimide like dicyclohexylcarbodiimide or diisopropylcarbodiimide or a carbonyldiazole like carbonyldiimidazole or O-((cyano(ethoxycarbonyl)methylene)-amino)-N,N,N',N'-tetramethyluronium tetrafluoroborate (TOTU) or propylphosphonic anhydride, optionally in the presence of an auxiliary agent such as 1-hydroxybenzotriazole, for example, in a solvent, for example a halogenated hydrocarbon or an ether or an amide such as tetrahydrofuran or dimethylformamide, at a temperature of from about 0°C to about 30°C, to provide an activated form of 5-bromo-2-furoic acid or 5-iodo-2-furoic acid, for example 5-bromo-2-furoyl chloride or 5-iodo-2-furoyl chloride, respectively, which activated form may be obtained and subsequently employed in form of a solution, for example a solution in toluene; and
b) combining the activated form of 5-bromo-2-furoic acid or 5-iodo-2-furoic acid with a compound of the formula VI, 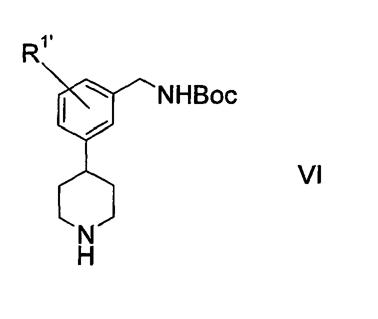 wherein R^{1'} is as defined for formula IV and Boc is tert-butoxycarbonyl, in a solvent, for example a hydrocarbon, a chlorinated hydrocarbon or an ether such as toluene or dichloromethane, optionally in the presence of a base, for example a tertiary amine like triethylamine.

A further embodiment of this third aspect provides a process for the preparation of a compound of the formula IV wherein X is bromo, i.e. a compound of the formula IVb, 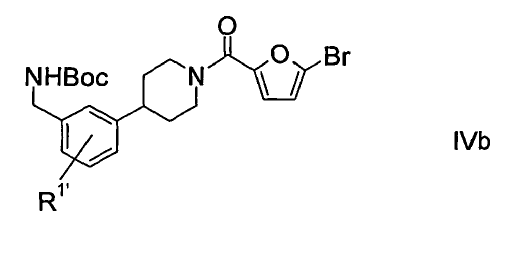 wherein
R^{1'} is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, (C₁-C₈)-alkoxy, (C₃-C₁₀)-cycloalkoxy, (C₆-C₁₄)-aryloxy, di((C₁-C₈)-alkyl)amino, di((C₃-C₁₀)-cycloalkyl)amino or di((C₆-C₁₄)-aryl)amino, or a protected derivative thereof or precursor moiety thereto; and
Boc is tert-butoxycarbonyl,
comprising the steps of
a) activating 5-bromo-2-furoic acid to provide an activated form of 5-bromo-2-furoic acid; and
b) combining the activated form of 5-bromo-2-furoic acid with a compound of the formula VI in a solvent, optionally in the presence of base,
where all before-mentioned explanations on the third aspect of the present invention also apply to this further embodiment. For example, 5-bromo-2-furoic acid can be activated by treatment with thionyl chloride in refluxing toluene to provide a solution of 5-bromo-2-furoyl chloride in toluene which is then reacted with a compound of the formula VI in the presence of base such as a tertiary amine in toluene or dichloromethane.

A fourth aspect of the present invention provides a process for the preparation of a compound of the formula IV in which R^{1'} is hydrogen, i.e. a compound of the formula IVa, 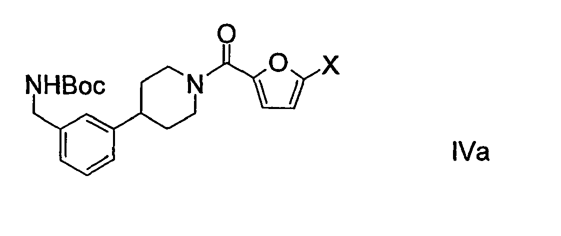
wherein X is bromo or iodo; and
Boc is tert-butoxycarbonyl,
comprising the steps of
a) activating 5-bromo-2-furoic acid or 5-iodo-2-furoic acid, for example by converting it into an activated acid derivative which may be prepared in situ or may be isolated, such as an acid halide, in particular an acid chloride, an active ester or an anhydride or mixed anhydride, for example by treatment with an halogenating agent such as thionyl chloride or oxalyl chloride in a solvent, such as a hydrocarbon or a chlorinated hydrocarbon, for example toluene, at a temperature of from about 0°C to about 120°C, for example at about reflux temperature of the solvent, or by treatment with an alkyl chlorocarbonate such as ethyl chlorocarbonate or isobutyl chlorocarbonate and a base such as, for example, triethylamine in a solvent, for example a halogenated hydrocarbon or an ether such as tetrahydrofuran, at a temperature of from about -10° to about 30°C, or by treating it with a condensing agent or coupling agent as is customarily used for the formation of amide bonds in peptide chemistry, for example, such as a carbodiimide like dicyclohexylcarbodiimide or diisopropylcarbodiimide or a carbonyldiazole like carbonyldiimidazole or O-((cyano(ethoxycarbonyl)methylene)amino)-N,N,N',N'-tetramethyluronium tetrafluoroborate (TOTU) or propylphosphonic anhydride, optionally in the presence of an auxiliary agent such as 1-hydroxybenzotriazole, for example, in an solvent, for example a halogenated hydrocarbon or an ether or an amide such as tetrahydrofuran or dimethylformamide, at a temperature of from about 0°C to about 30°C, to provide an activated form of 5-bromo-2-furoic acid or 5-iodo-2-furoic acid, for example 5-bromo-2-furoyl chloride or 5-iodo-2-furoyl chloride, respectively, which activated form may be obtained and subsequently employed in form of a solution, for example a solution in toluene; and
b) combining the activated form of 5-bromo-2-furoic acid or 5-iodo-2-furoic acid with a compound of the formula VIa, 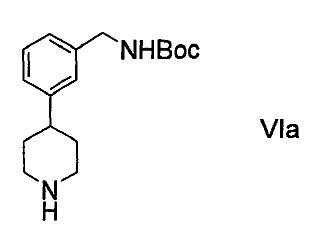 wherein Boc is tert-butoxycarbonyl, in a solvent, for example a hydrocarbon, a chlorinated hydrocarbon or an ether such as toluene or dichloromethane, optionally in the presence of a base, for example a tertiary amine like triethylamine.

A special embodiment of this fourth aspect provides a process for the preparation of a compound of the formula IVa in which X is bromo, i.e. the compound of the formula IVc, 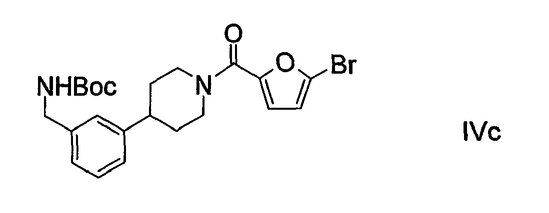 wherein Boc is tert-butoxycarbonyl,
comprising the steps of
a) activating 5-bromo-2-furoic acid to provide an activated form of 5-bromo-furoic acid, and
b) combining the activated form of 5-bromo-2-furoic acid with a compound of the formula VIa 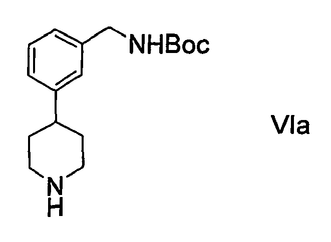
wherein Boc is tert-butoxycarbonyl, in a solvent, optionally in the presence of base,
where all before-mentioned explanations on the fourth aspect of the present invention also apply to this special embodiment. For example, 5-bromo-2-furoic acid can be activated by treatment with thionyl chloride in refluxing toluene to provide 5-bromo-2-furoyl chloride, preferably in form of a solution in toluene, which is then reacted with the compound of the formula VIa in the presence of base such as a tertiary amine in toluene or dichloromethane.

A fifth aspect of the present invention provides a process for the preparation of a compound of the formula VI 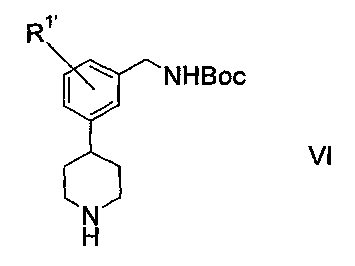
wherein R^{1'} is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, (C₁-C₈)-alkoxy, (C₃-C₁₀)-cycloalkoxy, (C₆-C₁₄)-aryloxy, di((C₁-C₈)-alkyl)amino, di((C₃-C₁₀)-cycloalkyl)amino or di((C₆-C₁₄)-aryl)amino, or a protected derivative thereof or precursor moiety thereto; and
Boc is tert-butoxycarbonyl, or its salt,
comprising the steps of
a) treating a salt of a 3-halobenzylamine of the formula VII, 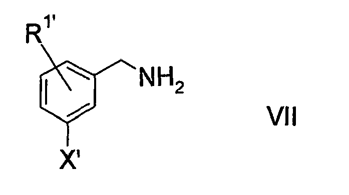 with 1,2-bis(chlorodimethylsilyl)ethane in a solvent, for example a halogenated hydrocarbon such as dichloromethane, in the presence of a base, for example a tertiary amine like triethylamine, to provide a 1-(3-halobenzyl)-2,2,5,5-tetramethyl-1-aza-2,5-disilacyclopentane of the formula VIII; 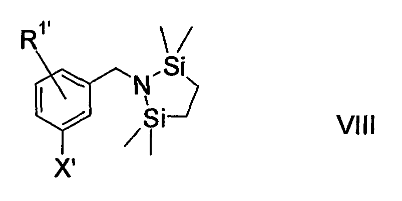
b) treating the 1-(3-halobenzyl)-2,2,5,5-tetramethyl-1-aza-2,5-disilacyclopentane of the formula VIII in a solvent, such as an ether, for example tetrahydrofuran, with an alkyllithium compound, for example n-butyllithium, and 1-benzyl-4-piperidone at a temperature of from about -80°C to about -40°C to provide an alcohol of the formula IX; 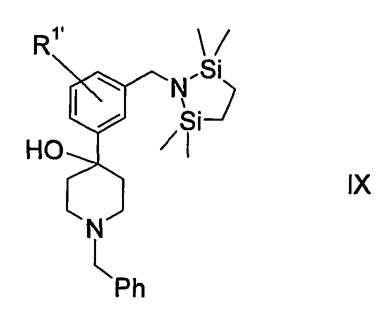
c) treating the alcohol of the formula IX with an acid, for example an inorganic acid such as phosphoric acid, in a solvent, for example a halogenated hydrocarbon such as dichloromethane, to provide a hydroxypiperidinylbenzylamine of the formula X 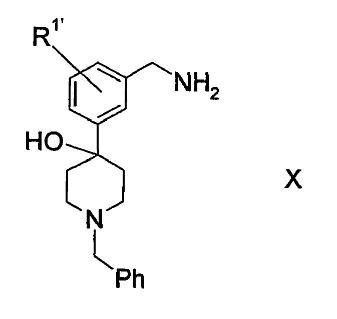 as the salt of the acid;
d) treating the hydroxypiperidinylbenzylamine of the formula X or its salt with a concentrated, non-oxidizing acid at a temperature of from about 70°C to about 150°C, followed by alkalinization to provide an olefin of the formula XI; 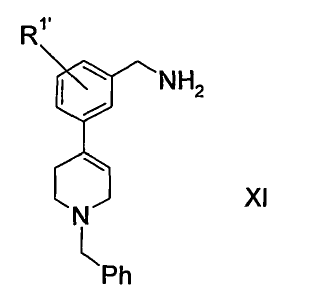
e) treating the olefin of the formula XI with di-tert-butyl dicarbonate in a solvent, for example an aliphatic alcohol such as methanol, ethyl acetate, an ether such as tetrahydrofuran, a halogenated hydrocarbon such as dichloromethane, a mixture of two or more of such solvents or a mixture of one or more of such solvents with water, in the presence of a base, for example an alkali metal hydroxide, carbonate or alcoholate or a tertiary amine, such as sodium hydroxide or triethylamine, preferably at a temperature of from about 0°C to about 40°C, to provide a protected amine of the formula XII 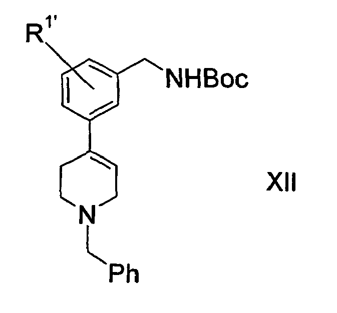
wherein Boc is tert-butoxycarbonyl, and where Ph in the formulae IX, X, XI and XII is phenyl, and where X' in the formulae VII and VIII is bromo or iodo, and where R^{1'} in the formulae VII, VIII, IX, X, XI and XII is as defined above for formula VI.

A special embodiment of this fifth aspect is that wherein X' is bromo.

Another special embodiment of this fifth aspect provides for further treating the protected amine of the formula XII, 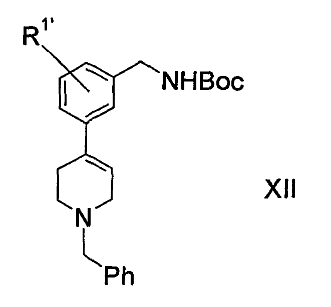 wherein Boc is tert-butoxycarbonyl, Ph is phenyl and R^{1'} is as defined above for formulae VI, with hydrogen at a pressure of from about 200 kPa to about 3000 kPa in a solvent, for example an aliphatic alcohol or ethyl acetate, in the presence of a palladium catalyst, in the presence of an organic or inorganic acid, for example acetic acid, to provide a compound of the formula VI, 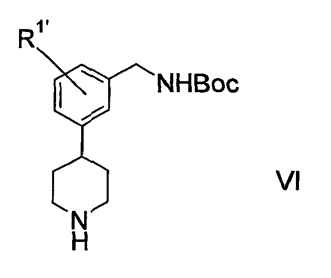 wherein R^{1'} is as defined above for formula VI and Boc is tert-butoxycarbonyl, in the form of its salt or, if desired, in the form of the free compound of the formula VI.

A sixth aspect of the present invention provides a process for the preparation of a compound of the formula VI, 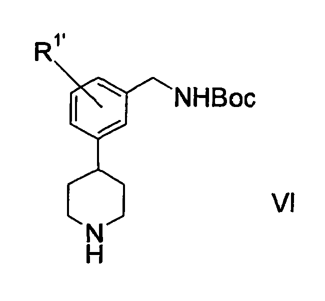
wherein R^{1'} is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, (C₁-C₈)-alkoxy, (C₃-C₁₀)-cycloalkoxy, (C₆-C₁₄)-aryloxy, di((C₁-C₈)-alkyl)amino, di((C₃-C₁₀)-cycloalkyl)amino or di((C₆-C₁₄)-aryl)amino, or a protected derivative thereof or precursor moiety thereto; and
Boc is tert-butoxycarbonyl,
or its salt,
comprising the steps of
a) treating a 3-(4-pyridyl)benzaldehyde of the formula XIII, 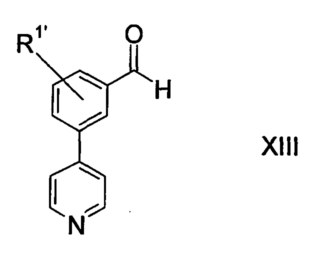 with hydroxylamine or a salt of hydroxylamine in a solvent, for example an aliphatic alcohol, optionally in the presence of base, at a temperature of from about 0°C to about 40°C to provide an oxime of the formula XIV or its salt; 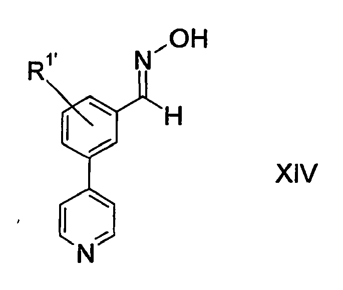
b) treating the oxime of the formula XIV or its salt with hydrogen at a pressure of from about 300 kPa to about 1500 kPa in a solvent, for example a polar organic solvent such as an aliphatic alcohol, for example methanol, in the presence of a palladium catalyst, at a temperature of from about 20°C to about 50°C to provide a 3-(4-pyridyl)benzylamine of the formula XV or its salt; 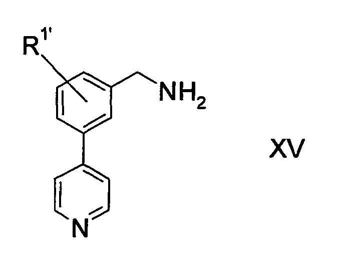
c) treating the 3-(4-pyridyl)benzylamine of the formula XV or its salt with di-tert-butyl dicarbonate in a solvent, for example an aliphatic alcohol such as methanol, ethyl acetate, an ether such as tetrahydrofuran, a halogenated hydrocarbon such as dichloromethane, a mixture of two or more of such solvents or a mixture of one or more of such solvents with water, in the presence of a base, for example an alkali metal hydroxide, carbonate or alcoholate or a tertiary amine, such as sodium hydroxide or triethylamine, preferably at a temperature of from about 0°C to about 40°C, to provide a Boc-protected 3-(4-pyridyl)benzylamine of the formula XVI, 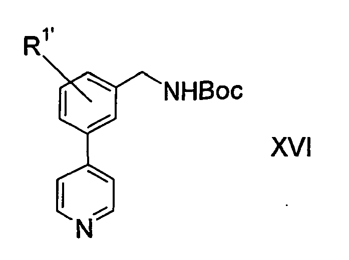 wherein Boc is tert-butoxycarbonyl; and
d) treating the Boc-protected 3-(4-pyridyl)benzylamine of the formula XVI with hydrogen in a solvent, for example an aliphatic alcohol such as ethanol, at a pressure of from about 2000 kPa to about 6000 kPa, in the presence of a platinum catalyst, in the presence of an acid such as, for example, hydrogen chloride or sulfuric acid, to provide a compound of the formula VI or its salt;
where R^{1'} in the formulae XIII, XIV, XV and XVI is as above defined for formula VI.

A particular embodiment of this sixth aspect provides a process for the preparation of a compound of the formula VI, 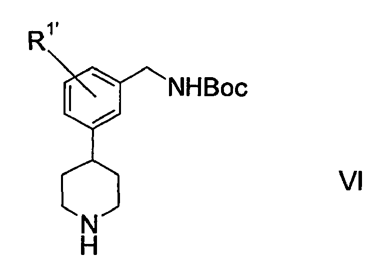
wherein R^{1'} is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, (C₁-C₈)-alkoxy, (C₃-C₁₀)-cycloalkoxy, (C₆-C₁₄)-aryloxy, di((C₁-C₈)-alkyl)amino, di((C₃-C₁₀)-cycloalkyl)amino or di((C₆-C₁₄)-aryl)amino, or a protected derivative thereof or precursor moiety thereto; and
Boc is tert-butoxycarbonyl,
or its salt,
comprising the steps of
a) treating a 3-(4-pyridyl)benzaldehyde of the formula XIII 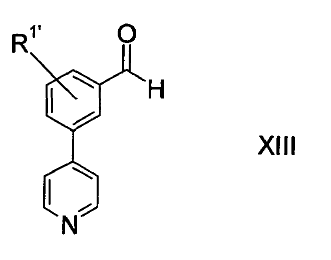 with hydroxylamine hydrochloride in a solvent, for example an aliphatic alcohol such as methanol, at a temperature of from about 0°C to about 40°C to provide an oxime hydrochloride of the formula XIVa; 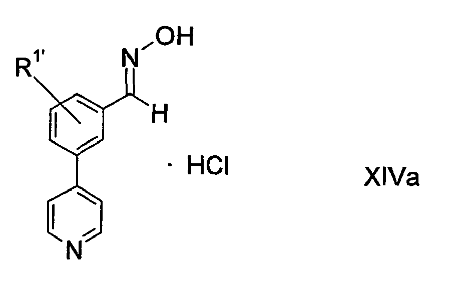
b) treating the oxime hydrochloride of the formula XIVa with hydrogen at a pressure of from about 300 kPa to about 1500 kPa in a solvent, for example a polar organic solvent such as an aliphatic alcohol, for example methanol, in the presence of a palladium catalyst, at a temperature of from about 20°C to about 50°C to provide a 3-(4-pyridyl)benzylamine hydrochloride of the formula XVa; 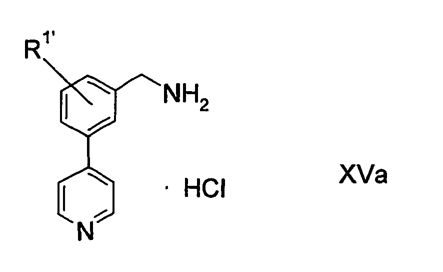
c) treating the 3-(4-pyridyl)benzylamine hydrochloride of the formula XVa with di-tert-butyl dicarbonate in a solvent, for example an aliphatic alcohol such as methanol, ethyl acetate, an ether such as tetrahydrofuran, a halogenated hydrocarbon such as dichloromethane, a mixture of two or more of such solvents or a mixture of one or more of such solvents with water, in the presence of a base, for example an alkali metal hydroxide, carbonate or alcoholate or a tertiary amine, such as sodium hydroxide or triethylamine, preferably at a temperature of from about 0°C to about 40°C, to provide a Boc-protected 3-(4-pyridyl)benzylamine of the formula XVI, 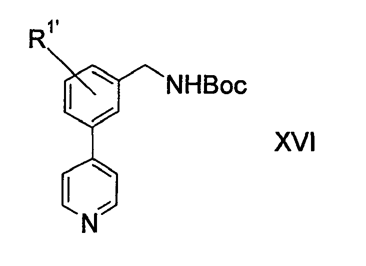 wherein Boc is tert-butoxycarbonyl; and
d) treating the Boc-protected 3-(4-pyridyl)benzylamine of the formula XVI with hydrogen in a solvent, for example an aliphatic alcohol such as ethanol, at a pressure of from about 2000 kPa to about 6000 kPa, in the presence of a platinum catalyst, in the presence of hydrogen chloride or sulfuric acid, to provide a compound of the formula VI or its salt;
where R^{1'} in the formulae XIII, XIVa, XVa and XVI is as above defined for formula VI.

A seventh aspect of the present invention provides a process for the preparation of a compound of the formula VI 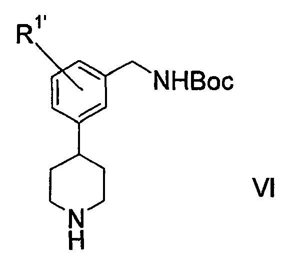
wherein R^{1'} is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, (C₁-C₈)-alkoxy, (C₃-C₁₀)-cycloalkoxy, (C₆-C₁₄)-aryloxy, di((C₁-C₈)-alkyl)amino, di((C₃-C₁₀)-cycloalkyl)amino or di((C₆-C₁₄)-aryl)amino, or a protected derivative thereof or precursor moiety thereto; and
Boc is tert-butoxycarbonyl,
or its salt,
comprising the steps of
a) treating a 3-(4-pyridyl)benzonitrile of the formula XVII, 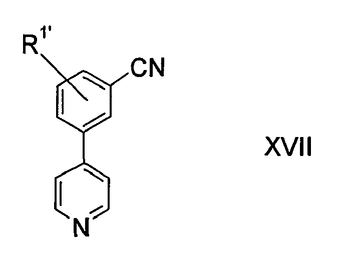 with hydrogen in the presence of a palladium catalyst at a pressure of from about 2000 kPa to about 6000 kPa in the presence of an acid, for example hydrogen chloride, or with a complex hydride such as lithium aluminium hydride, lithium borohydride or sodium borohydride, optionally in the presence of an auxiliary agent such as an acid or chlorotrimethylsifane, in a solvent, for example an aliphatic alcohol or an ether such as tetrahydrofuran, to provide a 3-(4-pyridyl)benzylamine of the formula XV or its salt; 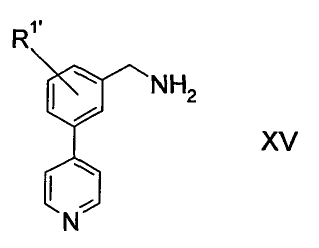
b) treating the 3-(4-pyridyl)benzylamine of the formula XV or its salt with di-tert-butyl dicarbonate in a solvent, for example an aliphatic alcohol such as methanol, ethyl acetate, an ether such as tetrahydrofuran, a halogenated hydrocarbon such as dichloromethane, a mixture of two or more of such solvents or a mixture of one or more of such solvents with water, in the presence of a base, for example an alkali metal hydroxide, carbonate or alcoholate or a tertiary amine, such as sodium hydroxide or triethylamine, preferably at a temperature of from about 0°C to about 40°C, to provide a Boc-protected 3-(4-pyridyl)benzylamine of the formula XVI, 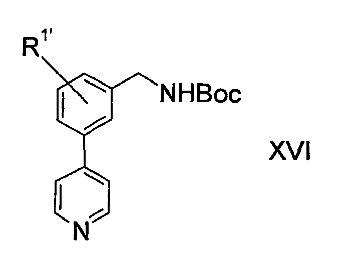 wherein Boc is tert-butoxycarbonyl; and
c) treating the Boc-protected 3-(4-pyridyl)benzylamine of the formula XVI with hydrogen in a solvent, for example an aliphatic alcohol such as ethanol, at a pressure of from about 2000 kPa to about 6000 kPa, in the presence of a platinum catalyst, in the presence of an acid such as hydrogen chloride or sulfuric acid, to provide a compound of the formula VI or its salt;
where R^{1'} in the formula XV, XVI and XVII is as above defined for formula VI.

An eighth aspect of the present invention provides a process for the preparation of 4-[3-(tert-butoxycarbonylaminomethyl)phenyl]piperidine or its salt,
comprising the steps of
a) treating 3-bromobenzylamine hydrochloride or 3-iodobenzylamine hydrochloride with 1,2-bis(chlorodimethylsilyl)ethane in a halogenated aliphatic hydrocarbon in the presence of a tertiary amine to provide the corresponding 1-(3-halobenzyl)-2,2,5,5-tetramethyl-1-aza-2,5-disilacyclopentane, where halo is bromo or iodo;
b) treating the 1-(3-halobenzyl)-2,2,5,5-tetramethyl-1-aza-2,5-disilacyclopentane in an ether with an alkyllithium compound and 1-benzyl-4-piperidone at a temperature of from about -80°C to about -40°C to provide 1-[3-(1-benzyl-4-hydroxypiperidin-4-yl)benzyl]-2,2,5,5-tetramethyl-1-aza-2,5-disilacyclopentane;
c) treating the 1-[3-(1-benzyl-4-hydroxypiperidin-4-yl)benzyl]-2,2,5,5-tetramethyl-1-aza-2,5-disilacyclopentane with an inorganic acid in a halogenated hydrocarbon, to provide 3-(1-benzyl-4-hydroxypiperidin-4-yl)benzylamine as the salt of the inorganic acid;
d) treating the 3-(1-benzyl-4-hydroxypiperidin-4-yl)benzylamine with a concentrated, non-oxidizing acid at a temperature of from about 70°C to about 150°C, followed by alkalinization to provide 3-(1-benzyl-1,2,3,6-tetrahydropyridin-4-yl)benzylamine; and
e) treating the 3-(1-benzyl-1,2,3,6-tetrahydropyridin-4-yl)benzylamine with di-tert-butyl dicarbonate in an aliphatic alcohol such as methanol, ethyl acetate, an ether such as tetrahydrofuran, a halogenated hydrocarbon such as dichloromethane, a mixture of two or more of such solvents or a mixture of one or more of such solvents with water, in the presence of an alkali metal hydroxide, carbonate or alcoholate or a tertiary amine, such as sodium hydroxide or triethylamine, preferably at a temperature of from about 0°C to about 40°C, to provide 3-(1-benzyl-1,2,3,6-tetrahydropyridin-4-yl)benzylcarbamic acid tert-butyl ester.

A special embodiment of this eighth aspect provides a process for the preparation of 4-[3-(tert-butoxycarbonylaminomethyl)phenyl]piperidine or its salt,
comprising the steps of
a) treating 3-bromobenzylamine hydrochloride with 1,2-bis(chlorodimethylsilyl)ethane in a halogenated aliphatic hydrocarbon, in the presence of a tertiary amine to provide 1-(3-bromobenzyl)-2,2,5,5-tetramethyl-1-aza-2,5-disilacyclopentane;
b) treating the 1-(3-bromobenzyl)-2,2,5,5-tetramethyl-1-aza-2,5-disilacyclopentane in an ether, with an alkyllithium compound and 1-benzyl-4-piperidone at a temperature of from about -80°C to about -40°C to provide 1-[3-(1-benzyl-4-hydroxypiperidin-4-yl)benzyl]-2,2,5,5-tetramethyl-1-aza-2,5-disilacyclopentane;
c) treating the 1-[3-(1-benzyl-4-hydroxypiperidin-4-yl)benzyl]-2,2,5,5-tetramethyl-1-aza-2,5-disilacyclopentane with an inorganic acid in a halogenated aliphatic hydrocarbon, to provide 3-(1-benzyl-4-hydroxypiperidin-4-yl)benzylamine as the salt of the inorganic acid;
d) treating the 3-(1-benzyl-4-hydroxypiperidin-4-yl)benzylamine with a concentrated, non-oxidizing acid at a temperature of from about 70°C to about 150°C, followed by alkalinization to provide 3-(1-benzyl-1,2,3,6-tetrahydropyridin-4-yl)benzylamine; and
e) treating the 3-(1-benzyl-1,2,3,6-tetrahydropyridin-4-yl)benzylamine with di-tert-butyl dicarbonate in an aliphatic alcohol such as methanol, ethyl acetate, an ether such as tetrahydrofuran, a halogenated hydrocarbon such as dichloromethane, a mixture of two or more of such solvents or a mixture of one or more of such solvents with water, in the presence of an alkali metal hydroxide, carbonate or alcoholate or a tertiary amine, such as sodium hydroxide or triethylamine, preferably at a temperature of from about 0°C to about 40°C, to provide 3-(1-benzyl-1,2,3,6-tetrahydropyridin-4-yl)benzylcarbamic acid tert-butyl ester.

Another special embodiment of this eighth aspect provides for further treating the 3-(1-benzyl-1,2,3,6-tetrahydropyridin-4-yl)benzylcarbamic acid tert-butyl ester with hydrogen at a pressure of from about 200 kPa to about 3000 kPa in an aliphatic alcohol or ethyl acetate, in the presence of a palladium catalyst, in the presence of an organic or inorganic acid, to provide 4-[3-(tert-butoxycarbonylaminomethyl)phenyl]-piperidine as the salt of the organic or inorganic acid.

An ninth aspect of the present invention provides a process for the preparation of 4-[3-(tert-butoxycarbonylaminomethyl)phenyl]piperidine or its salt,
comprising the steps of
a) treating 3-(4-pyridyl)benzaldehyde with hydroxylamine or a salt thereof in an aliphatic alcohol, optionally in the presence of base, at a temperature of from about 0°C to about 40°C to provide 3-(4-pyridyl)benzaldehyde oxime or its salt;
b) treating the 3-(4-pyridyl)benzaldehyde oxime or its salt with hydrogen at a pressure of from about 300 kPa to about 1500 kPa in an aliphatic alcohol, in the presence of a palladium catalyst, at a temperature of from about 20°C to about 50°C to provide 3-(4-pyridyl)benzylamine or its salt;
c) treating the 3-(4-pyridyl)benzylamine or its salt with di-tert-butyl dicarbonate in an aliphatic alcohol such as methanol, ethyl acetate, an ether such as tetrahydrofuran, a halogenated hydrocarbon such as dichloromethane, a mixture of two or more of such solvents or a mixture of one or more of such solvents with water, in the presence of an alkali metal hydroxide, carbonate or alcoholate or a tertiary amine, such as sodium hydroxide or triethylamine, preferably at a temperature of from about 0°C to about 40°C, to provide 3-(4-pyridyl)benzylcarbamic acid tert-butyl ester; and
d) treating the 3-(4-pyridyl)benzylcarbamic acid tert-butyl ester with hydrogen in a aliphatic alcohol, at a pressure of from about 2000 kPa to about 6000 kPa, in the presence of a platinum catalyst, in the presence of an acid such as hydrogen chloride or sulfuric acid.

A special embodiment of this ninth aspect provides a process for the preparation of 4-[3-(tert-butoxycarbonylaminomethyl)phenyl]piperidine or its salt,
comprising the steps of
a) treating 3-(4-pyridyl)benzaldehyde with hydroxylamine hydrochloride in an aliphatic alcohol, at a temperature of from about 0°C to about 40°C to provide 3-(4-pyridyl)benzaldehyde oxime hydrochloride;
b) treating the 3-(4-pyridyl)benzaldehyde oxime hydrochloride with hydrogen at a pressure of from about 300 kPa to about 1500 kPa in an aliphatic alcohol, in the presence of a palladium catalyst, at a temperature of from about 20°C to about 50°C to provide 3-(4-pyridyl)benzylamine hydrochloride;
c) treating the 3-(4-pyridyl)benzylamine hydrochloride, with di-tert-butyl dicarbonate in an aliphatic alcohol such as methanol, ethyl acetate, an ether such as tetrahydrofuran, a halogenated hydrocarbon such as dichloromethane, a mixture of two or more of such solvents or a mixture of one or more of such solvents with water, in the presence of an alkali metal hydroxide, carbonate or alcoholate or a tertiary amine, such as sodium hydroxide or triethylamine, preferably at a temperature of from about 0°C to about 40°C, to provide 3-(4-pyridyl)benzylcarbamic acid tert-butyl ester; and
d) treating 3-(4-pyridyl)benzylcarbamic acid tert-butyl ester with hydrogen in an aliphatic alcohol, at a pressure of from about 2000 kPa to about 6000 kPa, in the presence of a platinum catalyst, in the presence of an acid such as hydrogen chloride or sulfuric acid.

A tenth aspect of the present invention provides a process for the preparation of 4-[3-(tert-butoxycarbonylaminomethyl)phenyl]piperidine or its salt,
comprising the steps of
a) treating 3-(4-pyridyl)benzonitrile with hydrogen in the presence of a palladium catalyst at a pressure of from about 2000 kPa to about 6000 kPa in an aliphatic alcohol or a mixture of an aliphatic alcohol and water, or with a complex hydride such as lithium aluminium hydride, lithium borohydride or sodium borohydride, in an aliphatic alcohol or an ether such as methanol or tetrahydrofuran, at a temperature of from about 0°C to about 50°C, to provide 3-(4-pyridyl)benzylamine or its salt;
b) treating the 3-(4-pyridyl)benzylamine or its salt with di-tert-butyl dicarbonate in an aliphatic alcohol such as methanol, ethyl acetate, an ether such as tetrahydrofuran, a halogenated hydrocarbon such as dichloromethane, a mixture of two or more of such solvents or a mixture of one or more of such solvents with water, in the presence of an alkali metal hydroxide, carbonate or alcoholate or a tertiary amine, such as sodium hydroxide or triethylamine, preferably at a temperature of from about 0°C to about 40°C, to provide 3-(4-pyridyl)benzylcarbamic acid tert-butyl ester; and
c) treating the 3-(4-pyridyl)benzylcarbamic acid tert-butyl ester with hydrogen in an aliphatic alcohol, at a pressure of from about 2000 kPa to about 6000 kPa, in the presence of a platinum catalyst, in the presence of an acid such as hydrogen chloride or sulfuric acid.

The present invention also provides novel intermediates useful in the processes of the present invention for the preparation of a compound of the formula I, 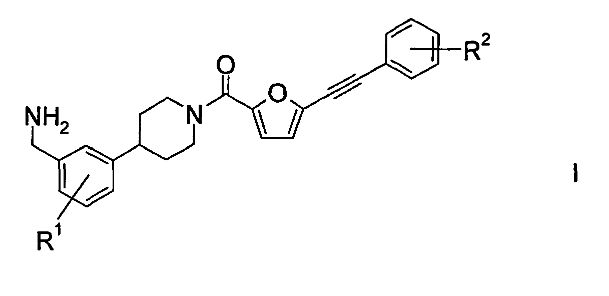 wherein
R¹ is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, (C₁-C₈)-alkoxy, (C₃-C₁₀)-cycloalkoxy, (C₆-C₁₄)-aryloxy, di((C₁-C₈)-alkyl)amino, di((C₃-C₁₀)-cycloalkyl)amino or di((C₆-C₁₄)-aryl)amino; and
R² is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, 5-membered to 10-membered heteroaryl comprising 1, 2 or 3 identical or different ring heteroatoms chosen from nitrogen, oxygen and sulfur, (C₁-C₈)-alkoxy, (C₃-C₁₀)-cycloalkoxy, (C₆-C₁₄)-aryloxy, 5-membered to 10-membered heteroaryloxy comprising 1, 2, or 3 identical or different ring heteroatoms chosen from nitrogen, oxygen and sulfur, di((C₁-C₈)-alkyl)amino, di-((C₃-C₁₀)-cycloalkyl)amino or di((C₆-C₁₄)-aryl)amino;
or its salt,
including:
a compound of the formula VIII, 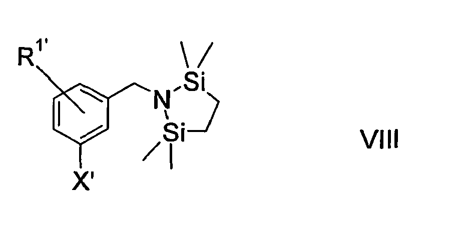 wherein R^{1'} is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, (C₁-C₈)-alkoxy, (C₃-C₁₀)-cycloalkoxy, (C₆-C₁₄)-aryloxy, di((C₁-C₈)-alkyl)amino, di((C₃-C₁₀)-cycloalkyl)amino or di((C₆-C₁₄)-aryl)amino, or a protected derivative thereof or precursor moiety thereto; and X' is bromo or iodo; the compound 1-(3-bromobenzyl)-2,2,5,5-tetramethyl-1-aza-2,5-disilacyclopentane being excluded;
a compound of the formula IX, 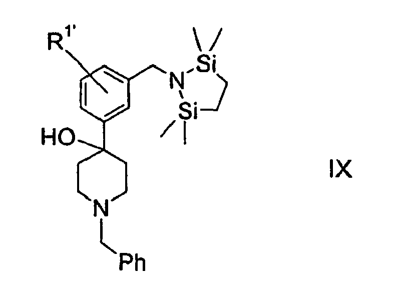
wherein R^{1'} is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, (C₁-C₈)-alkoxy, (C₃-C₁₀)-cycloalkoxy, (C₆-C₁₄)-aryloxy, di((C₁-C₈)-alkyl)amino, di((C₃-C₁₀)-cycloalkyl)amino or di((C₆-C₁₄)-aryl)amino, or a protected derivative thereof or precursor moiety thereto; and Ph is phenyl;
a compound of the formula X, 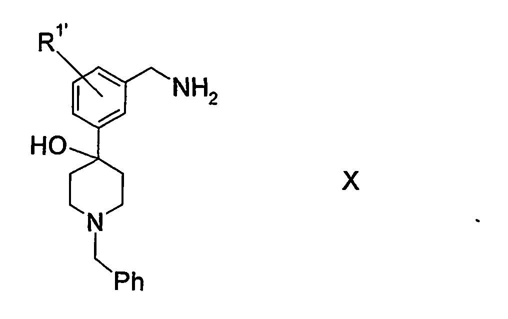
wherein R^{1'} is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, (C₁-C₈)-alkoxy, (C₃-C₁₀)-cydoalkoxy, (C₆-C₁₄)-aryloxy, di((C₁-C₈)-alkyl)amino, di((C₃-C₁₀)-cycloalkyl)amino or di((C₆-C₁₄)-aryl)amino, or a protected derivative thereof or precursor moiety thereto; and Ph is phenyl, or its salt;
a compound of the formula XI, 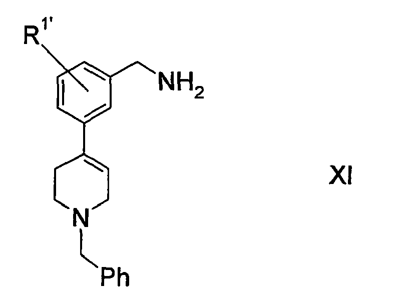
wherein R^{1'} is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, (C₁-C₈)-alkoxy, (C₃-C₁₀)-cycloalkoxy, (C₆-C₁₄)-aryloxy, di((C₁-C₈)-alkyl)amino, di((C₃-C₁₀)-cycloalkyl)amino or di((C₆-C₁₄)-aryl)amino, or a protected derivative thereof or precursor moiety thereto; and Ph is phenyl, or its salt;
a compound of the formula XII, 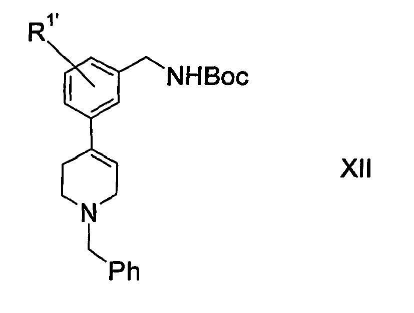
wherein R^{1'} is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, (C₁-C₈)-alkoxy, (C₃-C₁₀)-cycloalkoxy, (C₆-C₁₄)-aryloxy, di((C₁-C₈)-alkyl)amino, di((C₃-C₁₀)-cycloalkyl)amino or di((C₆-C₁₄)-aryl)amino, or a protected derivative thereof or precursor moiety thereto; Boc is tert-butoxycarbonyl; and Ph is phenyl, or its salt;
a compound of the formula XIV, 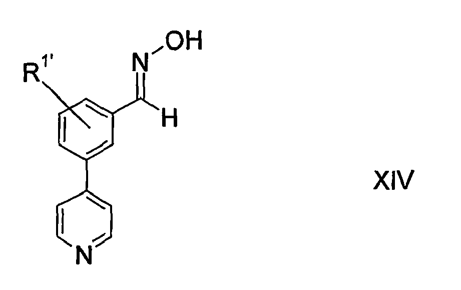
wherein R^{1'} is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, (C₁-C₈)-alkoxy, (C₃-C₁₀)-cycloalkoxy, (C₆-C₁₄)-aryloxy, di((C₁-C₈)-alkyl)amino, di((C₃-C₁₀)-cycloalkyl)amino or di((C₆-C₁₄)-aryl)amino, or a protected derivative thereof or precursor moiety thereto, or its salt;
a compound of the formula XV, 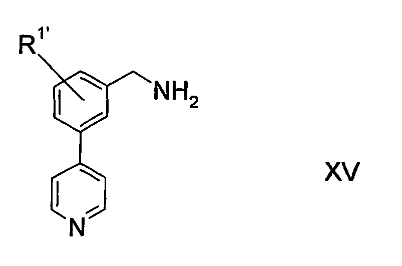
wherein R^{1'} is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, (C₁-C₈)-alkoxy, (C₃-C₁₀)-cycloalkoxy, (C₆-C₁₄)-aryloxy, di((C₁-C₈)-alkyl)amino, di((C₃-C₁₀)-cycloalkyl)amino or di((C₆-C₁₄)-aryl)amino, or a protected derivative thereof or precursor moiety thereto, or its salt;
a compound of the formula XVI, 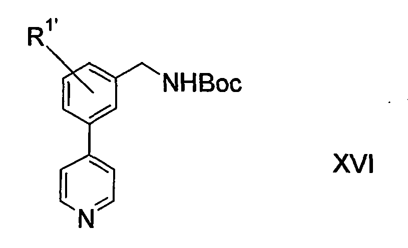
wherein R^{1'} is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, (C₁-C₈)-alkoxy, (C₃-C₁₀)-cydoalkoxy, (C₆-C₁₄)-aryloxy, di((C₁-C₈)-alkyl)amino, di((C₃-C₁₀)-cycloalkyl)amino or di((C₆-C₁₄)-aryl)amino, or a protected derivative thereof or precursor moiety thereto; and Boc is tert-butoxycarbonyl, or its salt; and
a compound of the formula IVb, 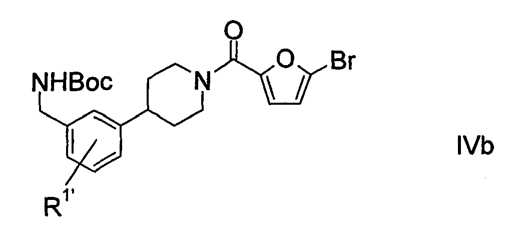
wherein R^{1'} is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, (C₁-C₈)-alkoxy, (C₃-C₁₀)-cycloalkoxy, (C₆-C₁₄)-aryloxy, di((C₁-C₈)-alkyl)amino, di((C₃-C₁₀)-cycloalkyl)amino or di((C₆-C₁₄)-aryl)amino, or a protected derivative thereof or precursor moiety thereto; and Boc is tert-butoxycarbonyl, or its salt;
and, in another aspect of the present invention, the use of each of these compounds as an intermediate, in particular as an intermediate for the preparation of a compound of the formula I, 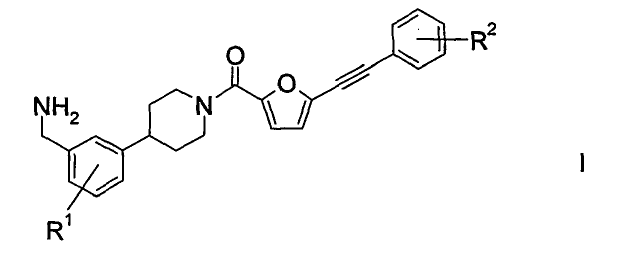
wherein R¹ and R² are defined as above, or its salt.

In a particular embodiment, the present invention also provides novel intermediates useful in the processes of the present invention for the preparation of 4-[3-(aminomethyl)phenyl]-1-[5-(2-fluorophenylethynyl)-2-furanoyl]piperidine or its salt, including:
1-(3-iodobenzyl)-2,2,5,5-tetramethyl-1-aza-2,5-disilacyclopentane;
1-[3-(1-benzyl-4-hydroxypiperidin4-yl)benzyl]-2,2,5,5-tetramethyl-1-aza-2,5-disilacyclopentane;
3-(1-benzyl-4-hydroxypiperidin-4-yl)benzylamine or its salt;
3-(1-benzyl-1,2,3,6-tetrahydropyridin-4-yl)benzylamine or its salt;
3-(1-benzyl-1,2,3,6-tetrahydropyridin-4-yl)benzylcarbamic acid tert-butyl ester or its salt;
3-(4-pyridyl)benzaldehyde oxime or its salt;
3-(4-pyridyl)benzylamine or its salt;
3-(4-pyridyl)benzylcarbamic acid tert-butyl ester or its salt; and
4-[3-(tert-butoxycarbonylaminomethyl)phenyl]-1-[5-bromo-2-furanoyl]piperidine;
and, in another particular embodiment of the present invention, the use of each of these compounds as an intermediate for the preparation of 4-[3-(aminomethyl)phenyl]-1-[5-(2-fluorophenylethynyl)-2-furanoyl]piperidine or its salt.

As used herein, and unless otherwise specified, the following terms are understood to have the following meanings.

"Alkyl" means a residue of an aliphatic saturated hydrocarbon group which may be straight or branched having from about 1 to about 8 carbon atoms, for example 1, 2, 3, 4, 5, 6, 7 or 8 carbon atoms. Preferred alkyl residues are those which have from about 1 to about 6 carbon atoms, in particular from about 1 to about 4 carbon atoms. Exemplary alkyl residues include methyl, ethyl, n-propyl, iso-propyl, n-butyl, isobutyl, s-butyl, t-butyl, n-pentyl, 3-pentyl, heptyl and octyl.

"Cycloalkyl" means a residue of a saturated monocyclic or bicyclic ring system containing from about 3 to about 10 ring carbon atoms, for example 3, 4, 5, 6, 7, 8, 9 or 10 carbon atoms. Preferred cycloalkyl residues are those which have from about 3 to about 7 carbon atoms. Exemplary cycloalkyl residues include cyclopropyl, cyclobutyl, cyclopentyl, cyclohexyl, cycloheptyl and cyclooctyl.

"Heterocycloalkyl" means a residue of an about 3-membered to about 10-membered, for example 3-, 4-, 5-, 6-, 7-, 8-, 9- or 10-membered, saturated, monocyclic or multicyclic heterocyclic ring system which comprises 1, 2 or 3 identical or different ring heteroatoms chosen from nitrogen, oxygen and sulfur, which can be bonded via any suitable ring carbon atom or ring nitrogen atom and nitrogen atoms of which, independently of one another, can carry a residue chosen from hydrogen, (C₁-C₈)-alkyl, (C₆-C₁₄)-aryl and (C₆-C₁₄)-aryl-(C₁-C₄)-alkyl-. Heterocycloalkyl includes, for example, (i) a cycloalkyl group of from about 3 to about 7 ring members of which 1, 2 or 3 are ring heteroatoms chosen from nitrogen, oxygen and sulfur, i. e. a saturated monocyclic heterocyclic group, and (ii) a residue of a saturated multicyclic, for example bicyclic or tricyclic, heterocyclic group of from about 6 to about 10 ring members of which 1, 2 or 3 are ring heteroatoms chosen from nitrogen, oxygen and sulfur, in which a saturated monocyclic heterocyclic group is fused to one or more, for example one or two, cycloalkyl groups and/or saturated monocyclic heterocyclic groups to form a multicyclic cyclic structure. Exemplary heterocycloalkyl groups include piperidinyl, pyrrolidinyl, morpholinyl, tetrahydropyranyl, or tetrahydrothienyl.

"Aryl" as a group or part of a group means a residue of a monocyclic or multicyclic ring system comprising from about 6 to about 14 ring carbon atoms, for example 6, 8, 9, 10, 12, 13 or 14 ring carbon atoms. Aryl includes: (i) a residue of a monocyclic or multicyclic, for example bicyclic or tricyclic, aromatic carbocyclic group of from about 6 to about 14 ring carbon atoms, all rings of which are aromatic, such as phenyl or naphthyl; or (ii) a residue of partially saturated multicyclic, for example bicyclic or tricyclic, aromatic carbocyclic group in which an aryl group and a cycloalkyl group are fused together to form a cyclic structure, such as a tetrahydronaphthyl or indanyl residue.

"Heteroaryl" means a residue of an about 5-membered to about 10-membered, for example 5-, 6-, 8-, 9- or 10-membered, heteroaromatic ring system which comprises 1, 2 or 3 identical or different ring heteroatoms chosen from nitrogen, oxygen and sulfur, which can be bonded via any suitable ring carbon atom or ring nitrogen atom and nitrogen atoms of which, independently of one another, can carry a residue chosen from hydrogen, (C₁-C₈)-alkyl, (C₆-C₁₄)-aryl and (C₆-C₁₄)-aryl-(C₁-C₄)-alkyl-. Heteroaryl includes: (i) a residue of a monocyclic or bicyclic aromatic heterocyclic group all rings of which are aromatic, including, for example, residues like benzimidazolyl, benzothiazolyl, benzoxazolyl, benzothienyl, furyl, imidazolyl, indolyl, indolizinyl, isoxazolyl, isoquinolinyl, isothiazolyl, oxadiazolyl, pyrazinyl, pyridazinyl, pyrazolyl, pyridyl, pyrimidinyl, pyrrolyl, quinazolinyl, quinolinyl, 1,3,4-thiadiazolyl, thiazolyl, thienyl or triazolyl, or (ii) a residue of a partially saturated bicyclic heteroaromatic group in which a heteroaryl group and a cycloalkyl group or an aryl group and a heterocycloalkyl group or a heteroaryl and a heterocycloalkyl group are fused together to form a cyclic structure, for example a pyrindanyl residue.

"Alkoxy" means an alkyl-O- residue in which the alkyl group is as described herein. Exemplary alkoxy groups include methoxy, ethoxy, n-propoxy, isopropoxy, n-butoxy, isobutoxy, tert-butoxy and heptoxy.

"Cycloalkoxy" means a cycloalkyl-O- residue, in which the cycloalkyl group is as described herein.

"Aryloxy" means an aryl-O- residue, in which the aryl group is as described herein.

"Heteroaryloxy" means a heteroaryl-O- residue, in which the heteroaryl group is as described herein.

"Dialkylamino" means an -N(alkyl)(alkyl) residue in which the alkyl groups may be the same or different and are as described herein.

"Dicycloalkylamino" means an -N(cycloalkyl)(cycloalkyl) residue in which the cycloalkyl groups may be the same or different and are as described herein.

"Diarylamino" means an -N(aryl)(aryl) residue in which the aryl groups may be the same or different and are as described herein.

In the processes and compounds according to the invention the groups R¹, R^{1'}, R² and R^{2'}, which are attached to the benzene rings depicted in the formulae herein, may independently of one another be present one or more times, for example one, two or three times. Preferably they are independently of one another present one or two times. More preferably, one group R¹ or R^{1'} is present and one group R² or R^{2'} is present. When more than one of any of the groups R¹, R^{1'}, R² and R^{2'} is present, they can all independently of one another have the meanings indicated herein and can be the same or different. Positions on the benzene rings depicted in the formulae herein which do not carry a group R¹, R^{1'}, R² or R^{2'} or another group drawn in the formulae, carry hydrogen atoms. I.e., in a compound of the formula I in which one group R¹ and one group R² is present, the benzene ring carrying R¹ carries three hydrogen atoms in addition to the group R¹ and the group -CH₂NH₂, and the benzene ring carrying R² carries four hydrogen atoms in addition to the group R². In a compound of the formula I in which two groups R² are present, the benzene ring carrying R² carries three hydrogen atoms in addition to the group R². The groups R¹, R^{1'}, R² and R^{2'} can be present in any desired positions. For example, in a compound of the formula I in which one group R² is present that is different from hydrogen, R² can be present in position 2, 3 and 4 with respect to the ethyne moiety which, by definition, is present in position 1. If two groups R² are present that are different from hydrogen, they can be present in 2,3-, 2,4-, 2,5-, 2,6-, 3,4- and 3,5-position. Similarly, if one group R¹ is present that is different from hydrogen, it can be present in position 2, 3, 4 and 6 with respect to the position of the piperidine residue which is present in position 1, if the position of the group -CH₂NH₂ in a compound of the formula I or of the group derived therefrom in the other compounds of the invention is designated as position 5 (if the position of the group -CH₂-NH₂ is designated as position 3, the group R¹ can be present in position 2, 4, 5 and 6).

Salts of a compound according to or related to the present invention, i.e. any compound which is employed into a process or which is obtained by a process or which occurs as an intermediate in a process described herein, including any compound which is a subject of the present invention per se, which contains one or more basic groups, i.e. protonatable groups such as amino groups, piperidinyl groups, pyridinyl groups and/or other basic heterocyclic groups, are in particular acid addition salts formed by the respective compound and inorganic or organic acids, including mineral acids and organic carboxylic acids and sulfonic acids. Examples of acids which may form such salts are hydrochloric acid, hydrobromic acid, hydroiodic acid, sulfuric acid, sulfamic acid, nitric acid, phosphoric acid, formic acid, acetic acid, propionic acid, oxalic acid, malonic acid, citric acid, trifluoroacetic acid, lactic acid, tartaric acid, fumaric acid, maleic acid, methanesulfonic acid, ethanesulfonic acid, benzenesulfonic acid, p-toluenesulfonic acid, etc. Compounds which contain two or more basic groups can form acid addition salts with one acid equivalent or with two or more acid equivalents. In particular in the case of compounds of the formulae I and la which are intended for use as in the production of pharmaceutical compositions or for use as pharmacologically active ingredients, preferred salts are physiologically acceptable salts or pharmaceutically acceptable salts which are non-toxic and exhibit a suitable property profile for the intended use. However, the present invention includes also salts which, owing to low physiological compatibility, are not directly suitable for use in pharmaceuticals but which can be used, for example, as intermediates for chemical reactions or for the preparation of physiologically acceptable salts, for example by ion exchange procedures. Salts may be obtained by any process as described herein or by customary methods which are known to the person skilled in the art such as, for example, by contacting a basic compound with an acid in a solvent, or by ion exchange from another salt. Likewise, by applying customary methods such as contacting a salt with a base in a solvent, a salt can be converted into the free base if desired, for example if an intermediate which has been isolated as a salt is needed in a subsequent reaction step as the free base.

In the processes and compounds according to the present invention, and in particular for various values of R¹ and R², it may be advantageous or necessary to protect reactive functional groups, where these are desired in the final product, or let them be present initially in form of precursor moieties which are later converted into the desired group, in order to avoid their unwanted participation in reactions according to processes of the present invention. Conventional protecting groups and precursor moieties and processes for introduction and their removal or conversion into the desired groups may be used in accordance with standard practice known to one skilled in the art, for example as described in T.W. Greene and P.G.M. Wuts in "Protective Groups in Organic Chemistry", John Wiley and Sons, 1991.

In the processes and compounds according to the present invention R¹ and R^{1'} preferably are H, F, CF₃, OCF₃, (C₁-C₄)-alkyl, (C₃-C₇)-cycloalkyl, phenyl, (C₁-C₄)-alkoxy, phenoxy or di((C₁-C₄)-alkyl)amino, more preferably H, F, CF₃, OCF₃, (C₁-C₄)-alkyl, (C₁-C₄)-alkoxy, or di((C₁-C₄)-alkyl)amino, particularly preferably H, F, CF₃, OCF₃, (C₁-C₄)-alkyl or (C₁-C₄)-alkoxy, more particularly preferably H, F, CF₃, OCF₃, CH₃ or OCH₃, especially preferably H or F, moreover preferably H. In case that one residue R¹ or R^{1'} is present which is F, the flourine atom is preferably present in position 2 with respect to the position of the piperidine residue which is present in position 1, if the position of the group -CH₂-NH₂ in a compound of the formula I, or of the group derived therefrom in the other compounds of the invention, is designated as position 5. R² and R^{2'} preferably are H, F, CF₃, OCF₃, (C₁-C₄)-alkyl, (C₃-C₇)-cycloalkyl, phenyl, (C₁-C₄)-alkoxy, phenoxy or di((C₁-C₄)-alkyl)amino, more preferably H, F, CF₃, OCF₃, (C₁-C₄)-alkyl, (C₁-C₄)-alkoxy, or di((C₁-C₄)-alkyl)amino, particularly preferably H, F, CF₃, OCF₃, (C₁-C₄)-alkyl or (C₁-C₄)-alkoxy, more particularly preferably H, F, CF₃, OCF₃, CH₃ or OCH₃, especially preferably H or F. In case that one residue R² or R^{2'} is present, in a moreover preferred embodiment of the present invention R² or R^{2'} is F, in particular a fluorine atom in ortho position to the ethyne moiety.

The processes according to the present invention are usually performed in the presence of a solvent (or diluent) but, depending on the individual case, may also be performed without addition of a solvent. Solvents that may be used include inorganic solvents, for example water, and organic solvents. Examples of organic solvents are hydrocarbons, including aliphatic and aromatic hydrocarbons, for example pentane, hexane, heptane, petrol ether, ligroin, cyclohexane, methylcyclohexane, benzene, toluene or xylene, halogenated hydrocarbons, including halogenated aliphatic and aromatic hydrocarbons, for example dichloromethane, trichloromethane, carbon tetrachloride, dichloroethane, trichloroethane, chlorobenzene or dichlorobenzene, ethers, including aliphatic ethers and cyclic ethers, for example diethyl ether, di-n-propylether, di-isopropyl ether, dibutylether, ethyleneglycol dimethyl ether, diethyleneglycol dimethyl ether, tetrahydrofuran or dioxane, esters, for example ethyl acetate, butyl acetate or dimethyl carbonate, amides, for example dimethylformamide, dimethylacetamide or N-methylpyrrolidone, alcohols including aliphatic alcohols, for example methanol, ethanol, propanol including n-propanol and isopropanol, butanol including n-butanol and isobutanol, or ethyleneglycol, acids including aliphatic acids, for example formic acid, acetic acid or trifluoroacetic, and others, for example acetone, butanone, dimethyl sulfoxide or acetonitrile. The term "solvent", as used herein, includes besides a single solvent also a mixture of two or more solvents which may be miscible or partly miscible or immiscible and may form two or more phases. The term "solvent" thus includes, for example, mixtures of two or more organic solvents, such as mixtures of an ether and an alcohol or mixtures of an ether and a hydrocarbon, and mixtures of one or two or more organic solvents, for example methanol, tetrahydrofuran or dichloromethane, with water. The choice of a suitable solvent which is advantageously used in a specific preparation process, will be made by a person skilled in the art in view of the characteristics of the reaction, the compounds which are employed and obtained, and technical and other aspects. Generally, inert solvents are used which do not undergo unwanted reactions with the employed compounds and agents and/or the obtained compounds. Depending on the individual case, it may be preferable to use a solvent which dissolves the employed compounds and agents and/or the obtained compounds, or a solvent which dissolves the employed compounds and agents and/or the obtained compounds to some extent, or a solvent which only slightly dissolves the employed compounds and agents and/or the obtained compounds. Examples of suitable solvents are given above and below in the explanations on the processes according to the invention.

In various processes according to the present invention a base is employed, for example, to produce a chemical conversion or to deprotonate a compound or liberate a basic starting compound from its salt or to scavenge an acid formed during a reaction. Bases that may be used include organic bases, for example amines including tertiary amines such as triethylamine, tributylamine, ethyldiisopropylamine or N-methylmorpholine, and inorganic bases. Inorganic bases usually are basic compounds of the alkali metals lithium, sodium and potassium and the alkaline earth metals magnesium and calcium and include hydroxides, for example lithium hydroxide, sodium hydroxide, potassium hydroxide or calcium hydroxide, carbonates and hydrogencarbonates, for example sodium carbonate, sodium hydrogencarbonate or potassium carbonate, alcoholates, for example sodium methanolate, sodium ethanolate, sodium tert-butanolate, potassium methanolate, potassium ethanolate, potassium tert-butanolate, magnesium methanolate, magnesium ethanolate, calcium methanolate or calcium ethanolate, and others, for example sodium hydride, sodium amide, lithium diisopropylamide, Na₂HPO₄, Na₃PO₄ or sodium acetate. Besides a single base also mixtures of two or more bases may be used. The choice of a suitable base that is advantageously used in a specific preparation process, will be made by a person skilled in the art in view of the characteristics of the reaction, the compounds which are employed and obtained, and technical and other aspects. Examples of suitable bases are given above and below in the explanations on the processes according to the invention.

In the following, further explanations on the processes according to the present invention are given. In the Schemes below groups like R¹, R², R^{1'} and R^{2'} are defined as above, Boc is tert-butoxycarbonyl, Ph is phenyl and nBu is n-butyl.

The compounds of the formula I are prepared by processes according to the present invention as shown in Scheme 1.

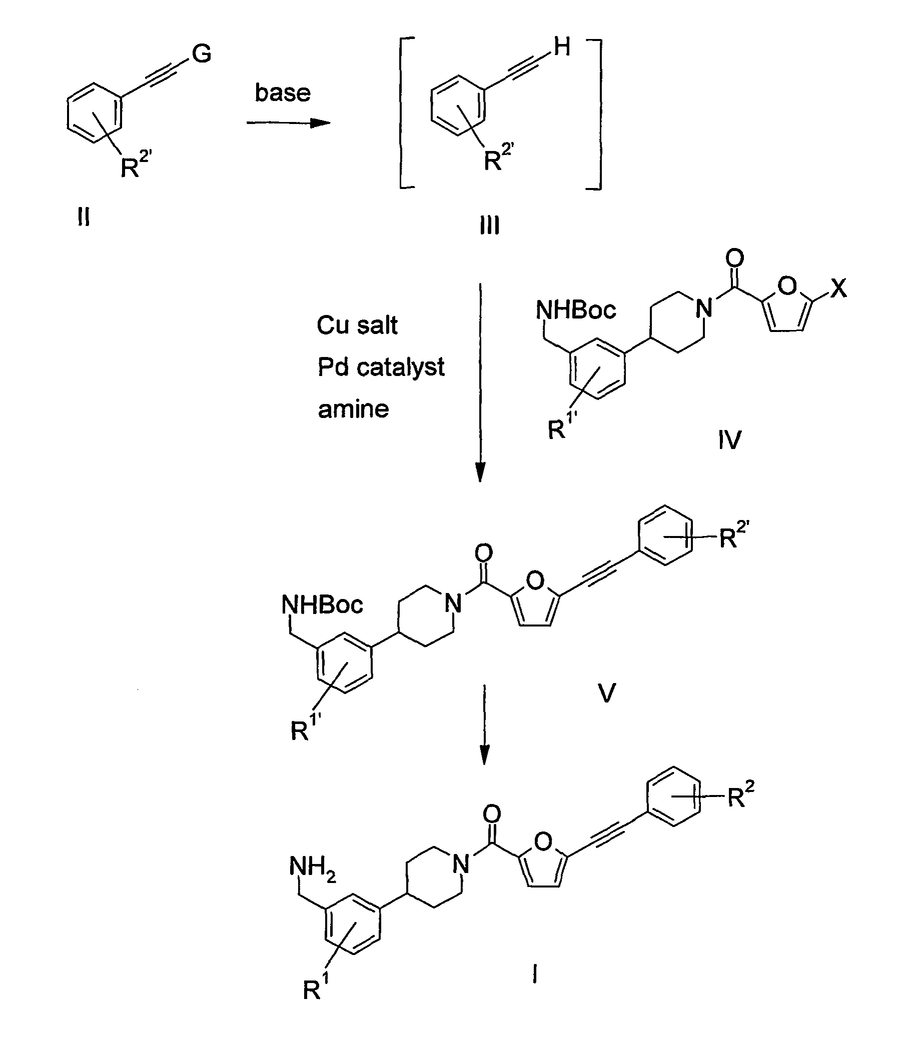

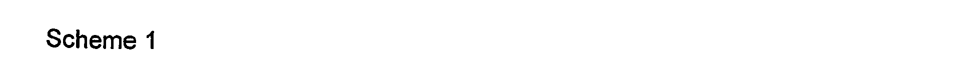

The phenylethynylsilyl compound of the formula II, wherein G is trimethylsilyl, triethylsilyl, tri-isopropylsilyl, or dimethyl-tert-butylsilyl, is treated with an alkali metal hydroxide, carbonate or alcoholate, for example the respective sodium or potassium compound, or an alkaline earth metal alcoholate, for example a magnesium or calcium alcoholate, preferably with potassium carbonate, in a solvent, such as an aliphatic alcohol or a mixture of an aliphatic alcohol and an ether, for example methanol or ethanol or a mixture of methanol and tetrahydrofuran, at a temperature of from about 0°C to about 50°C, preferably of from about 10°C to about 30°C, to give a solution of the corresponding phenylethynyl compound of the formula III which phenylethynyl compound is not isolated, thus avoiding the hazards that would be associated with the handling of the unstable phenylethynyl compound. The base can be employed in a catalytic amount, for example in an amount of from about 0.02 to about 0.05 mol per mol of the compound of formula II, or in a greater amount up to an about equivalent amount, for example an amount of from about 0.02 to about 1.1 mol of a monovalent base per mol of the compound of the formula II.

The solution of the phenylethynyl compound is combined with the amide of the formula IV in a solvent, such as an ether, for example tetrahydrofuran, dioxane or 1,2-dimethoxyethane, or dimethyl carbonate, or a mixture of an ether with an aliphatic alcohol such as methanol or ethanol, and reacted in the presence of a homogeneous palladium catalyst, for example Pd(PPh₃)₂Cl₂, a cuprous salt, for example copper(I)iodide, copper(I)bromide, copper(I)chloride or copper(I)trifluoromethanesulfonate, where the cuprous salt can also be formed in situ from a cupric salt, for example copper(II)acetate, and a hindered amine, for example triethylamine, tributylamine, ethyldiisopropylamine, diethylamine, pyrrolidine or piperidine, at a temperature of from about 30°C to about 70°C. The compound of the formula III or II, respectively, is preferably employed in a slight excess, for example in an amount of from about 1 to about 1.5 mol per mol of the compound of the formula IV. The employed amount of the palladium catalyst and the cuprous salt each preferably is from about 0.005 to about 0.2, more preferably from about 0.005 to about 0.05 mol per mol of the compound of the formula IV, and the amount of the hindered amine preferably is from about 1 to about 10, more preferably from about 1 to about 5 mol per mol of the compound of the formula II.

Work-up of the reaction mixture can be performed by customary procedures, as applies to all processes of the present invention, including treatment of the organic phase with brine, dilute hydrochloric acid, and aqueous sodium carbonate, to provide the Boc-protected compound of the formula V. In a surprising aspect of the present invention the reagents present in the solution resulting from treatment of the phenylethynylsilyl compound of the formula II do not interfere with the subsequent coupling reaction with the compound of the formula IV.

The Boc-protected compound of the formula V can be used as a storage form of the compound of the formula I, for example. If it is desired to prepare the compound of the formula I itself or its salt, the compound of the formula V is subsequently deprotected by splitting off the Boc group and, if applicable, removal of any other protective groups and/or conversion of precursor moieties that may be present in R^{1'} and/or R^{2'}. Preferably the compound of the formula V is deprotected under acidic conditions, in particular from a compound in which R^{1'} and R^{2'} are R¹ and R², advantageously by means of a physiologically acceptable acid such as methanesulfonic acid, for example, in a solvent such as isopropanol, for example, at a temperature of from about 10°C to about 80°C, to yield the compound of formula I or its salt, preferably the salt with the acid employed into the deprotection step.

The compound of the formula IV can be prepared as illustrated in Scheme 2. When starting from 5-bromo-2-furoic acid or 5-iodo-2-furoic acid of the formula XVIII, wherein X is bromo or iodo, the acid is first activated as outlined above to give an activated acid derivative of the formula XIX wherein X is bromo or iodo and A is a leaving group, for example chloro or bromo, or the formula XIX represents the adduct obtained form the acid and a condensing agent such as a carbodiimide, or the mixed anhydride obtained from the acid and an alkyl chlorocarbonate. Preferably the acid of the formula XVIII is treated with thionyl chloride in refluxing toluene until gas evolution ceases to provide a solution of the acid chloride of the formula XIX wherein A is chloro, which solution may be used, if desired, in the subsequent step without isolation of the acid chloride. The activated acid derivative of the formula XIX is combined with the 4-[3-(tert-butoxycarbonylaminomethyl)phenyl]piperidine of the formula VI in a solvent, optionally in the presence of a base, for example a tertiary amine like triethylamine, to provide the corresponding amide of the formula IV, generally at a temperature of from about -10°C to about 40°C, preferably from about 10°C to about 30°C. The 4-[3-(tert-butoxycarbonylaminomethyl)phenyl]piperidine of the formula VI may be liberated from a salt of a compound of the formula VI, for example a salt with p-toluenesulfonic acid, by treatment with a base, for example an alkali metal hydroxide or alcoholate, such as sodium hydroxide, and extracting the compound of the formula VI into a solvent such as, for example, toluene or dichloromethane, followed by drying of the solution.

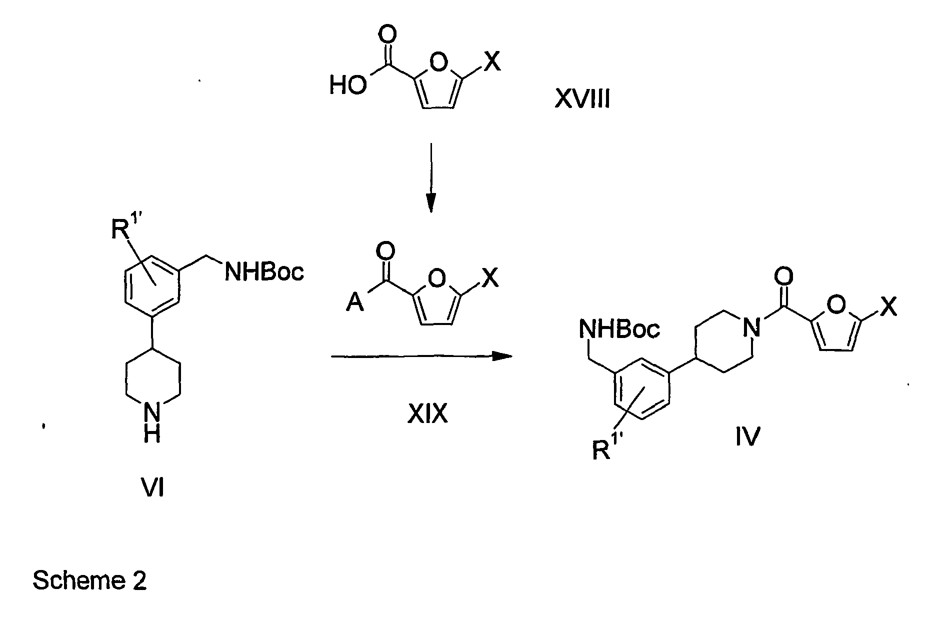

Previously described processes for the preparation of compounds of the formula I include the process for the preparation of the specific compound 4-[3-(aminomethyl)phenyl]-1-[5-(2-fluorophenylethynyl)-2-furanoyl]piperidine methanesulfonic acid salt which is described in WO 2004/060884 (international patent application PCT/US2003/040653) and shown in Scheme 3.

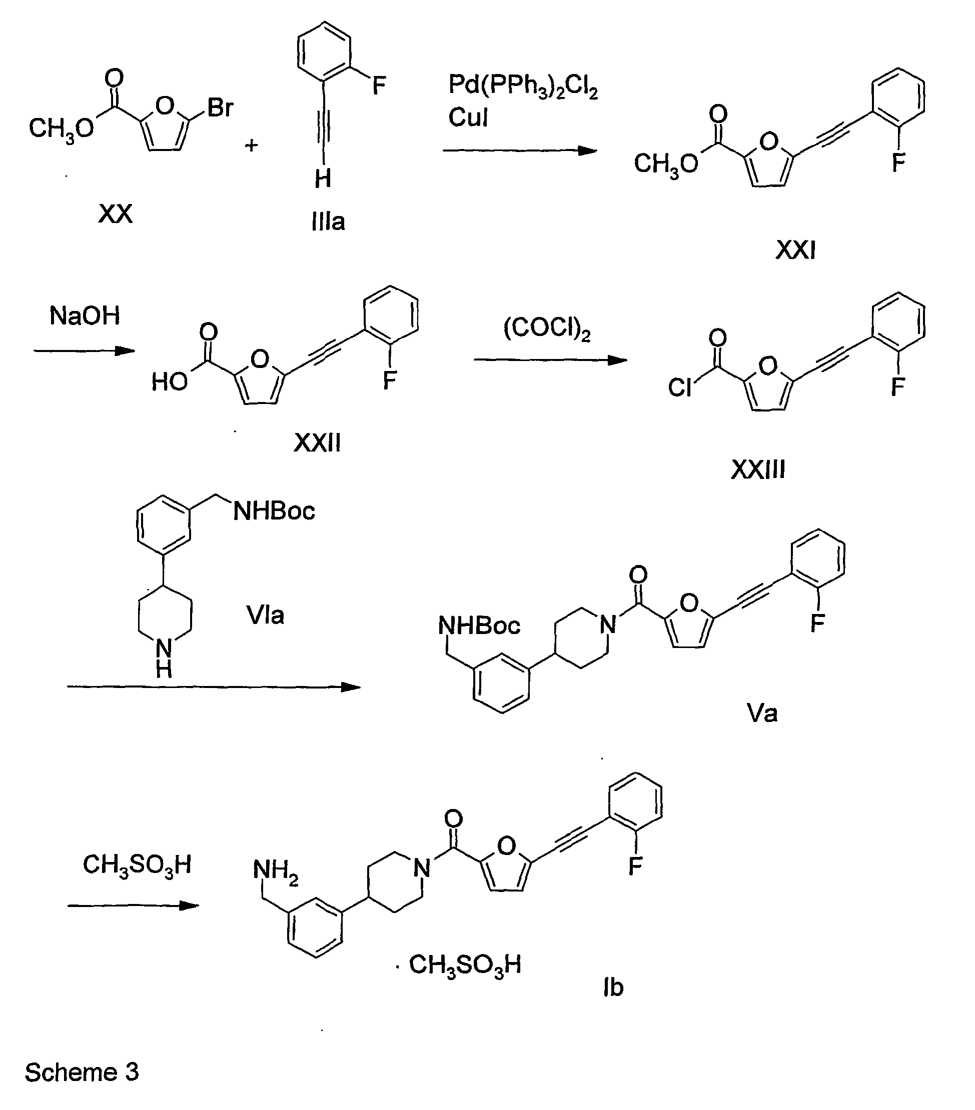

According to the previously described process, 2-fluorophenylethyne of the formula IIIa is coupled to methyl 5-bromo-2-furoate of the formula XX via a palladium mediated process. Work-up and chromatography yields methyl 5-(2-fluorophenylethynyl)-2-furoate of the formula XXI, which is saponified and recrystallized to give the respective acid of the formula XXII. Conversion of the acid into the acid chloride of the formula XXIII is accomplished with oxalyl chloride. Acylation of 4-[3-(tert-butoxycarbonylaminomethyl)phenyl]piperidine of the formula VIa which, according to the prior process, is prepared in a tedious process using costly reagents as outlined below, with the acid chloride gives N-Boc-protected 4-[3-(aminomethyl)phenyl]-1-[5-(2-fluorophenylethynyl)-2-furanoyl]piperidine of the formula Va. The crude product is purified by flash chromatography followed by trituration with diethyl ether/pentane or, alternatively, recrystallization. By treatment with methanesulfonic acid the Boc group is removed and 4-[3-(aminomethyl)phenyl]-1-[5-(2-fluorophenylethynyl)-2-furanoyl]piperidine methanesulfonic acid salt of the formula lb is obtained.

A substantial drawback of the earlier disclosed processes is the use of phenylethynes as starting materials which are known to be unstable. For example, 2-fluorophenylethyne decomposes violently at approximately 120°C. The use of phenylethynes thus poses a safety hazard, in particular in large scale productions. The present invention provides advantageous processes which are useful in the preparation of compounds of the formula I and are distinguished by generation of the phenylethyne from the silyl compound in situ without need for isolation of the phenylethyne, thus allowing to avoid the handling of hazardous compounds.

The 4-[3-(tert-butoxycarbonylaminomethyl)phenyl]piperidines of the formula VI useful as intermediates in the processes of the present invention may be prepared by processes of the present invention, for example, as shown in Scheme 4.

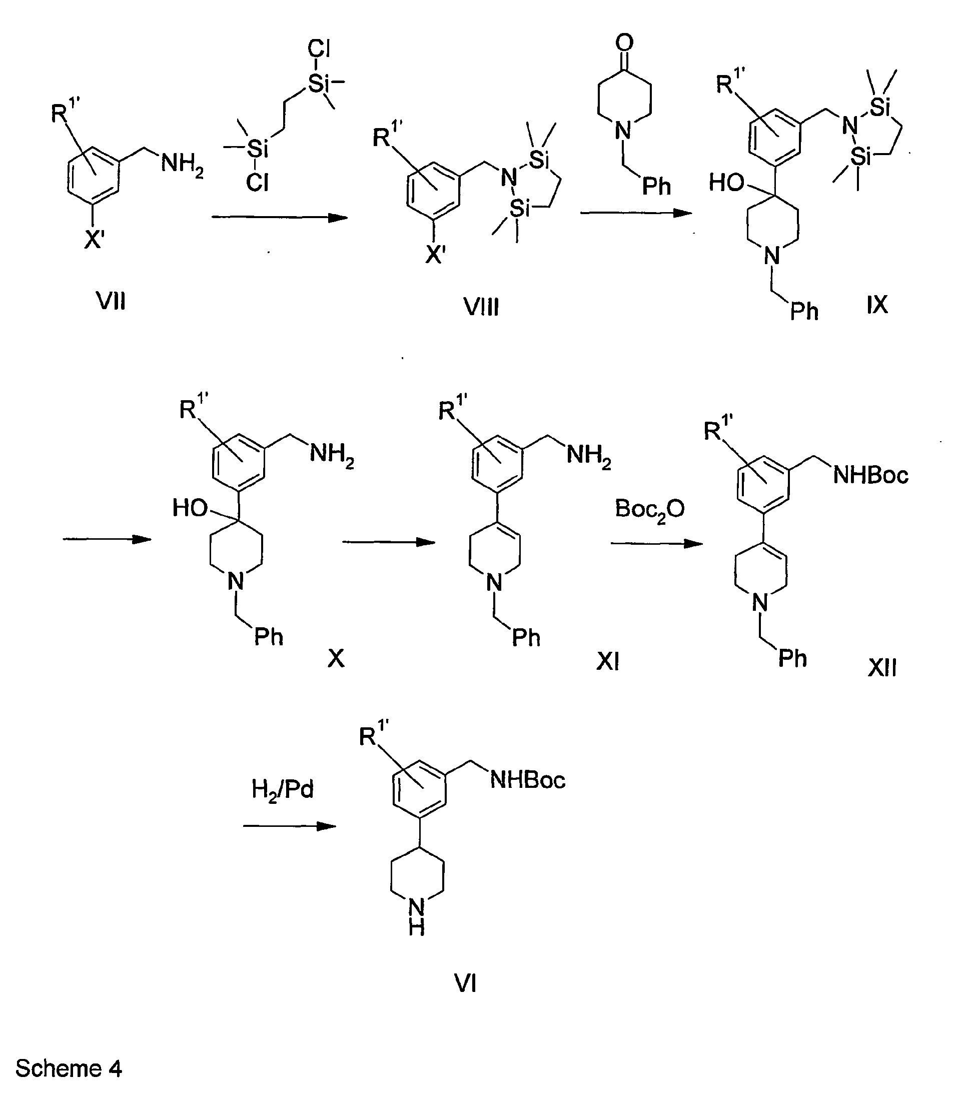

Accordingly, a 3-iodobenzylamine or 3-bromobenzylamine of the formula VII in the form of its salt, for example the hydrochloride salt, in a solvent, for example a halogenated aliphatic hydrocarbon such as dichloromethane, is treated with an about equimolar amount of 1,2-bis(chlorodimethylsilyl)ethane in the presence of a base, for example a tertiary amine such as triethylamine, preferably at a temperature of from about 10°C to about 30°C. Work-up of the reaction mixture can be accomplished by filtration, evaporation of the filtrate, and treatment of the residue with an aliphatic or aromatic hydrocarbon, for example pentane, to remove any residual amine salt, to provide the corresponding 3-halobenzyl compound of the formula VIII, i.e., the corresponding 1-(3-bromobenzyl)-2,2,5,5-tetramethyl-1-aza-2,5-disilacyclopentane or 1-(3-iodobenzyl)-2,2,5,5-tetramethyl-1-aza-2,5-disilacyclopentane.

The compound of the formula VIII is then treated with an alkyllithium compound, for example n-butyllithium, sec-butyllithium, t-butyllithium or hexyllithium, in a solvent, for example an ether such as diethyl ether or tetrahydrofuran, and subsequently with 1-benzyl-4-piperidone at a reduced temperature, such as a temperature of from about -80°C to about -40°C, for example at about -60°C, to provide the corresponding alcohol of the formula IX, which may be carried forward, if desired, without further purification. The alkyllithium compound and the 1-benzyl-4-piperidone are usually employed in an amount of from about 1 to about 1.2 and about 1 to about 1.1 mol, respectively, per mol of the compound of the formula VIII.

The alcohol of the formula IX is then treated with an inorganic acid, for example sulfuric acid, hydrochloric acid or phosphoric acid, preferably phosphoric acid, which may be dilute or concentrated, in a solvent, for example a halogenated aliphatic hydrocarbon such as dichloromethane, in the presence of water, at a temperature of from about 10°C to about 30°C, to provide the corresponding benzylamine of the formula X as its salt with the employed acid, which may be carried forward, if desired, without further purification.

The salt of the benzylamine of the formula X is then dehydrated with a concentrated, non-oxidizing strong acid, for example phosphoric acid, sulfuric acid, trifluoroacetic acid, or toluenesulfonic acid, preferably phosphoric acid, at an elevated temperature, for example at a temperature of from about 70°C to about 150°C, preferably at about 100°C, followed by alkalinization with a base such as an alkali metal hydroxide, carbonate or alcoholate, for example sodium hydroxide, to provide the corresponding olefin of the formula XI, which may be carried forward, if desired, without further purification.

The compound of the formula XI is then treated with di-tert-butyl dicarbonate (Boc₂O), which is generally employed in a slight excess of from about 1 to about 1.2 molar equivalents, in a solvent, for example an aliphatic alcohol such as methanol, ethyl acetate, an ether such as tetrahydrofuran, a halogenated hydrocarbon such as dichloromethane, a mixture of two or more of such solvents or a mixture of one or more of such solvents with water, in the presence of a base, for example an alkali metal hydroxide, carbonate or alcoholate or a tertiary amine, such as sodium hydroxide or triethylamine, preferably at a temperature of from about 0°C to about 40°C, preferably at a temperature of from about 10°C to about 30°C to provide the Boc-protected amine of the formula XII.

In a favorable manner, achieving simultaneous reduction of the olefinic double bond and removal of the benzyl group, the Boc-protected amine of the formula XII can then be treated with hydrogen at a pressure of from about 200 kPa to about 3000 kPa, in a solvent, for example an aliphatic alcohol such as methanol, ethanol or isopropanol, or ethyl acetate, in the presence of a palladium catalyst, for example palladium on carbon or palladium hydroxide on carbon, for example Pearlman's catalyst, and an organic or inorganic acid, for example acetic acid, at a temperature of from about 10°C to about 40°C, to provide the corresponding piperidine of the formula VI as its salt with the employed acid. If desired, the salt can then be converted to the 4-[3-(tert-butoxycarbonylaminomethyl)phenyl]piperidine of the formula VI by treatment with a base, for example an alkali metal hydroxide, carbonate or alcoholate.

As indicated above, in the process for the preparation of the compounds of the formulae XII and VI according to the present invention many intermediates can advantageously be taken to the next reaction step, if desired, without a separate purification step and intermediary isolation.

The 4-[3-(tert-butoxycarbonylaminomethyl)phenyl]piperidines useful as intermediates in the processes of the present invention also may be prepared by processes of the present invention, for example, as shown in Scheme 5.

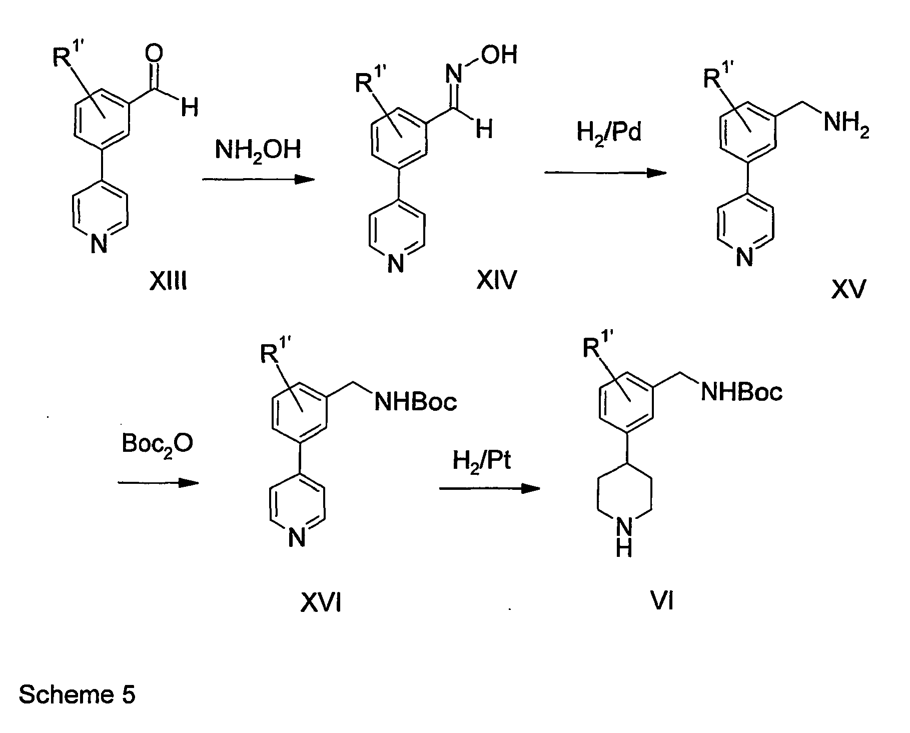

Accordingly, a 3-(4-pyridyl)benzaldehyde of the formula XIII is treated with hydroxylamine or a salt of hydroxylamine, for example hydroxylammonium chloride, sulfate or phosphate, in a solvent, for example an aliphatic alcohol such as methanol, ethanol or isopropanol, optionally in the presence of a base, for example a tertiary amine such as triethylamine, an alkali metal carbonate such as sodium carbonate, or an alkali metal hydrogencarbonate such as sodium hydrogencarbonate, at a temperature of from about 0°C to about 40°C, preferably at a temperature of from about 10°C to about 30°C, to provide the corresponding oxime of the formula XIV, or its salt. The solution of the oxime may be carried forward to the next step, if desired, without further purification.

The oxime of the formula XIV or its salt, in a solvent, for example a polar organic solvent such as an aliphatic alcohol or a carboxylic acid, for example methanol, ethanol, isopropanol or acetic acid, is then treated with hydrogen at a pressure of from about 300 kPa to about 1500 kPa, for example at about 500 kPa, in the presence of a palladium catalyst, for example palladium on carbon or palladium hydroxide, at a temperature of from about 20°C to about 50°C, for example at about 35°C, to provide the corresponding benzylamine of the formula XV, or its salt. Upon filtration to remove the catalyst, the solution may be used, if desired, without further purification for the next step.

The benzylamine of the formula XV or its salt is then treated with di-tert-butyl dicarbonate (Boc₂O), which is generally employed in a slight excess of from about 1 to about 1.2 molar equivalents, in a solvent, for example an aliphatic alcohol such as methanol, ethyl acetate, an ether such as tetrahydrofuran, a halogenated hydrocarbon such as dichloromethane, a mixture of two or more of such solvents or a mixture of one or more of such solvents with water, in the presence of a base, for example an alkali metal hydroxide, carbonate or alcoholate or a tertiary amine, such as sodium hydroxide or triethylamine, preferably at a temperature of from about 0°C to about 40°C, preferably at a temperature of from about 10°C to about 30°C, to provide the corresponding Boc-protected amine of the formula XVI, which may be used, if desired, without further purification for the next step.

The 3-(4-pyridyl)benzylcarbamic acid tert-butyl ester of the formula XVI in a solvent, for example an aliphatic alcohol such as ethanol or isopropanol, is then treated with hydrogen at a pressure of from about 2000 kPa to about 6000 kPa in the presence of a platinum catalyst, for example platinum on carbon or a platinum oxide, for example in the presence of 5% platinum on carbon, at a temperature of from about 20°C to about 80°C, for example at about 50°C, in the presence of an acid, for example a mineral acid such as hydrogen chloride or sulfuric acid. In a surprising aspect of the present invention, the Boc-protecting group survives the presence of acid in the reduction mixture. When the absorption of hydrogen is complete, for work-up the solvent can be removed in vacuo, followed by treatment of the mixture with aqueous base, for example aqueous sodium hydroxide, and extraction into an organic solvent, for example dichloromethane or toluene. The residue upon evaporation of the solvent, if desired, may then be treated with an acid, for example p-toluenesulfonic acid, to provide the desired piperidine of the formula VI as its salt, for example the salt with p-toluenesulfonic acid.

As outlined above, instead of from a pyridylbenzaldehyde of the formula XIII, by converting it into the oxime and hydrogenating the oxime function, the amine of the formula XV or its salt can further be prepared from a pyridylbenzonitrile of the formula XVII by reducing the nitrile function, for example by catalytic hydrogenation in the presence of a palladium catalyst such as palladium on charcoal or by means of a complex hydride reducing agent. The catalytic hydrogenation of the nitrile function is preferably performed in a solvent, for example an aliphatic alcohol such as methanol or a mixture of an aliphatic alcohol and water, at a temperature of from about 10°C to about 40°C, at a hydrogen pressure of from about 2000 kPa to about 6000 kPa, in the presence of an acid, for example a mineral acid such as hydrochloric acid. The reduction of the nitrile function by means of a complex hydride is preferably performed in a solvent, such as a protic solvent, for example an aliphatic alcohol like methanol or ethanol, when using a complex hydride like sodium borohydride, or an aprotic solvent, for example an ether like tetrahydrofuran, when using a complex hydride like lithium aluminium hydride, at a temperature of from about 0°C to about 50°C. The amine of the formula XV or its salt may be used, if desired, without further purification for the next step in which it is treated with di-tert-butyl dicarbonate as explained above. The starting compounds of the formulae XIII and XVII are commercially available or can be prepared by or analogously to procedures described in the literature, for example in WO 98/21210, Z.Y. Wang et al., Chin. Chem. Lett. 2003, 14, pages 13-16, or M.A. Massa et al., Bioorg. Med. Chem. Lett. 2001, 11, pages 1625-1628.

As indicated above, in the processes for the preparation of a compound of the formula VI from a compound of the formula XIII or XVII according to the present invention the intermediates can in an advantageous manner be taken to the next reaction step, if desired, without a separate purification step and intermediary isolation.

According to the processes of the present invention, the 4-[3-(tert-butoxycarbonylaminomethyl)phenyl]piperidines of the formula VI can be prepared in a considerably simpler manner and with the use of cheaper reagents than according to the previously described processes. Previously described processes for the preparation of 4-[3-(tert-butoxycarbonylaminomethyl)phenyl]piperidines include that shown in Scheme 6, disclosed in WO 2004/060884 (international patent application PCT/US2003/040653).

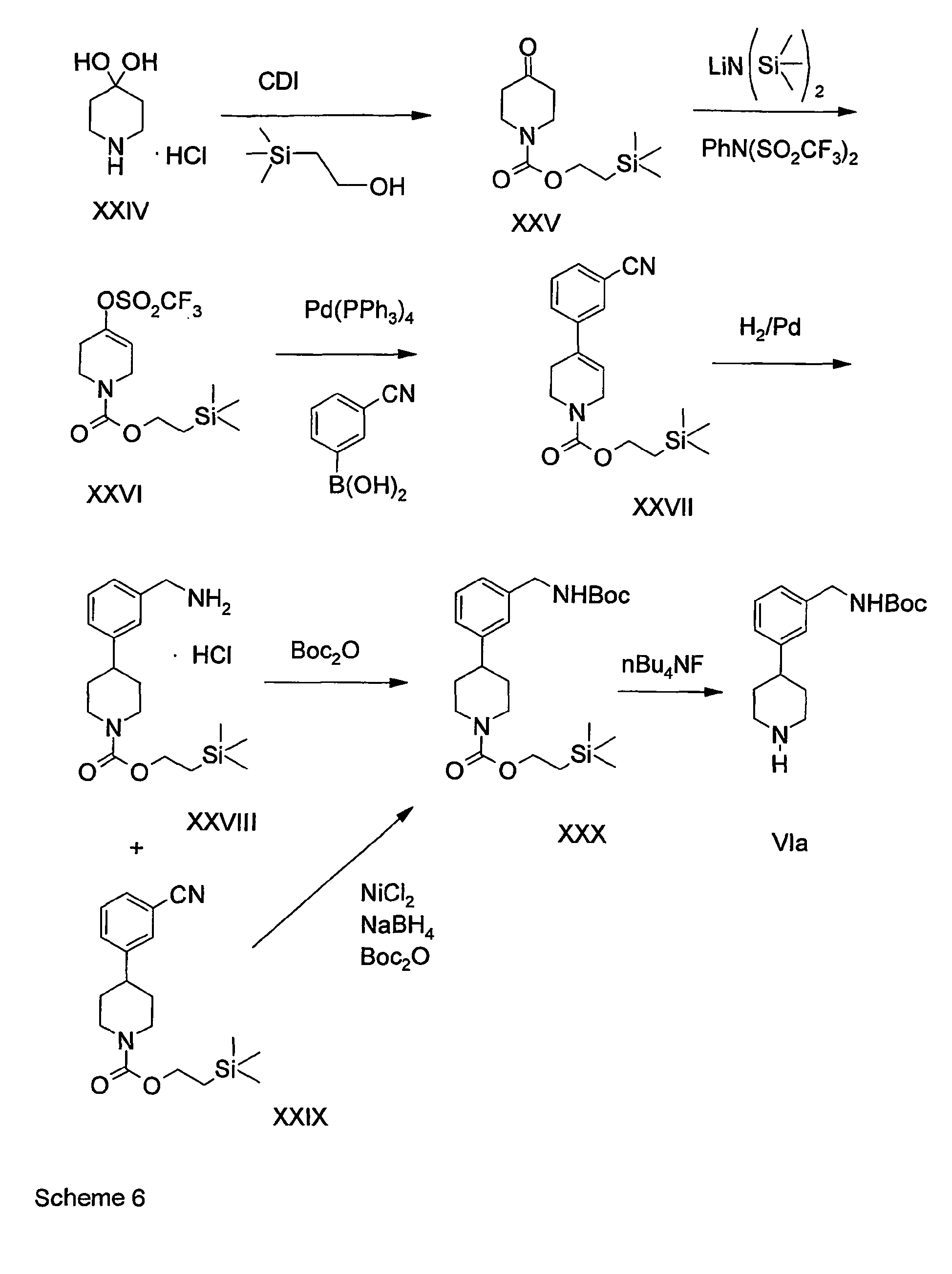

The first step of the previously described process, protection of the 4-piperidone derivative of the formula XXIV as the N-Teoc (2-(trimethylsilyl)ethoxycarbonyl) derivative of the formula XXV, utilizing in situ generation of the carbamate formed from 1,1'-carbonyldiimidazole (CDI) and 2-(trimethylsilyl)ethanol, requires a high vacuum distillation of the product at 156-160°C, as well as the use of the expensive reagent 2-(trimethylsilyl)ethanol. Enolization of the ketone of the formula XXV requires, at a temperature of -78°C, the reaction with lithium bis(trimethylsilyl)amide in tetrahydrofuran, followed by quenching of the enolate with N-phenyl-bis(trifluoromethanesulfonimide), another very expensive reagent, to give the vinyl triflate of the formula XXVI. The triflate of the formula XXVI is used in a Suzuki coupling with the expensive reagent 3-cyanophenylboronic acid in the presence of the expensive catalyst tetrakistriphenylphosphine palladium(0) to give the nitrile of the formula XXVII which must be purified by chromatography. Hydrogenation of both the nitrile function and the double bond using Pd/C in the presence of hydrogen chloride gives a mixture of the desired amine hydrochloride of the formula XXVIII and the partially reduced nitrile of the formula XXIX. The amine must be washed free of the nitrile and is then converted into the Boc-protected derivative of the formula XXX in the presence of di-tert-butyl dicarbonate (Boc₂O). The residual nitrile of the formula XXIX may be converted to the desired amine, and protected as the Boc derivative of the formula XXX in the presence of NiCl₂, a toxic reagent, Boc₂O, and NaBH₄. The Boc derivative of the formula XXX must be isolated and further purified by trituration with pentane, or it can alternatively be recrystallized from cyclohexane/hexane. Removal of the N-Teoc group with tetra-n-butylammonium fluoride at 50°C gives, after trituration with diethyl ether/pentane, the desired piperidine of the formula VIa, i.e., the compound of formula VI wherein R^{1'} is hydrogen.

The prior art process of preparing the piperidine of the formula VIa requires low temperature reaction conditions for one step, several chromatography and purification steps, isolation of intermediates, and the use of toxic and expensive reagents. Processes of the present invention, as described herein, provide the compounds of formula VI using less toxic and less expensive reagents, isolating fewer intermediates, and requiring fewer intermediate purification steps, thereby rendering these processes more suitable for the production of commercial quantities of such compounds under economical and technical aspects.

The invention is further explained and illustrated by the following exemplary procedures. These examples should in no way be construed, however, as limiting the scope of the invention.

### Example 1

### 4-[3-(tert-Butoxycarbonylaminomethyl)phenyl]piperidine

### a) 1-(3-Bromobenzyl)-2,2,5,5-tetramethyl-1-aza-2,5-disilacyclopentane

To a stirred suspension of 3-bromobenzylamine hydrochloride (310 g, 1.39 mol) in dichloromethane (2 L) is added triethylamine (437 g, 4.32 mol), followed by a solution of 1,2-bis(chlorodimethylsilyl)ethane (300 g, 1.39 mol) in dichloromethane (700 mL). The resulting suspension is stirred for 30 min and then filtered. The filtrate is concentrated in vacuo and pentane is added. After filtration, the solvent is removed in vacuo to yield the title compound (435 g, 95%) as a colorless oil. ¹H NMR (CDCl₃) δ = 7.4-7.1 (m, 4 H), 4.0 (s, 2 H), 0.8 (t, 4 H), 0.2 (s, 12 H).

### b) 3-(1-Benzyl-1,2,3,6-tetrahydropyridin-4-yl)benzylcarbamic acid tert-butyl ester

To a solution of 1-(3-bromobenzyl)-2,2,5,5-tetramethyl-1-aza-2,5-disilacyclopentane (435 g, 1.33 mol) in tetrahydrofuran (4 L) is added a 2.5 M solution of n-butyllithium (n-BuLi) (609 mL, 1.52 mol) at -60°C. After 30 min, a solution of 1-benzyl-4-piperidone (276 g, 1.46 mol) in tetrahydrofuran is added at that temperature. After stirring for a further 30 min, the reaction is quenched by addition of methanol and allowed to warm to room temperature. The solution is concentrated in vacuo to yield the crude alcohol as a sticky brown oil.

The crude residue from above is dissolved in dichloromethane (1.5 L). To this solution is added concentrated phosphoric acid (H₃PO₄) (1 equiv.) and the mixture is concentrated in vacuo. The yellowish phosphate is solidified by the addition of heptane and isolated by filtration.

The dried solid from above is added to concentrated H₃PO₄ (1.5 L) and the resulting mixture is heated to 100°C for two hours. After cooling to room temperature, the solution is diluted with water and extracted with ether. The aqueous layer is then basified with sodium hydroxide. The precipitate is isolated by filtration and is used in the next step without drying.

The solid from above is suspended in a mixture of methanol (4 L) and water (2 L) containing sodium hydroxide (10.0 g, 0.25 mol). Boc₂O (290 g, 1.33 mol) is added and the mixture is stirred overnight at room temperature. The solid is isolated by filtration and then dried in vacuo to yield the title compound (390 g, 76%) as a light yellow solid. ¹H NMR (CDCl₃) δ = 7.1-7.4 (m, 9 H), 6.03 (m, 1 H), 4.90 (m, 1 H), 4.25 (d, 2 H), 3.68 (s, 2 H), 3.18 (m, 2 H), 2.69 (m, 2 H), 2.57 (m, 2 H), 1.42 (s, 9 H). MS m/z 379 (M + H), 380 (M + 2H).

### c) 4-[3-(tert-Butoxycarbonylaminomethyl)phenyl]piperidine acetic acid salt

A mixture of 3-(1-benzyl-1,2,3,6-tetrahydropyridin-4-yl)benzylcarbamic acid tert-butyl ester (190 g, 0.50 mol), Pearlman's catalyst (Pd(OH)₂/C) (9.5 g) and acetic acid (60.2 g, 1.0 mol) in methanol is hydrogenated overnight at room temperature, at 345 kPa hydrogen pressure. After filtration and evaporation, the obtained oil is dissolved in diisopropyl ether which causes precipitation of the reaction product. After drying in vacuo, the pure title compound (159 g, 90%) is isolated as a yellowish solid. ¹H NMR (300 MHz, CDCl₃) δ = 7.1-7.4 (m, 4 H), 4.89 (m, 1 H), 4.29 (d, 2 H), 3.42 (m, 2 H), 2.86 (m, 2 H), 2.69 (m, 1 H), 2.05 (s, 3 H), 1.97 (m, 4 H), 1.45 (s, 9 H). MS m/z 291 (M + H), 292 (M + 2H).

### d) 4-[3-(tert-Butoxycarbonylaminomethyl)phenyl]piperidine

4-[3-(tert-Butoxycarbonylaminomethyl)phenyl]piperidine acetic acid salt (147 g, 0.42 mol) is dissolved in dilute hydrochloric acid and the solution extracted with ether. The aqueous phase is basified with sodium hydroxide and extracted with ether. The organic phase is concentrated in vacuo and the product is precipitated from pentane/ether yielding the title compound (105 g, 86%) as an off-white solid. ¹H NMR (300 MHz, CDCl₃) δ = 7.25 (m, 1 H), 7.07-7.13 (m, 3 H), 5.12 (m, 1 H), 4.29 (d, 2 H), 3.17 (m, 3 H), 2.72 (m, 2 H), 2.60 (m, 1 H), 1.81 (m, 2 H), 1.55-1.70 (m, 2 H), 1.46 (s, 9 H). MS (ESI) m/z 291 (M⁺ + 1, 100).

### Example 2

### 4-[3-(tert-Butoxycarbonylaminomethyl)phenyl]piperidine p-toluenesulfonic acid salt

### a) 3-(4-Pyridyl)benzaldehyde oxime hydrochloride

3-(4-Pyridyl)benzaldehyde (100 g, 546 mmol) is dissolved in methanol (500 mL) and then added to a solution of hydroxylamine hydrochloride (40.2 g, 573 mmol) in methanol (260 mL). After complete conversion a small sample of the reaction mixture is concentrated in vacuo. MS (DCI) m/z 181.1, 199.1, 200.2. ¹H-NMR (400 MHz, DMSO-d₆) δ = 7.56 (t, J = 7.7 Hz, 1 H), 7.70-7.75 (m, 3 H), 7.79-7.83 (m, 1 H), 7.97-7.99 (m, 1 H), 8.25 (s, 1 H), 8.65-8.67 (m, 2 H), 11.4 (s, 1 H).

### b) 3-(4-Pyridyl)benzylamine hydrochloride

The methanolic solution of 3-(4-pyridyl)benzaldehyde oxime hydrochloride from above is added to palladium on charcoal (13.1 g, 5% Pd/C). The mixture is stirred at 35°C and 500 kPa hydrogen pressure until the consumption of hydrogen ceased. The catalyst is filtered off. HPLC showed complete conversion. A small sample of the reaction solution is concentrated in vacuo. MS (DCI) m/z 185.2, 186.2. ¹H-NMR (400 MHz, DMSO-d₆) δ = 4.10 (s, 2 H), 7.54-7.61 (m, 2 H), 7.74-7.77 (m, 2 H), 7.83 (dt, J = 2.1, 6.6 Hz, 1 H), 8.02-8.05 (m, 1 H), 8.53 (bs, 2 H), 8.67-8.70 (m, 2 H).

### c) 3-(4-Pyridyl)benzylcarbamic acid tert-butyl ester

The methanolic solution of 3-(4-pyridyl)benzylamine hydrochloride from above is added to NaHCO₃ (109 g, 1.30 mol). The mixture is stirred until the evolution of gas ceased. A solution of di-tert-butyl dicarbonate (139 g, 623 mmol) in methanol (450 mL) is added. The mixture is stirred at room temperature until HPLC showed complete conversion. Three quarters of the methanol is distilled off in vacuo. Water (500 mL) is added and the remaining methanol is distilled off in vacuo. The aqueous layer is extracted with dichloromethane. The solvent is removed in vacuo and the residue is dissolved in ethanol (100 mL). A small sample of the reaction solution is concentrated in vacuo. MS (DCI) m/z 229.3, 285.4, 286.4, 287.4. ¹H-NMR (400 MHz, CDCl₃) δ = 1.48 (s, 9 H), 4.37-4.44 (m, 2 H), 4.95 (bs, 1 H), 7.34-7.39 (m, 1 H), 7.42-7.48 (m, 1 H), 7.48-7.51 (m, 2 H), 7.52-7.56 (m, 2 H), 8.64-8.67 (m, 2 H).

### d) 4-[3-(tert-Butoxycarbonylaminomethyl)phenyl]piperidine p-toluenesulfonic acid salt

Charcoal (17 g) is added to half of the above ethanolic solution of 3-(4-pyridyl)benzyl-carbamic acid tert-butyl ester from above. The mixture is stirred at room temperature, filtered and the filter is washed with additional ethanol (260 mL). The obtained solution is added to platinum on charcoal (44.7 g, 5% Pt/C). A mixture of conc. hydrochloric acid (31 g) and ethanol (250 mL) is added. The reaction mixture is diluted with ethanol (500 mL) and then hydrogenated at 50°C and a hydrogen pressure of 3000 kPa until the consumption of hydrogen ceased. The catalyst is filtered off. HPLC showed complete conversion. Three quarters of the ethanol is removed in vacuo. A mixture of water (310 mL) and sodium hydroxide solution (33%, 33 g) is added before the remainder of ethanol is removed in vacuo. The aqueous solution is extracted with dichloromethane. The solvent is removed in vacuo and the residue is dissolved in isopropanol (65 mL) and diisopropyl ether (2.8 L). The solution is cooled to 0°C and then a solution of p-toluenesulfonic acid (57.5 g) in isopropanol (260 mL) is added. The mixture is subsequently stirred at 0°C for 1 h. The precipitate is filtered off, washed with diisopropyl ether and dried in vacuo to give 94.5 g of the title compound as a white solid (overall yield over 4 steps: 77%). MS (ESI) m/z 291.26, 292.28, 293.28. ¹H-NMR (400 MHz, DMSO-d₆) δ = 1.39 (s, 9 H), 1.70-1.95 (m, 4 H), 2.29 (s, 3 H), 2.76-2.85 (m, 1 H), 2.96-3.05 (m, 2 H), 3.25-3.40 (m, 1 H), 4.08-4.13 (m, 2 H), 7.05-7.14 (m, 4 H), 7.23-7.30 (m, 1 H), 7.36-7.40 (m, 1 H), 7.46-7.56 (m, 2 H), 8.37 (bs, 2 H).

### Example 3

### 3-(4-Pyridyl)benzylamine dihydrochloride

Hydrochloric acid (34.0 g, 30 % w/w) and palladium on charcoal (12.0 g, 10% Pd/C) are added to a solution of 3-(4-pyridyl)benzonitrile (25.0 g, 139 mmol) in methanol/water (1:1, 250 mL). The mixture is stirred at room temperature and a hydrogen pressure of 3000 kPa until the consumption of hydrogen ceases. The catalyst is filtered off. HPLC shows complete conversion. A sample of the reaction solution is concentrated in vacuo. MS (DCI) m/z 185.3. ¹H-NMR (400 MHz, DMSO-d₆) δ = 4.15 (s, 2 H), 7.62 - 7.66 (m, 1 H), 7.72 (d, J = 7.6 Hz, 1 H), 7.99 (d, J = 7.8 Hz, 1 H), 8.20 (s, 1 H), 8.41 (d, J = 6.5 Hz, 2 H), 8.92 (d, J = 6.5 Hz, 2 H).

### Example 4

### 4-[3-(tert-Butoxycarbonylaminomethyl)phenyl]-1-[5-bromo-2-furanoyl]piperidine

To a suspension of 4-[3-(tert-butoxycarbonylaminomethyl)phenyl]piperidine p-toluenesulfonic acid salt (185 g, 0.40 mol) in toluene is added aqueous sodium hydroxide (1.1 equiv.) and the mixture is stirred at room temperature for 30 min. The phases are separated and the toluene layer is washed with water. It is dried azeotropically by distilling off part of the toluene. To this solution is added triethylamine (72 mL, 0.52 mol). This mixture is used directly in the next step.

To a refluxing suspension of 5-bromo-2-furoic acid (80.8 g, 0.44 mol) in toluene (400 mL) is added thionyl chloride (37 mL, 0.52 mol). The mixture is refluxed for a further 30 min, then concentrated by distilling off part of the solvent. The obtained solution is cooled to room temperature and added to the solution of amines from above. After stirring for one hour, water is added, the organic layer is separated and diluted with heptane. The solid is filtered off and dried in vacuo. Recrystallization of the crude product from isopropanol/water yields the pure title compound (143 g, 77%) as an off-white solid. ¹H NMR (400 MHz, DMSO-d₆) δ = 7.34 (m, 1 H), 7.24 (t, J = 7.6 Hz, 1 H), 7.12 (m, 2 H), 7.06 (m, 1 H), 7.02 (d, J = 3.4 Hz, 1 H), 6.75 (d, J = 3.4 Hz, 1 H), 4.40 (bs, 2 H), 4.10 (d, J = 6.1 Hz, 2 H), 3.25-2.90 (bs, 2 H), 2.87-2.79 (m, 1 H), 1.83 (m, 2 H), 1.63-1.53 (m, 2 H), 1.39 (s, 9 H). MS (ESI) m/z 363 (M⁺ - 100, 100).

### Example 5

### 4-[3-(tert-Butoxycarbonylaminomethyl)phenyl]-1-[5-(2-fluorophenylethynyl)-2-furanoyl]piperidine

A solution of (2-fluorophenylethynyl)trimethylsilane (68.0 g, 0.35 mol) in methanol (40 mL) is added to a suspension of K₂CO₃ (0.76 g, 5.51 mmol) in methanol (40 mL). The solution is stirred for 30 min at room temperature and then added to a mixture of 4-[3-(tert-butoxycarbonylaminomethyl)phenyl]-1-[5-bromo-2-furanoyl]piperidine (126 g, 0.27 mol), Pd(PPh₃)₂Cl₂ (3.90 g, 5.56 mmol), Cul (1.0 g, 5.25 mmol) and triethylamine (94 mL) in tetrahydrofuran (550 mL). The reaction mixture is refluxed for one hour. An aqueous solution of the trisodium salt of trimercaptotriazine (0.2 equiv.) is introduced and the mixture refluxed for an additional hour. After cooling to room temperature, brine is added, the organic phase separated and washed successively with dilute hydrochloric acid and sodium carbonate solution. The organic phase is refluxed with charcoal for 30 min, filtered and concentrated in vacuo. The resulting brown oil is dissolved in dichloromethane (400 mL) and stirred overnight at room temperature with Deloxan^{®} THP II (135 g). After filtration the solvent is evaporated and the product crystallized from isopropanol/diisopropyl ether to yield the title compound (74.6 g, 55%) as a yellow powder. ¹H NMR (300 MHz, CDCl₃) δ = 7.53 (td, 1 H), 7.41-7.28 (m, 2 H), 7.18-7.08 (m, 5 H), 7.04 (d, 1 H, J = 3.6 Hz), 6.76 (d, 1 H, J = 3.6 Hz), 4.83 (bs, 1 H), 4.72 (bm, 2 H), 4.31 (d, 2 H, J = 5.6 Hz), 3.1 (bs, 1 H), 2.82 (m, 2 H), 1.96 (m, 2 H), 1.79 (td, 2 H, J = 12.8, 4.0 Hz), 1.46 (s, 9 H). MS (ESI) m/z 503 (M⁺ + 1, 100).

### Example 6

### 4-[3-(Aminomethyl)phenyl]-1-[5-(2-fluorophenylethynyl)-2-furanoyl]piperidine methanesulfonic acid salt

To a solution of 4-[3-(tert-butoxycarbonylaminomethyl)phenyl]-1-[5-(2-fluorophenylethynyl)-2-furanoyl]piperidine (67.4 g, 0.13 mol) in isopropanol (350 mL) is added methanesulfonic acid (13.5 g, 0.14 mol) at 70°C. The mixture is stirred at that temperature for three hours and then cooled slowly to room temperature. The precipitate is filtered off, washed with acetone and dried in vacuo. Recrystallization of the crude product from aqueous isopropanol/diisopropyl ether yields the analytically pure title compound (56.5 g, 84%). ¹H NMR (300 MHz, DMSO-d₆) δ = 8.12 (bs, 3 H), 7.67 (tm, 1 H, J = 7.5 Hz), 7.54 (qm, 1 H, J = 7.0 Hz), 7.42-7.26 (m, 6 H), 7.10 (m, 2 H), 4.34 (bs, 2 H), 4.02 (q, 2 H, J = 5.5 Hz), 3.5-2.7 (bs, 2 H), 2.89 (m, 1 H), 2.34 (s, 3 H), 1.87 (m, 2 H), 1.65 (m, 2 H). MS (ESI) m/z 403 (M⁺ + 1, 100).

### Example 7

### 4-[5-(tert-Butoxycarbonylaminomethyl)-2-fluorophenyl]piperidine

The title compound is prepared analogously to the preparation of 4-[3-(tert-butoxycarbonylaminomethyl)phenyl]piperidine described in example 1. Briefly, in step a) 3-bromo-4-fluorobenzylamine hydrochloride is converted into 1-(3-bromo-4-fluorobenzyl)-2,2,5,5-tetramethyl-1-aza-2,5-disilacyclopentane. In step b) the latter compound is reacted with n-BuLi and 1-benzyl-4-piperidone at -75°C to -70°C to give 1-[3-(1-benzyl-4-hydroxypiperidin-4-yl)-4-fluorobenzyl]-2,2,5,5-tetramethyl-1-aza-2,5-disilacyclopentane which, by treatment with phosphoric acid, is converted into 3-(1-benzyl-4-hydroxypiperidin-4-yl)-4-fluorobenzylamine (as phosphoric acid salt) and subsequently into 3-(1-benzyl-1,2,3,6-tetrahydropyridin-4-yl)-4-fluorobenzylamine. Reaction with Boc₂O then gives 3-(1-benzyl-1,2,3,6-tetrahydropyridin-4-yl)-4-fluorobenzylcarbamic acid tert-butyl ester. In step c) the latter compound is hydrogenated to give 4-[5-(tert-butoxycarbonylaminomethyl)-2-fluorophenyl]piperidine acetic acid salt which is purified by trituration with tert-butyl methyl ether. In step d) the said acetic acid salt is converted into 4-[5-(tert-butoxycarbonylaminomethyl)-2-fluorophenyl]piperidine which is precipitated from pentane as a white solid. ¹H NMR (300 MHz, CDCl₃) δ = 7.10 (m, 2 H), 6.95 (m, 1 H), 4.84 (bs, 1 H), 4.26 (d, 2 H, J = 5.6 Hz), 3.18 (d, 2 H, J = 12.0 Hz), 2.95 (m, 1 H), 2.77 (m, 2 H), 1.81 -1.48 (m, 5 H), 1.46 (s, 9 H). MS (ESI) m/z 309 (M + H).

## Claims

1. A process for the preparation of a compound of the formula I, 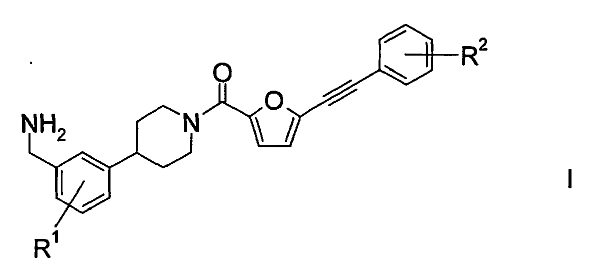 wherein
R¹ is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, (C₁-C₈)-alkoxy, (C₃-C₁₀)-cycloalkoxy, (C₆-C₁₄)-aryloxy, di((C₁-C₈)-alkyl)amino, di((C₃-C₁₀)-cycloalkyl)amino or di((C₆-C₁₄)-aryl)amino; and
R² is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, 5-membered to 10-membered heteroaryl comprising 1, 2 or 3 identical or different ring heteroatoms chosen from nitrogen, oxygen and sulfur, (C₁-C₈)-alkoxy, (C₃-C₁₀)-cycloalkoxy, (C₆-C₁₄)-aryloxy, 5-membered to 10-membered heteroaryloxy comprising 1, 2, or 3 identical or different ring heteroatoms chosen from nitrogen, oxygen and sulfur, di((C₁-C₈)-alkyl)amino, di-((C₃-C₁₀)-cycloalkyl)amino or di((C₆-C₁₄)-aryl)amino;
or its salt,
comprising
a) treating a phenylethynylsilane compound of the formula II, 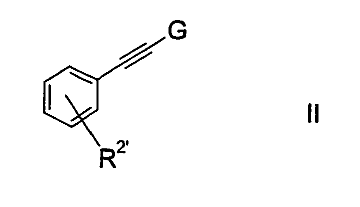 wherein G is trimethylsilyl, triethylsilyl, tri-isopropylsilyl, or dimethyl-tert-butylsilyl, and R^{2'} is R² as defined above or is a protected derivative thereof or precursor moiety thereto, in a solvent with an alkali metal hydroxide, carbonate or alcoholate or an alkaline earth metal alcoholate, to form a solution of a phenylethyne of the formula III; 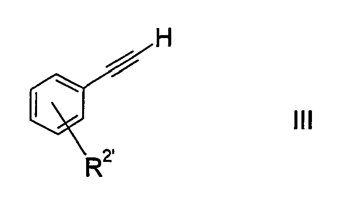 and
b) combining the resulting solution of the phenylethyne with a compound of the formula IV, 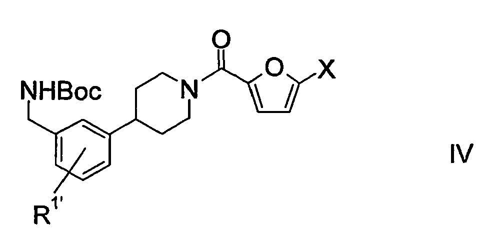 wherein X is bromo or iodo, R^{1'} is R¹ as defined above or is a protected derivative thereof or precursor moiety thereto, and Boc is tert-butoxycarbonyl, in the presence of homogeneous palladium catalyst, a cuprous salt, and a hindered amine in a solvent to form a protected compound of the formula V, 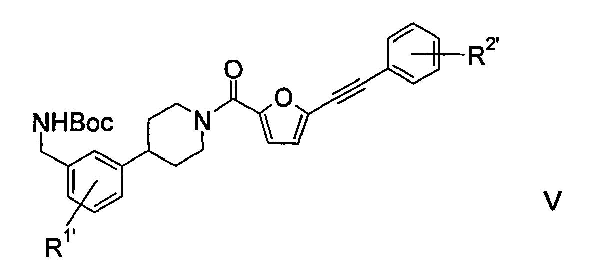
wherein R^{1'} and R^{2'} are R¹ and R², respectively, as defined above or are a protected derivative thereof or precursor moiety thereto, and Boc is tert-butoxycarbonyl, or its salt.

2. A process according to claim 1 for the preparation of 4-[3-(aminomethyl)phenyl]-1-[5-(2-fluorophenylethynyl)-2-furanoyl]piperidine of the formula la, 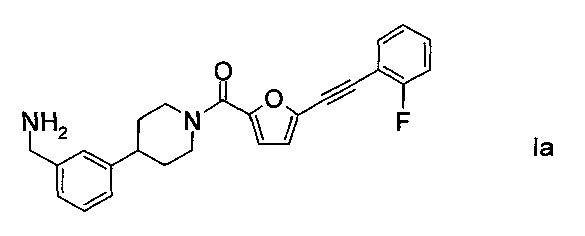 or its salt,
comprising
a) treating a (2-fluorophenylethynyl)silyl compound of the formula IIa, 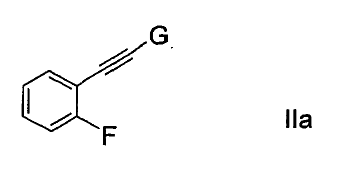 wherein G is trimethylsilyl, triethylsilyl, tri-isopropylsilyl, or dimethyl-tert-butylsilyl, in a solvent with an alkali metal hydroxide, carbonate or alcoholate or an alkaline earth metal alcoholate to form a solution of 2-fluorophenylethyne; and
b) combining the resulting solution of 2-fluorophenylethyne with a compound of the formula IVa, 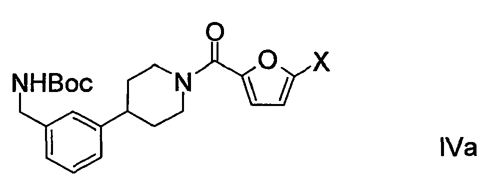 wherein X is bromo or iodo, and Boc is tert-butoxycarbonyl, in the presence of homogeneous palladium catalyst, a cuprous salt, and a hindered amine in a solvent to form 4-[3-(tert-butoxycarbonylaminomethyl)phenyl]-1-[5-(2-fluorophenylethynyl)-2-furanoyl]piperidine of the formula Va, 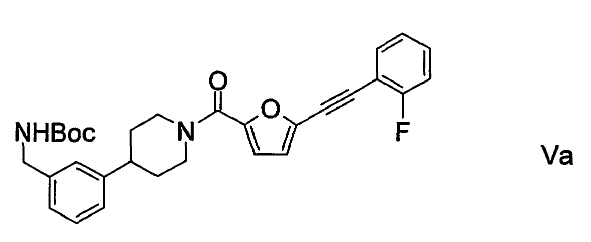
wherein Boc is tert-butoxycarbonyl.

3. A process according to claim 1 or 2 wherein G is trimethylsilyl.

4. A process according to any one of claims 1 to 3 wherein X is bromo.

5. A process according to any one of claims 1 to 4, further comprising removal of the tert-butoxycarbonyl group and other protecting groups and/or conversion of precursor moieties present in a compound of the formula V or Va to provide a compound of the formula I or la or its salt.

6. A process according to any one of claims 1 to 5, further comprising removal of the tert-butoxycarbonyl group from a compound of the formula V or Va in the presence of an acid to provide a compound of the formula I or la or its salt.

7. A process according to any one of claims 1 to 6, further comprising removal of the tert-butoxycarbonyl group from 4-[3-(tert-butoxycarbonylaminomethyl)phenyl]-1-[5-(2-fluorophenylethynyl)-2-furanoyl]piperidine in the presence of methanesulfonic acid to provide the methanesulfonic acid salt of 4-[3-(aminomethyl)phenyl]-1-[5-(2-fluorophenylethynyl)-2-furanoyl]piperidine.

8. A process for the preparation of a compound of the formula IV, 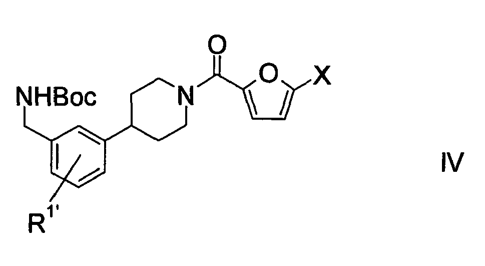 wherein
R^{1'} is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, (C₁-C₈)-alkoxy, (C₃-C₁₀)-cycloalkoxy, (C₆-C₁₄)-aryloxy, di((C₁-C₈)-alkyl)amino, di((C₃-C₁₀)-cycloalkyl)amino or di((C₆-C₁₄)-aryl)amino, or a protected derivative thereof or precursor moiety thereto; and
X is bromo or iodo,
comprising
a) activating 5-bromo-2-furoic acid or 5-iodo-2-furoic acid to provide an activated form of 5-bromo-2-furoic acid or 5-iodo-2-furoic acid; and
b) combining the activated form of 5-bromo-2-furoic acid or 5-iodo-2-furoic acid with a compound of the formula VI, 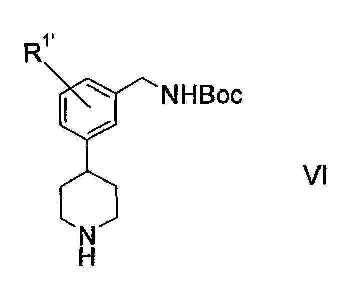
wherein R^{1'} is as defined for formula IV and Boc is tert-butoxycarbonyl, in a solvent.

9. A process according to claim 8 wherein R^{1'} is hydrogen.

10. A process according to claim 8 or 9 for the preparation of the compound of the formula IVc, 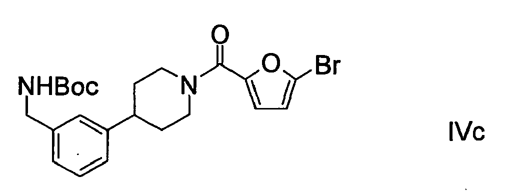 wherein Boc is tert-butoxycarbonyl,
comprising
a) treating 5-bromo-2-furoic acid with thionyl chloride to provide 5-bromo-2-furoyl chloride, and
b) combining the 5-bromo-2-furoyl chloride with the compound of the formula VIa 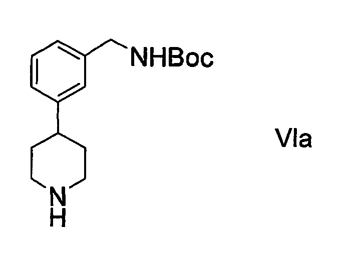
wherein Boc is tert-butoxycarbonyl, in a solvent in the presence of base.

11. A process for the preparation of a compound of the formula VI 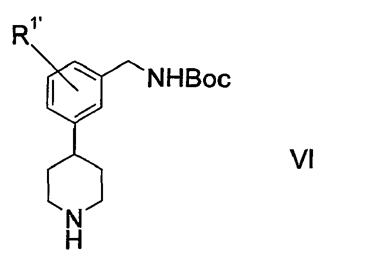
wherein R^{1'} is H, F, CF₃, OCF₃, (C₁-C₈)=alkyl, (C₃-C₁₀)-cycloalkyl 3-membered to 10-membered heterocycloalkyl comprising 1, 2 or 3 identical or different ring heteroatoms chosen from nitrogen, oxygen and sulfur, (C₆-C₁₄)-aryl, (C₁-C₈)-alkoxy, (C₃-C₁₀)-cycloalkoxy, (C₆-C₁₄)-aryloxy, di((C₁-C₈)-alkyl)amino, di((C₃-C₁₀)-cycloalkyl)amino or di((C₆-C₁₄)-aryl)amino, or a protected derivative thereof or precursor moiety thereto; and
Boc is tert-butoxycarbonyl,
or its salt,
comprising
a) treating a salt of a 3-halobenzylamine of the formula VII, 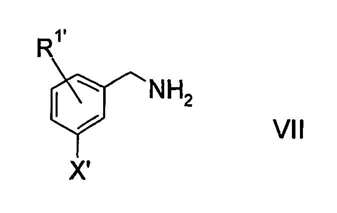 with 1,2-bis(chlorodimethylsilyl)ethane in a solvent in the presence of a base to provide a 1-(3-halobenzyl)-2,2,5,5-tetramethyl-1-aza-2,5-disilacyclopentane of the formula VIII; 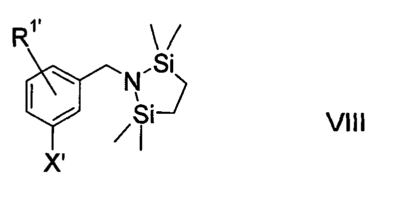
b) treating the 1-(3-halobenzyl)-2,2,5,5-tetramethyl-1-aza-2,5-disilacyclopentane of the formula VIII in a solvent with an alkyllithium compound and 1-benzyl-4-piperidone at a temperature of from about -80°C to about -40°C to provide an alcohol of the formula IX; 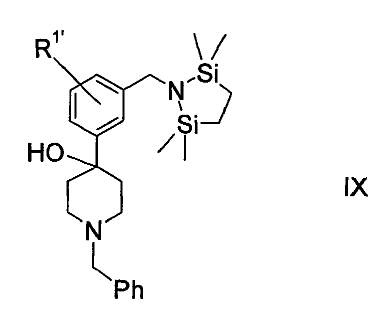
c) treating the alcohol of the formula IX with an acid in a solvent to provide a hydroxypiperidinylbenzylamine of the formula X 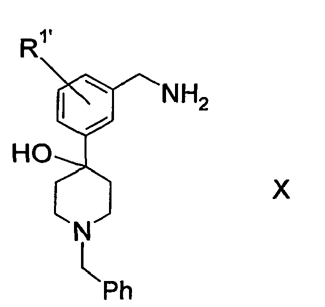 as the salt of the acid;
d) treating the hydroxypiperidinylbenzylamine of the formula X or its salt with a concentrated, non-oxidizing acid at a temperature of from about 70°C to about 150°C, followed by alkalinization to provide an olefin of the formula XI; 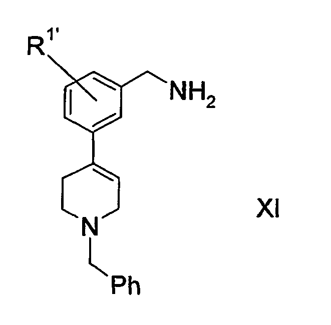
e) treating the olefin of the formula XI with di-tert-butyl dicarbonate in a solvent in the presence of a base to provide a protected amine of the formula XII 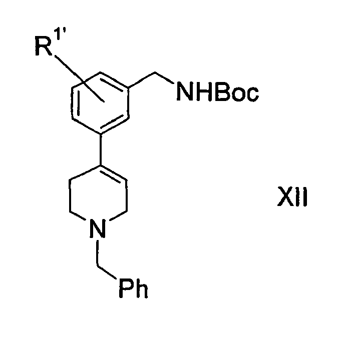
wherein Boc is tert-butoxycarbonyl, and where Ph in the formulae IX, X, XI and XII is phenyl, X' in the formulae VII and VIII is bromo or iodo, and R^{1'} in the formulae VII, VIII, IX, X, XI and XII is as defined for formula VI.

12. A process according to claim 11 for the preparation of 4-[3-(tert-butoxycarbonylaminomethyl)phenyl]piperidine or its salt,
comprising
a) treating 3-bromobenzylamine hydrochloride or 3-iodobenzylamine hydrochloride with 1,2-bis(chlorodimethylsilyl)ethane in a halogenated aliphatic hydrocarbon in the presence of a tertiary amine to provide the corresponding 1-(3-halobenzyl)-2,2,5,5-tetramethyl-1-aza-2,5-disilacyclopentane, where halo is bromo or iodo;
b) treating the 1-(3-halobenzyl)-2,2,5,5-tetramethyl-1-aza-2,5-disilacyclopentane in an ether with an alkyllithium compound and 1-benzyl-4-piperidone at a temperature of from about -80°C to about -40°C to provide 1-[3-(1-benzyl-4-hydroxypiperidin-4-yl)benzyl]-2,2,5,5-tetramethyl-1-aza-2,5-disilacyclopentane;
c) treating the 1-[3-(1-benzyl-4-hydroxypiperidin-4-yl)benzyl]-2,2,5,5-tetramethyl-1-aza-2,5-disilacyclopentane with an inorganic acid in a halogenated hydrocarbon to provide 3-(1-benzyl-4-hydroxypiperidin-4-yl)benzylamine as the salt of the inorganic acid;
d) treating the 3-(1-benzyl-4-hydroxypiperidin-4-yl)benzylamine with a concentrated, non-oxidizing acid at a temperature of from about 70°C to about 150°C, followed by alkalinization to provide 3-(1-benzyl-1,2,3,6-tetrahydropyridin-4-yl)benzylamine; and
e) treating the 3-(1-benzyl-1,2,3,6-tetrahydropyridin-4-yl)benzylamine with di-tert-butyl dicarbonate in an aliphatic alcohol, ethyl acetate, an ether, a halogenated hydrocarbon, a mixture of two or more of such solvents or a mixture of one or more of such solvents with water, in the presence of an alkali metal hydroxide, carbonate or alcoholate or a tertiary amine to provide 3-(1-benzyl-1,2,3,6-tetrahydropyridin-4-yl)benzylcarbamic acid tert-butyl ester.

13. A process according to claims 11 or 12, further comprising treating the amine of the formula XII with hydrogen in a solvent in the presence of a palladium catalyst and in the presence of an acid to provide a compound of the formula VI or its salt.

14. A process for the preparation of a compound of the formula VI, 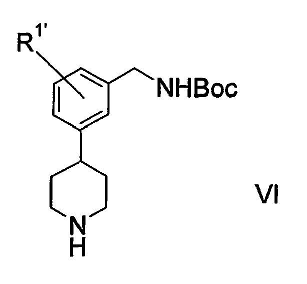
wherein R^{1'} is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, (C₁-C₈)-alkoxy, (C₃-C₁₀)-cycloalkoxy, (C₆-C₁₄)-aryloxy, di((C₁-C₈)-alkyl)amino, di((C₃-C₁₀)-cycloalkyl)amino or di((C₆-C₁₄)-aryl)amino, or a protected derivative thereof or precursor moiety thereto; and
Boc is tert-butoxycarbonyl,
or its salt,
comprising
a) treating a 3-(4-pyridyl)benzaldehyde of the formula XIII, 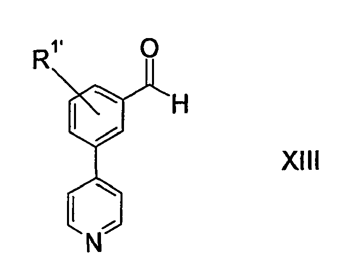 with hydroxylamine or a salt of hydroxylamine in a solvent to provide an oxime of the formula XIV or its salt; 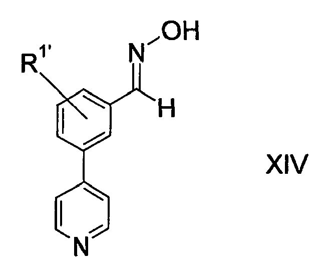
b) treating the oxime of the formula XIV or its salt with hydrogen in a solvent in the presence of a palladium catalyst to provide a 3-(4-pyridyl)benzylamine of the formula XV or its salt; 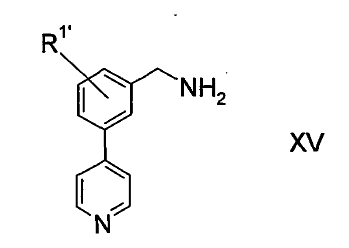
c) treating the 3-(4-pyridyl)benzylamine of the formula XV or its salt with di-tert-butyl dicarbonate in a solvent in the presence of a base to provide a Boc-protected 3-(4-pyridyl)benzylamine of the formula XVI, 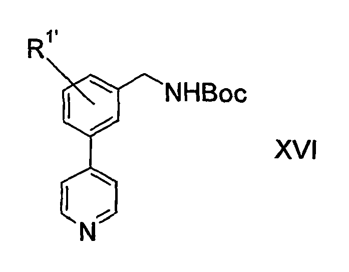 wherein Boc is tert-butoxycarbonyl; and
d) treating the Boc-protected 3-(4-pyridyl)benzylamine of the formula XVI with hydrogen in a solvent in the presence of a platinum catalyst and in the presence of an acid to provide a compound of the formula VI or its salt;
where R^{1'} in the formulae XIII, XIV, XV and XVI is as above defined for formula VI.

15. A process according to claim 14 for the preparation of 4-[3-(tert-butoxycarbonylaminomethyl)phenyl]piperidine or its salt,
comprising
a) treating 3-(4-pyridyl)benzaldehyde with hydroxylamine or a salt thereof in an aliphatic alcohol at a temperature of from about 0°C to about 40°C to provide 3-(4-pyridyl)benzaldehyde oxime or its salt;
b) treating the 3-(4-pyridyl)benzaldehyde oxime or its salt with hydrogen at a pressure of from about 300 kPa to about 1500 kPa in an aliphatic alcohol in the presence of a palladium catalyst at a temperature of from about 20°C to about 50°C to provide 3-(4-pyridyl)benzylamine or its salt;
c) treating the 3-(4-pyridyl)benzylamine or its salt with di-tert-butyl dicarbonate in an aliphatic alcohol, ethyl acetate, an ether, a halogenated hydrocarbon, a mixture of two or more of such solvents or a mixture of one or more of such solvents with water, in the presence of an alkali metal hydroxide, carbonate or alcoholate or a tertiary amine to provide 3-(4-pyridyl)benzylcarbamic acid tert-butyl ester; and
d) treating the 3-(4-pyridyl)benzylcarbamic acid tert-butyl ester with hydrogen in a aliphatic alcohol at a pressure of from about 2000 kPa to about 6000 kPa in the presence of a platinum catalyst and in the presence of an acid.

16. A process for the preparation of a compound of the formula VI 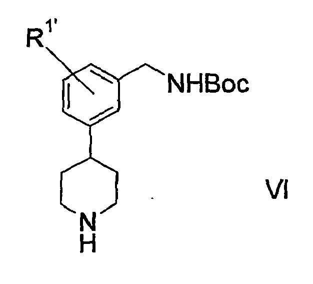
wherein R^{1'} is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, (C₁-C₈)-alkoxy, (C₃-C₁₀)-cycloalkoxy, (C₆-C₁₄)-aryloxy, di((C₁-C₈)-alkyl)amino, di((C₃-C₁₀)-cycloalkyl)amino or di((C₆-C₁₄)-aryl)amino, or a protected derivative thereof or precursor moiety thereto; and
Boc is tert-butoxycarbonyl,
or its salt,
comprising
a) treating a 3-(4-pyridyl)benzonitrile of the formula XVII, 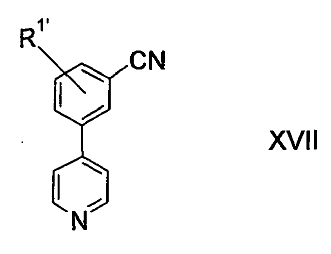 with hydrogen in the presence of a palladium catalyst and in the presence of an acid, or with a complex hydride, in a solvent to provide a 3-(4-pyridyl)benzylamine of the formula XV or its salt; 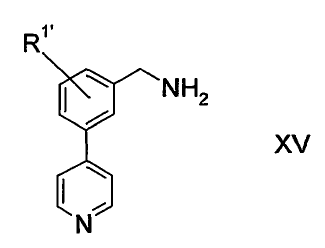
b) treating the 3-(4-pyridyl)benzylamine of the formula XV or its salt with di-tert-butyl dicarbonate in a solvent in the presence of a base to provide a Boc-protected 3-(4-pyridyl)benzylamine of the formula XVI, 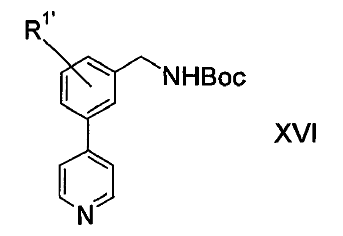 wherein Boc is tert-butoxycarbonyl; and
c) treating the Boc-protected 3-(4-pyridyl)benzylamine of the formula XVI with hydrogen in a solvent in the presence of a platinum catalyst and in the presence of an acid to provide a compound of the formula VI or its salt;
where R^{1'} in the formula XV, XVI and XVII is as above defined for formula VI.

17. A process according to claim 16 for the preparation of 4-[3-(tert-butoxycarbonylaminomethyl)phenyl]piperidine or its salt,
comprising
a) treating 3-(4-pyridyl)benzonitrile with hydrogen in the presence of a palladium catalyst at a pressure of from about 2000 kPa to about 6000 kPa in an aliphatic alcohol or a mixture of an aliphatic alcohol and water, or with a complex hydride in an aliphatic alcohol or an ether, at a temperature of from about 0°C to about 50°C, to provide 3-(4-pyridyl)benzylamine or its salt;
b) treating the 3-(4-pyridyl)benzylamine or its salt with di-tert-butyl dicarbonate in an aliphatic alcohol, ethyl acetate, an ether, a halogenated hydrocarbon, a mixture of two or more of such solvents or a mixture of one or more of such solvents with water, in the presence of an alkali metal hydroxide, carbonate or alcoholate or a tertiary amine, to provide 3-(4-pyridyl)benzylcarbamic acid tert-butyl ester; and
c) treating the 3-(4-pyridyl)benzylcarbamic acid tert-butyl ester with hydrogen in an aliphatic alcohol at a pressure of from about 2000 kPa to about 6000 kPa in the presence of a platinum catalyst and in the presence of an acid.

18. A compound of the formula VIII, 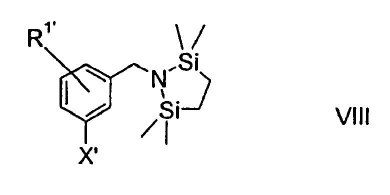 wherein R^{1'} is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, (C₁-C₈)-alkoxy, (C₃-C₁₀)-cycloalkoxy, (C₆-C₁₄)-aryloxy, di((C₁-C₈)-alkyl)amino, di((C₃-C₁₀)-cycloalkyl)amino or di((C₆-C₁₄)-aryl)amino, or a protected derivative thereof or precursor moiety thereto; and X' is bromo or iodo; the compound 1-(3-bromobenzyl)-2,2,5,5-tetramethyl-1-aza-2,5-disilacyclopentane being excluded.

19. A compound according to claim 18 which is 1-(3-iodobenzyl)-2,2,5,5-tetramethyl-1-aza-2,5-disilacyclopentane.

20. A compound of the formula IX, 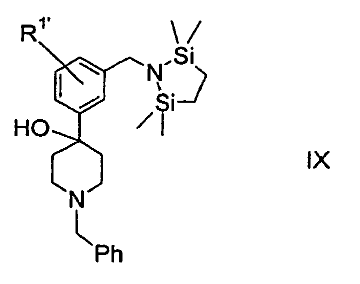 wherein R^{1'} is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, (C₁-C₈)-alkoxy, (C₃-C₁₀)-cycloalkoxy, (C₆-C₁₄)-aryloxy, di((C₁-C₈)-alkyl)amino, di((C₃-C₁₀)-cycloalkyl)amino or di((C₆-C₁₄)-aryl)amino, or a protected derivative thereof or precursor moiety thereto; and Ph is phenyl.

21. A compound according to claim 20 which is 1-[3-(1-benzyl-4-hydroxypiperidin-4-yl)benzyl]-2,2,5,5-tetramethyl-1-aza-2,5-disilacyclopentane.

22. A compound of the formula X, 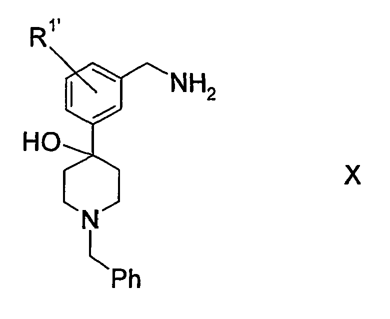 wherein R^{1'} is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, (C₁-C₈)-alkoxy, (C₃-C₁₀)-cycloalkoxy, (C₆-C₁₄)-aryloxy, di((C₁-C₈)-alkyl)amino, di((C₃-C₁₀)-cycloalkyl)amino or di((C₆-C₁₄)-aryl)amino, or a protected derivative thereof or precursor moiety thereto; and Ph is phenyl, or its salt.

23. A compound according to claim 22 which is 3-(1-benzyl-4-hydroxypiperidin-4-yl)benzylamine or its salt.

24. A compound of the formula XI, 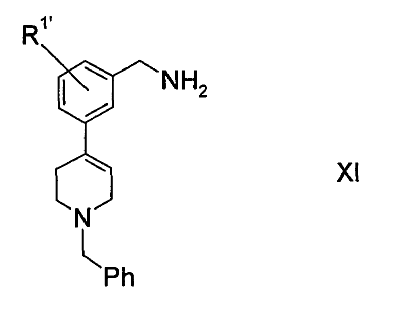 wherein R^{1'} is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, (C₁-C₈)-alkoxy, (C₃-C₁₀)-cycloalkoxy, (C₆-C₁₄)-aryloxy, di((C₁-C₈)-alkyl)amino, di((C₃-C₁₀)-cycloalkyl)amino or di((C₆-C₁₄)-aryl)amino, or a protected derivative thereof or precursor moiety thereto; and Ph is phenyl, or its salt.

25. A compound according to claim 24 which is 3-(1-benzyl-1,2,3,6-tetrahydropyridin-4-yl)benzylamine or its salt.

26. A compound of the formula XII, 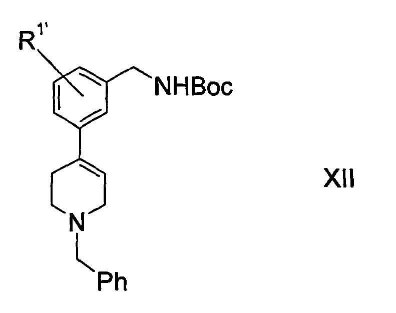 wherein R^{1'} is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, (C₁-C₈)-alkoxy, (C₃-C₁₀)-cycloalkoxy, (C₆-C₁₄)-aryloxy, di((C₁-C₈)-alkyl)amino, di((C₃-C₁₀)-cycloalkyl)amino or di((C₆-C₁₄)-aryl)amino, or a protected derivative thereof or precursor moiety thereto; Boc is tert-butoxycarbonyl; and Ph is phenyl, or its salt.

27. A compound according to claim 26 which is 3-(1-benzyl-1,2,3,6-tetrahydropyridin-4-yl)benzylcarbamic acid tert-butyl ester or its salt.

28. A compound of the formula XIV, 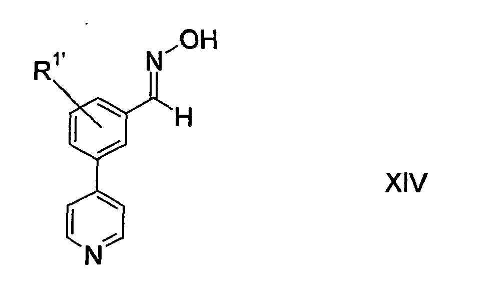 wherein R^{1'} is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, (C₁-C₈)-alkoxy, (C₃-C₁₀)-cycloalkoxy, (C₆-C₁₄)-aryloxy, di((C₁-C₈)-alkyl)amino, di((C₃-C₁₀)-cycloalkyl)amino or di((C₆-C₁₄)-aryl)amino, or a protected derivative thereof or precursor moiety thereto, or its salt.

29. A compound according to claim 28 which is 3-(4-pyridyl)benzaldehyde oxime or its salt.

30. A compound of the formula XV, 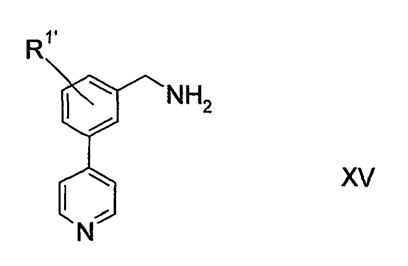 wherein R^{1'} is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, (C₁-C₈)-alkoxy, (C₃-C₁₀)-cycloalkoxy, (C₆-C₁₄)-aryloxy, di((C₁-C₈)-alkyl)amino, di((C₃-C₁₀)-cycloalkyl)amino or di((C₆-C₁₄)-aryl)amino, or a protected derivative thereof or precursor moiety thereto, or its salt.

31. A compound according to claim 30 which is 3-(4-pyridyl)benzylamine or its salt.

32. A compound of the formula XVI, 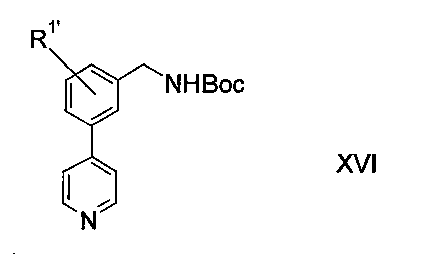 wherein R^{1'} is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, (C₁-C₈)-alkoxy, (C₃-C₁₀)-cycloalkoxy, (C₆-C₁₄)-aryloxy, di((C₁-C₈)-alkyl)amino, di((C₃-C₁₀)-cycloalkyl)amino or di((C₆-C₁₄)-aryl)amino, or a protected derivative thereof or precursor moiety thereto; and Boc is tert-butoxycarbonyl, or its salt.

33. A compound according to claim 32 which is 3-(4-pyridyl)benzylcarbamic acid tert-butyl ester or its salt.

34. A compound of the formula IVb, 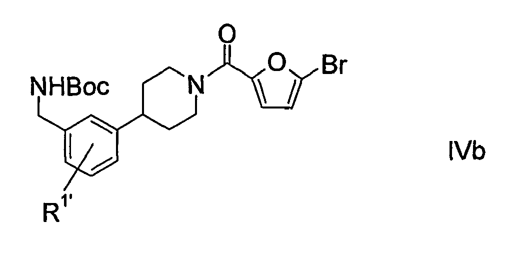
wherein R^{1'} is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, (C₁-C₈)-alkoxy, (C₃-C₁₀)-cycloalkoxy, (C₆-C₁₄)-aryloxy, di((C₁-C₈)-alkyl)amino, di((C₃-C₁₀)-cycloalkyl)amino or di((C₆-C₁₄)-aryl)amino, or a protected derivative thereof or precursor moiety thereto;
and Boc is tert-butoxycarbonyl,
or its salt.

35. A compound according to claim 34 which is 4-[3-(tert-butoxycarbonylaminomethyl)phenyl]-1-[5-bromo-2-furanoyl]piperidine.

36. The use of a compound as claimed in any one of claims 18 to 35 as an intermediate for the preparation of a compound of the formula I, 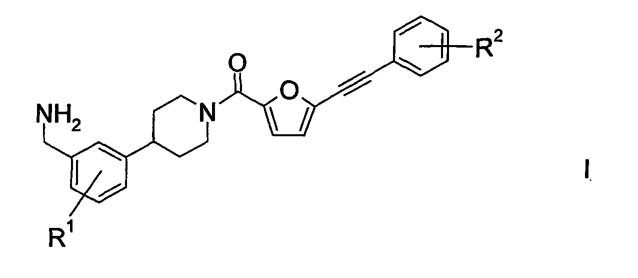 wherein
R¹ is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, (C₁-C₈)-alkoxy, (C₃-C₁₀)-cycloalkoxy, (C₆-C₁₄)-aryloxy, di((C₁-C₈)-alkyl)amino, di((C₃-C₁₀)-cycloalkyl)amino or di((C₆-C₁₄)-aryl)amino; and
R² is H, F, CF₃, OCF₃, (C₁-C₈)-alkyl, (C₃-C₁₀)-cycloalkyl, 3-membered to 10-membered heterocycloalkyl comprising 1, 2 or 3 identical or different ring heteroatoms chosen from nitrogen, oxygen and sulfur, (C₆-C₁₄)-aryl, 5-membered to 10-membered heteroaryl comprising 1, 2 or 3 identical or different ring heteroatoms chosen from nitrogen, oxygen and sulfur, (C₁-C₈)-alkoxy, (C₃-C₁₀)-cycloalkoxy, (C₆-C₁₄)-aryloxy, 5-membered to 10-membered heteroaryloxy comprising 1, 2, or 3 identical or different ring heteroatoms chosen from nitrogen, oxygen and sulfur, di((C₁-C₈)-alkyl)amino, di-((C₃-C₁₀)-cycloalkyl)amino or di((C₆-C₁₄)-aryl)amino;
or its salt.

## Patentansprüche

1. Verfahren zur Herstellung einer Verbindung der Formel I, 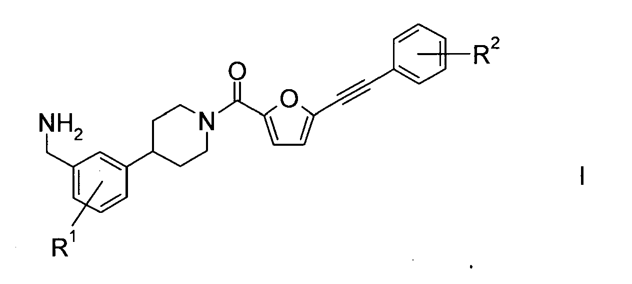 wobei
R¹ für H, F, CF₃, OCF₃, (C₁-C₈)-Alkyl, (C₃-C₁₀)-Cycloalkyl, dreigliedriges bis zehngliedriges Heterocycloalkyl mit 1, 2 oder 3 gleichen oder verschiedenen Ringheteroatomen ausgewählt aus Stickstoff, Sauerstoff und Schwefel, (C₆-C₁₄)-Aryl, (C₁-C₈)-Alkoxy, (C₃-C₁₀)-Cycloalkoxy, (C₆-C₁₄)-Aryloxy, Di((C₁-C₈)-alkyl)amino, Di((C₃-C₁₀)-cycloalkyl)amino oder Di((C₆-C₁₄)-aryl)amino steht; und
R² für H, F, CF₃, OCF₃, (C₁-C₈)-Alkyl, (C₃-C₁₀)-Cycloalkyl, dreigliedriges bis zehngliedriges Heterocycloalkyl mit 1, 2 oder 3 gleichen oder verschiedenen Ringheteroatomen ausgewählt aus Stickstoff, Sauerstoff und Schwefel, (C₆-C₁₄)-Aryl, fünfgliedriges bis zehngliedriges Heteroaryl mit 1, 2 oder 3 gleichen oder verschiedenen Ringheteroatomen ausgewählt aus Stickstoff, Sauerstoff und Schwefel, (C₁-C₈)-Alkoxy, (C₃-C₁₀)-Cycloalkoxy, (C₆-C₁₄)-Aryloxy, fünfgliedriges bis zehngliedriges Heteroaryloxy mit 1, 2 oder 3 gleichen oder verschiedenen Ringheteroatomen ausgewählt aus Stickstoff, Sauerstoff und Schwefel, Di((C₁-C₈)-alkyl)amino, Di((C₃-C₁₀)-cycloalkyl)amino oder Di((C₆-C₁₄)-aryl)amino steht;
oder deren Salz,
bei dem man
a) eine Phenylethinylsilanverbindung der Formeln II, 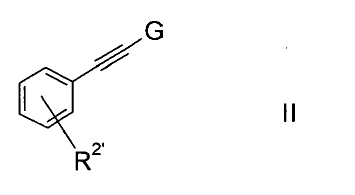 wobei G für Trimethylsilyl, Triethylsilyl, Triisopropylsilyl oder Dimethyl-tert-butylsilyl steht, und R^{2'} für wie oben definiertes R² oder ein geschütztes Derivat davon oder eine Vorstufengruppe davon steht, in einem Lösungsmittel mit einem Alkalimetallhydroxid, -carbonat oder -alkoholat oder einem Erdalkalimetallalkoholat behandelt, unter Entstehung einer Lösung eines Phenylethins der Formel III; 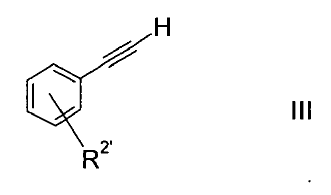 und
b) die erhaltene Lösung des Phenylethins in Gegenwart eines homogenen Palladiumkatalysators, eines Kupfer(I)-Salzes und eines gehinderten Amins in einem Lösungsmittel mit einer Verbindung der Formel IV, 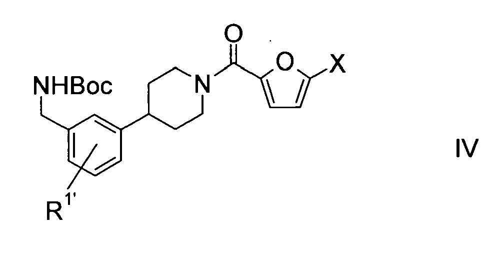
wobei X für Brom oder Iod steht, R^{1'} für wie oben definiertes R¹ oder ein geschütztes Derivat davon oder eine Vorstufengruppe davon steht und Boc für tert-Butoxycarbonyl steht, vereint unter Bildung einer geschützten Verbindung der Formel V, 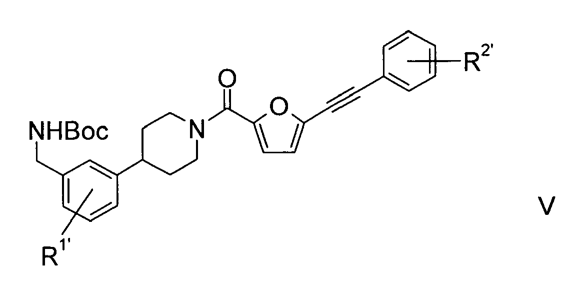 wobei R^{1'} und R^{2'} für wie oben definiertes R¹ bzw. R² oder ein geschütztes Derivat davon oder eine Vorstufengruppe davon stehen und Boc für tert-Butoxycarbonyl steht, oder deren Salz.

2. Verfahren nach Anspruch 1 zur Herstellung von 4-[3-(Aminomethyl)phenyl]-1-[5-(2-fluorphenylethinyl)-2-furanoyl]piperidin der Formel la, 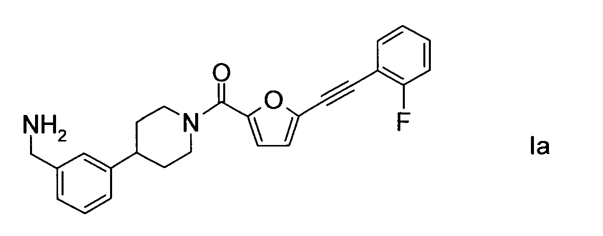 oder dessen Salz,
bei dem man
a) eine (2-Fluorphenylethinyl)silylverbindung der Formel IIa, 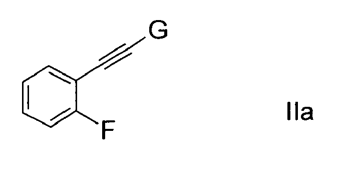 wobei G für Trimethylsilyl, Triethylsilyl, Triisopropylsilyl oder Dimethyl-tert-butylsilyl steht, in einem Lösungsmittel mit einem Alkalimetallhydroxid, -carbonat oder -alkoholat oder einem Erdalkalimetallalkoholat behandelt, unter Entstehung einer Lösung von 2-Fluorphenylethin; und
b) die erhaltene Lösung von 2-Fluorphenylethin in Gegenwart eines homogenen Palladiumkatalysators, eines Kupfer(I)-Salzes und eines gehinderten Amins in einem Lösungsmittel mit einer Verbindung der Formel IVa, 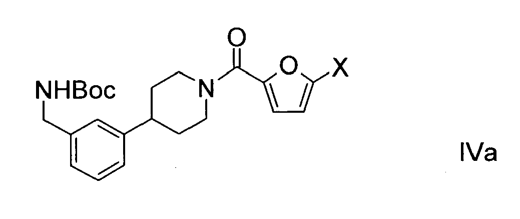
wobei X für Brom oder Iod steht und Boc für tert-Butoxycarbonyl steht, vereint unter Bildung von 4-[3-(tert-Butoxycarbonylaminomethyl)phenyl]-1-[5-(2-fluorphenylethinyl)-2-furanoyl]piperidin der Formel Va, 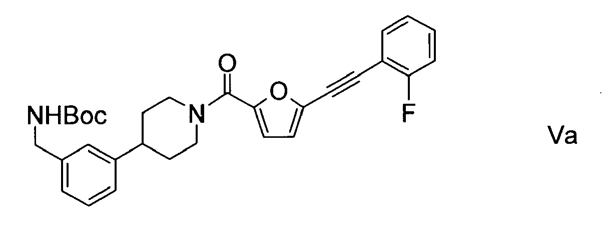 wobei Boc für tert-Butoxycarbonyl steht.

3. Verfahren nach Anspruch 1 oder 2, wobei G für Trimethylsilyl steht.

4. Verfahren nach einem der Ansprüche 1 bis 3, wobei X für Brom steht.

5. Verfahren nach einem der Ansprüche 1 bis 4, bei dem man weiterhin die tert-Butoxycarbonylgruppe und andere Schutzgruppen entfernt und/oder Vorstufengruppen umwandelt, die in einer Verbindung der Formel V oder Va vorhanden sind, unter Bildung einer Verbindung der Formel I bzw. la oder deren Salz.

6. Verfahren nach einem der Ansprüche 1 bis 5, bei dem man weiterhin die tert-Butoxycarbonylgruppe aus einer Verbindung der Formel V oder Va in Gegenwart einer Säure entfernt, unter Bildung einer Verbindung der Formel I bzw. la oder deren Salz.

7. Verfahren nach einem der Ansprüche 1 bis 6, bei dem man weiterhin die tert-Butoxycarbonylgruppe aus 4-[3-(tert-Butoxycarbonylaminomethyl)phenyl]-1-[5-(2-fluorphenylethinyl)-2-furanoyl]piperidin in Gegenwart von Methansulfonsäure entfernt, unter Bildung des Methansulfonsäuresalzes von 4-[3-(Aminomethyl)phenyl]-1-[5-(2-fluorphenylethinyl)-2-furanoyl]piperidin.

8. Verfahren zur Herstellung einer Verbindung der Formel IV, 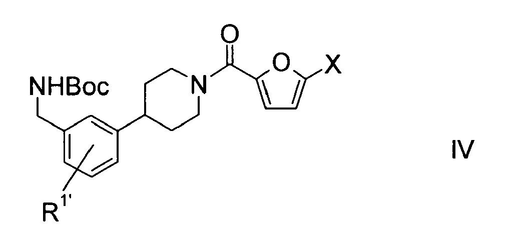 wobei
R^{1'} für H, F, CF₃, OCF₃, (C₁-C₈)-Alkyl, (C₃-C₁₀)-Cycloalkyl, dreigliedriges bis zehngliedriges Heterocycloalkyl mit 1, 2 oder 3 gleichen oder verschiedenen Ringheteroatomen ausgewählt aus Stickstoff, Sauerstoff und Schwefel, (C₆-C₁₄)-Aryl, (C₁-C₈)-Alkoxy, (C₃-C₁₀)-Cycloalkoxy, (C₆-C₁₄)-Aryloxy, Di((C₁-C₈)-alkyl)amino, Di((C₃-C₁₀)-cycloalkyl)amino oder Di((C₆-C₁₄)-aryl)amino steht, oder ein geschütztes Derivat davon oder eine Vorstufengruppe davon; und
X für Brom oder Iod steht,
bei dem man
a) 5-Bromfuran-2-carbonsäure oder 5-lodfuran-2-carbonsäure aktiviert unter Bildung einer aktivierten Form von 5-Bromfuran-2-carbonsäure bzw. 5-lodfuran-2-carbonsäure; und
b) die aktivierte Form von 5-Bromfuran-2-carbonsäure bzw. 5-lodfuran-2-carbonsäure in einem Lösungsmittel mit einer Verbindung der Formel VI, 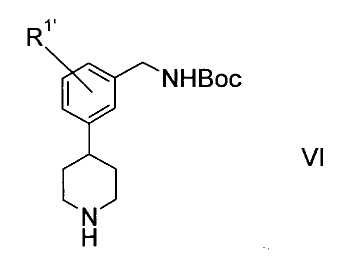
wobei R^{1'} wie für Formel IV definiert ist und Boc für tert-Butoxycarbonyl steht, vereint.

9. Verfahren nach Anspruch 8, wobei R^{1'} für Wasserstoff steht.

10. Verfahren nach Anspruch 8 oder 9 zur Herstellung der Verbindung der Formel IVc, 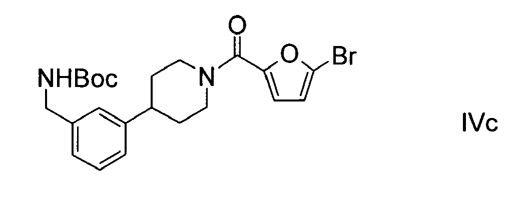 wobei Boc für tert-Butoxycarbonyl steht,
bei dem man
a) 5-Bromfuran-2-carbonsäure mit Thionylchlorid behandelt, unter Bildung von 5-Bromfuran-2-carbonsäurechlorid, und
b) das 5-Bromfuran-2-carbonsäurechlorid in einem Lösungsmittel in Gegenwart von Base mit der Verbindung der Formel VIa 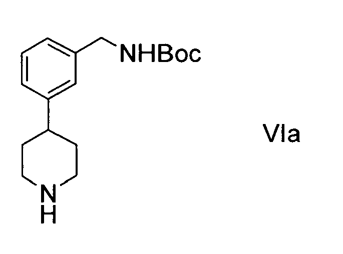
wobei Boc für tert-Butoxycarbonyl steht, vereint.

11. Verfahren zur Herstellung einer Verbindung der Formel VI 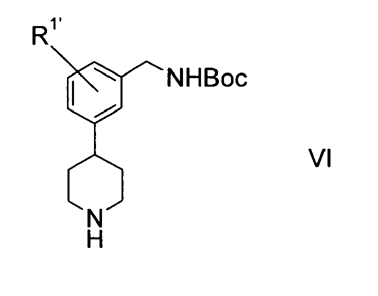
wobei R^{1'} für H, F, CF₃, OCF₃, (C₁-C₈)-Alkyl, (C₃-C₁₀)-Cycloalkyl, dreigliedriges bis zehngliedriges Heterocycloalkyl mit 1, 2 oder 3 gleichen oder verschiedenen Ringheteroatomen ausgewählt aus Stickstoff, Sauerstoff und Schwefel, (C₆-C₁₄)-Aryl, (C₁-C₈)-Alkoxy, (C₃-C₁₀)-Cycloalkoxy, (C₆-C₁₄)-Aryloxy, Di((C₁-C₈)-alkyl)amino, Di((C₃-C₁₀)-cycloalkyl)amino oder Di((C₆-C₁₄)-aryl)amino steht, oder ein geschütztes Derivat davon oder eine Vorstufengruppe davon; und
Boc für tert-Butoxycarbonyl steht,
oder deren Salz,
bei dem man
a) ein Salz eines 3-Halogenbenzylamins der Formel VII, 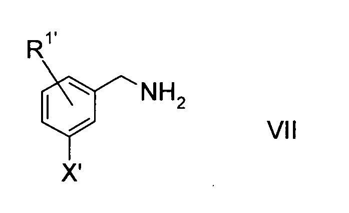 in einem Lösungsmittel in Gegenwart einer Base mit 1,2-Bis(chlordimethylsilyl)ethan behandelt, unter Bildung eines 1-(3-Halogenbenzyl)-2,2,5,5-tetramethyl-1-aza-2,5-disilacyclopentans der Formel VIII; 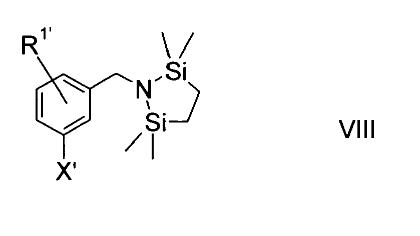
b) das 1-(3-Halogenbenzyl)-2,2,5,5-tetramethyl-1-aza-2,5-disilacyclopentan der Formel VIII bei einer Temperatur von etwa -80°C bis etwa -40°C in einem Lösungsmittel mit einer Alkyllithiumverbindung und 1-Benzyl-4-piperidon behandelt, unter Bildung eines Alkohols der Formel IX; 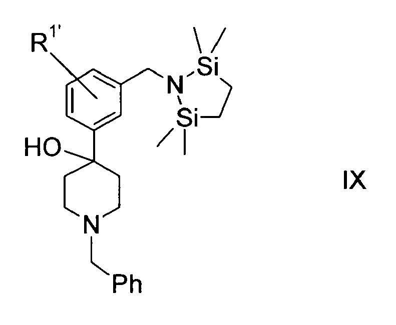
c) den Alkohol der Formel IX in einem Lösungsmittel mit einer Säure behandelt, unter Bildung eines Hydroxypiperidinylbenzylamins der Formel X 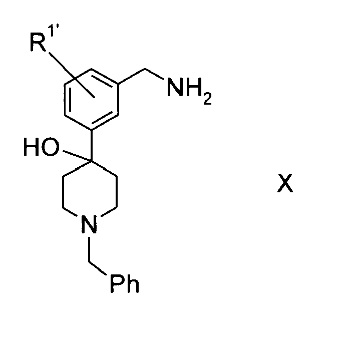 als dem Salz der Säure;
d) das Hydroxypiperidinylbenzylamin der Formel X oder dessen Salz bei einer Temperatur von etwa 70°C bis etwa 150°C mit einer konzentrierten, nicht oxidierenden Säure behandelt und anschließend alkalisch stellt, unter Bildung eines Olefins der Formel XI; 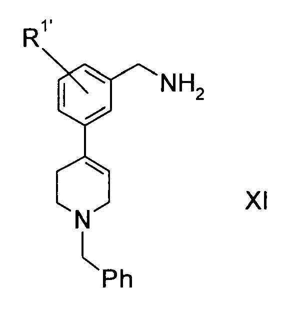
e) das Olefin der Formel XI in einem Lösungsmittel in Gegenwart einer Base mit Di-tert-butyl-dicarbonat behandelt, unter Bildung eines geschützten Amins der Formel XII 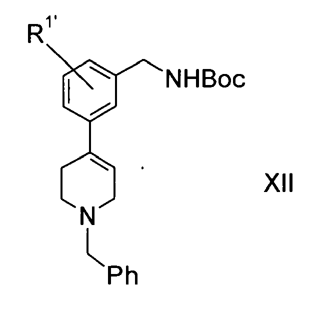
wobei Boc für tert-Butoxycarbonyl steht und wobei Ph in den Formeln IX, X, XI und XII für Phenyl steht, X' in den Formeln VII und VIII für Brom oder Iod steht und R^{1'} in den Formeln VII, VIII, IX, X, XI und XII wie für Formel VI definiert ist.

12. Verfahren nach Anspruch 11 zur Herstellung von 4-[3-(tert-Butoxycarbonylaminomethyl)phenyl]piperidin oder dessen Salz,
bei dem man
a) 3-Brombenzylaminhydrochlorid oder 3-lodbenzylaminhydrochlorid in einem halogenierten aliphatischen Kohlenwasserstoff in Gegenwart eines tertiären Amins mit 1,2-Bis(chlordimethylsilyl)ethan behandelt, unter Bildung des entsprechenden 1-(3-Halogenbenzyl)-2,2,5,5-tetramethyl-1-aza-2,5-disilacyclopentans, wobei Halogen für Brom bzw. Iod steht;
b) das 1-(3-Halogenbenzyl)-2,2,5,5-tetramethyl-1-aza-2,5-disilacyclopentan bei einer Temperatur von etwa -80°C bis etwa -40°C in einem Ether mit einer Alkyllithiumverbindung und 1-Benzyl-4-piperidon behandelt, unter Bildung von 1-[3-(1-Benzyl-4-hydroxypiperidin-4-yl)benzyl]-2,2,5,5-tetramethyl-1-aza-2,5-disilacyclopentan;
c) das 1-[3-(1-Benzyl-4-hydroxypiperidin-4-yl)benzyl]-2,2,5,5-tetramethyl-1-aza-2,5-disilacyclopentan in einem halogenierten Kohlenwasserstoff mit einer anorganischen Säure behandelt, unter Bildung von 3-(1-Benzyl-4-hydroxypiperidin-4-yl)benzylamin als dem Salz der anorganischen Säure;
d) das 3-(1-Benzyl-4-hydroxypiperidin-4-yl)benzylamin bei einer Temperatur von etwa 70°C bis etwa 150°C mit einer konzentrierten, nicht oxidierenden Säure behandelt, und anschließend alkalisch stellt, unter Bildung von 3-(1-Benzyl-1,2,3,6-tetrahydropyridin-4-yl)benzylamin; und
e) das 3-(1-Benzyl-1,2,3,6-tetrahydropyridin-4-yl)benzylamin in einem aliphatischen Alkohol, Essigsäureethylester, einem Ether, einem halogenierten Kohlenwasserstoff, einer Mischung von zwei oder mehreren dieser Lösungsmittel oder einer Mischung von einem oder mehreren dieser Lösungsmittel mit Wasser in Gegenwart eines Alkalimetallhydroxids, -carbonats oder -alkoholats oder eines tertiären Amins mit Di-tert-butyl-dicarbonat behandelt, unter Bildung von 3-(1-Benzyl-1,2,3,6-tetrahydropyridin-4-yl)benzylcarbaminsäure-tert-butylester.

13. Verfahren nach Anspruch 11 oder 12, bei dem man weiterhin das Amin der Formel XII in einem Lösungsmittel in Gegenwart eines Palladiumkatalysators und in Gegenwart einer Säure mit Wasserstoff behandelt, unter Bildung einer Verbindung der Formel VI oder deren Salz.

14. Verfahren zur Herstellung einer Verbindung der Formel VI, 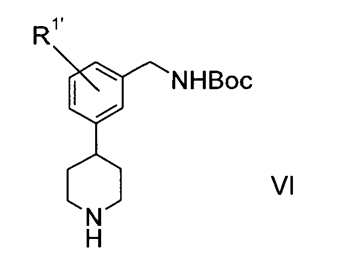 wobei
R^{1'} für H, F, CF₃, OCF₃, (C₁-C₈)-Alkyl, (C₃-C₁₀)-Cycloalkyl, dreigliedriges bis zehngliedriges Heterocycloalkyl mit 1, 2 oder 3 gleichen oder verschiedenen Ringheteroatomen ausgewählt aus Stickstoff, Sauerstoff und Schwefel, (C₆-C₁₄)-Aryl, (C₁-C₈)-Alkoxy, (C₃-C₁₀)-Cycloalkoxy, (C₆-C₁₄)-Aryloxy, Di((C₁-C₈)-alkyl)amino, Di((C₃-C₁₀)-cycloalkyl)amino oder Di((C₆-C₁₄)-aryl)amino steht, oder ein geschütztes Derivat davon oder eine Vorstufengruppe davon; und
Boc für tert-Butoxycarbonyl steht,
oder deren Salz,
bei dem man
a) einen 3-(4-Pyridyl)benzaldehyd der Formel XIII, 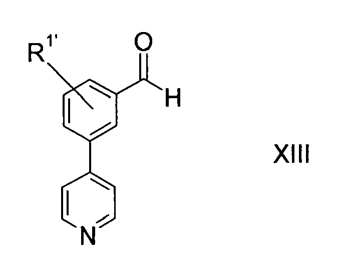 in einem Lösungsmittel mit Hydroxylamin oder einem Salz von Hydroxylamin behandelt, unter Bildung eines Oxims der Formel XIV oder dessen Salz; 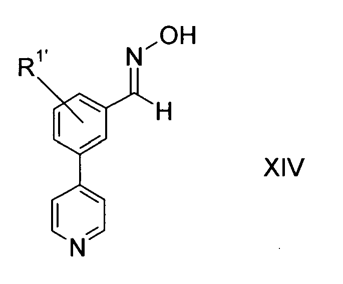
b) das Oxim der Formel XIV oder dessen Salz in einem Lösungsmittel in Gegenwart eines Palladiumkatalysators mit Wasserstoff behandelt, unter Bildung eines 3-(4-Pyridyl)benzylamins der Formel XV oder dessen Salz; 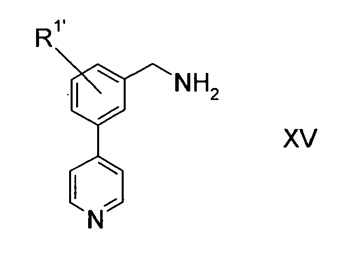
c) das 3-(4-Pyridyl)benzylamin der Formel XV oder dessen Salz in einem Lösungsmittel in Gegenwart einer Base mit Di-tert-butyl-dicarbonat behandelt, unter Bildung eines Boc-geschützten 3-(4-Pyridyl)benzylamins der Formel XVI, 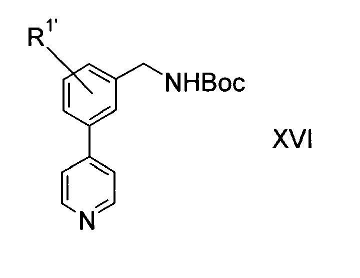 wobei Boc für tert-Butoxycarbonyl steht; und
d) das Boc-geschützte 3-(4-Pyridyl)benzylamin der Formel XVI in einem Lösungsmittel in Gegenwart eines Platinkatalysators und in Gegenwart einer Säure mit Wasserstoff behandelt, unter Bildung einer Verbindung der Formel VI oder deren Salz;
wobei R^{1'} in den Formeln XIII, XIV, XV und XVI wie oben für Formel VI definiert ist.

15. Verfahren nach Anspruch 14 zur Herstellung von 4-[3-(tert-Butoxycarbonylaminomethyl)phenyl]piperidin oder dessen Salz,
bei dem man
a) 3-(4-Pyridyl)benzaldehyd bei einer Temperatur von etwa 0°C bis etwa 40°C in einem aliphatischen Alkohol mit Hydroxylamin oder einem Salz davon behandelt, unter Bildung von 3-(4-Pyridyl)benzaldehydoxim oder dessen Salz;
b) das 3-(4-Pyridyl)benzaldehydoxim oder dessen Salz bei einer Temperatur von etwa 20°C bis etwa 50°C in einem aliphatischen Alkohol in Gegenwart eines Palladiumkatalysators bei einem Druck von etwa 300 kPa bis etwa 1500 kPa mit Wasserstoff behandelt, unter Bildung von 3-(4-Pyridyl)benzylamin oder dessen Salz;
c) das 3-(4-Pyridyl)benzylamin oder dessen Salz in einem aliphatischen Alkohol, Essigsäureethylester, einem Ether, einem halogenierten Kohlenwasserstoff, einer Mischung von zwei oder mehreren dieser Lösungsmittel oder einer Mischung von einem oder mehreren dieser Lösungsmittel mit Wasser in Gegenwart eines Alkalimetallhydroxids, -carbonats oder -alkoholats oder eines tertiären Amins mit Di-tert-butyl-dicarbonat behandelt, unter Bildung von 3-(4-Pyridyl)benzylcarbaminsäure-tert-butylester; und
d) den 3-(4-Pyridyl)benzylcarbaminsäure-tert-butylester in einem aliphatischen Alkohol bei einem Druck von etwa 2000 kPa bis etwa 6000 kPa in Gegenwart eines Platinkatalysators und in Gegenwart einer Säure mit Wasserstoff behandelt.

16. Verfahren zur Herstellung einer Verbindung der Formel VI 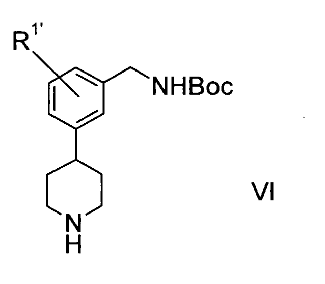 wobei
R^{1'} für H, F, CF₃, OCF₃, (C₁-C₈)-Alkyl, (C₃-C₁₀)-Cycloalkyl, dreigliedriges bis zehngliedriges Heterocycloalkyl mit 1, 2 oder 3 gleichen oder verschiedenen Ringheteroatomen ausgewählt aus Stickstoff, Sauerstoff und Schwefel, (C₆-C₁₄)-Aryl, (C₁-C₈)-Alkoxy, (C₃-C₁₀)-Cycloalkoxy, (C₆-C₁₄)-Aryloxy, Di((C₁-C₈)-alkyl)amino, Di((C₃-C₁₀)-cycloalkyl)amino oder Di((C₆-C₁₄)-aryl)amino steht, oder ein geschütztes Derivat davon oder eine Vorstufengruppe davon; und
Boc für tert-Butoxycarbonyl steht,
oder deren Salz,
bei dem man
a) ein 3-(4-Pyridyl)benzonitril der Formel XVII, 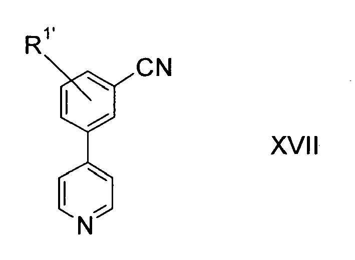 in einem Lösungsmittel mit Wasserstoff in Gegenwart eines Palladiumkatalysators und in Gegenwart einer Säure behandelt, oder mit einem komplexen Hydrid behandelt, unter Bildung eines 3-(4-Pyridyl)benzylamins der Formel XV oder dessen Salz; 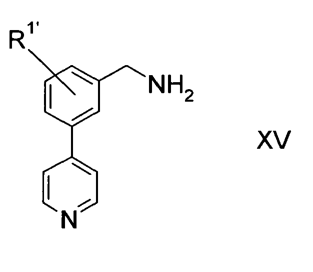
b) das 3-(4-Pyridyl)benzylamin der Formel XV oder dessen Salz in einem Lösungsmittel in Gegenwart einer Base mit Di-tert-butyl-dicarbonat behandelt, unter Bildung eines Boc-geschützten 3-(4-Pyridyl)benzylamins der Formel XVI, 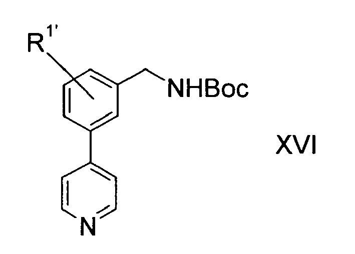 wobei Boc für tert-Butoxycarbonyl steht; und
c) das Boc-geschützte 3-(4-Pyridyl)benzylamin der Formel XVI in einem Lösungsmittel in Gegenwart eines Platinkatalysators und in Gegenwart einer Säure mit Wasserstoff behandelt, unter Bildung einer Verbindung der Formel VI oder deren Salz;
wobei R^{1'} in den Formeln XV, XVI und XVII wie oben für Formel VI definiert ist.

17. Verfahren nach Anspruch 16 zur Herstellung von 4-[3-(tert-Butoxycarbonylaminomethyl)phenyl]piperidin oder dessen Salz,
bei dem man
a) bei einer Temperatur von etwa 0°C bis etwa 50°C 3-(4-Pyridyl)benzonitril in Gegenwart eines Palladiumkatalysators bei einem Druck von etwa 2000 kPa bis etwa 6000 kPa in einem aliphatischen Alkohol oder einer Mischung eines aliphatischen Alkohols mit Wasser mit Wasserstoff behandelt, oder in einem aliphatischen Alkohol oder einem Ether mit einem komplexen Hydrid behandelt, unter Bildung von 3-(4-Pyridyl)benzylamin oder dessen Salz;
b) das 3-(4-Pyridyl)benzylamin oder dessen Salz in einem aliphatischen Alkohol, Essigsäureethylester, einem Ether, einem halogenierten Kohlenwasserstoff, einer Mischung von zwei oder mehreren dieser Lösungsmittel oder einer Mischung von einem oder mehreren dieser Lösungsmittel mit Wasser in Gegenwart eines Alkalimetallhydroxids, -carbonats oder -alkoholats oder eines tertiären Amins mit Di-tert-butyl-dicarbonat behandelt, unter Bildung von 3-(4-Pyridyl)benzylcarbaminsäure-tert-butylester; und
c) den 3-(4-Pyridyl)benzylcarbaminsäure-tert-butylester in einem aliphatischen Alkohol bei einem Druck von etwa 2000 kPa bis etwa 6000 kPa in Gegenwart eines Platinkatalysators und in Gegenwart einer Säure mit Wasserstoff behandelt.

18. Verbindung der Formel VIII, 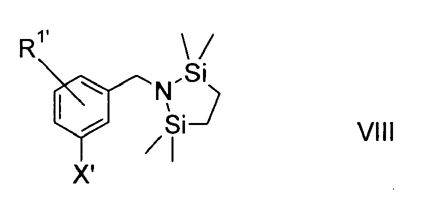 wobei R^{1'} für H, F, CF₃, OCF₃, (C₁-C₈)-Alkyl, (C₃-C₁₀)-Cycloalkyl, dreigliedriges bis zehngliedriges Heterocycloalkyl mit 1, 2 oder 3 gleichen oder verschiedenen Ringheteroatomen ausgewählt aus Stickstoff, Sauerstoff und Schwefel, (C₆-C₁₄)-Aryl, (C₁-C₈)-Alkoxy, (C₃-C₁₀)-Cycloalkoxy, (C₆-C₁₄)-Aryloxy, Di((C₁-C₈)-alkyl)amino, Di((C₃-C₁₀)-cycloalkyl)amino oder Di((C₆-C₁₄)-aryl)amino steht, oder ein geschütztes Derivat davon oder eine Vorstufengruppe davon; und X' für Brom oder lod steht; mit Ausnahme der Verbindung 1-(3-Brombenzyl)-2,2,5,5-tetramethyl-1-aza-2,5-disilacyclopentan.

19. Verbindung nach Anspruch 18, bei der es sich um 1-(3-lodbenzyl)-2,2,5,5-tetramethyl-1-aza-2,5-disilacyclopentan handelt.

20. Verbindung der Formel IX, 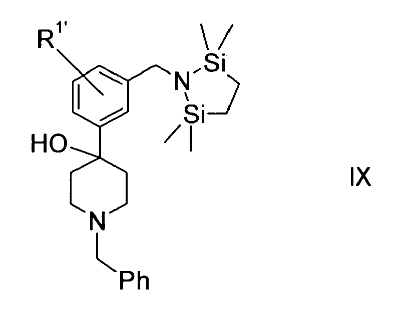 wobei R^{1'} für H, F, CF₃, OCF₃, (C₁-C₈)-Alkyl, (C₃-C₁₀)-Cycloalkyl, dreigliedriges bis zehngliedriges Heterocycloalkyl mit 1, 2 oder 3 gleichen oder verschiedenen Ringheteroatomen ausgewählt aus Stickstoff, Sauerstoff und Schwefel, (C₆-C₁₄)-Aryl, (C₁-C₈)-Alkoxy, (C₃-C₁₀)-Cycloalkoxy, (C₆-C₁₄)-Aryloxy, Di((C₁-C₈)-alkyl)amino, Di((C₃-C₁₀)-cycloalkyl)amino oder Di((C₆-C₁₄)-aryl)amino steht, oder ein geschütztes Derivat davon oder eine Vorstufengruppe davon; und Ph für Phenyl steht.

21. Verbindung nach Anspruch 20, bei der es sich um 1-[3-(1-Benzyl-4-hydroxypiperidin-4-yl)benzyl]-2,2,5,5-tetramethyl-1-aza-2,5-disilacyclopentan handelt.

22. Verbindung der Formel X, 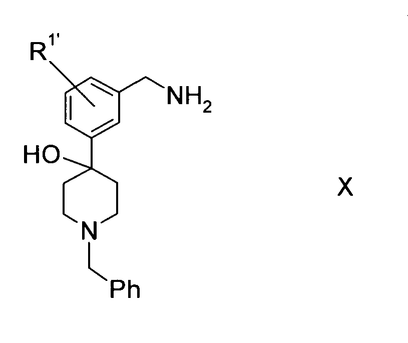 wobei R^{1'} für H, F, CF₃, OCF₃, (C₁-C₈)-Alkyl, (C₃-C₁₀)-Cycloalkyl, dreigliedriges bis zehngliedriges Heterocycloalkyl mit 1, 2 oder 3 gleichen oder verschiedenen Ringheteroatomen ausgewählt aus Stickstoff, Sauerstoff und Schwefel, (C₆-C₁₄)-Aryl, (C₁-C₈)-Alkoxy, (C₃-C₁₀)-Cycloalkoxy, (C₆-C₁₄)-Aryloxy, Di((C₁-C₈)-alkyl)amino, Di((C₃-C₁₀)-cycloalkyl)amino oder Di((C₆-C₁₄)-aryl)amino steht, oder ein geschütztes Derivat davon oder eine Vorstufengruppe davon; und Ph für Phenyl steht, oder deren Salz.

23. Verbindung nach Anspruch 22, bei der es sich um 3-(1-Benzyl-4-hydroxypiperidin-4-yl)benzylamin oder dessen Salz handelt.

24. Verbindung der Formel XI, 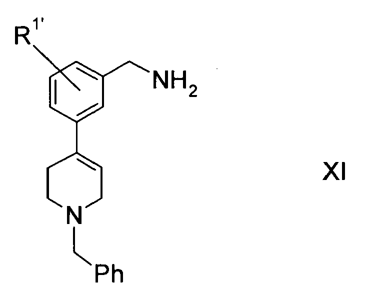 wobei R^{1'} für H, F, CF₃, OCF₃, (C₁-C₈)-Alkyl, (C₃-C₁₀)-Cycloalkyl, dreigliedriges bis zehngliedriges Heterocycloalkyl mit 1, 2 oder 3 gleichen oder verschiedenen Ringheteroatomen ausgewählt aus Stickstoff, Sauerstoff und Schwefel, (C₆-C₁₄)-Aryl, (C₁-C₈)-Alkoxy, (C₃-C₁₀)-Cycloalkoxy, (C₆-C₁₄)-Aryloxy, Di((C₁-C₈)-alkyl)amino, Di((C₃-C₁₀)-cycloalkyl)amino oder Di((C₆-C₁₄)-aryl)amino steht, oder ein geschütztes Derivat davon oder eine Vorstufengruppe davon; und Ph für Phenyl steht, oder deren Salz.

25. Verbindung nach Anspruch 24, bei der es sich um 3-(1-Benzyl-1,2,3,6-tetrahydropyridin-4-yl)benzylamin oder dessen Salz handelt.

26. Verbindung der Formel XII, 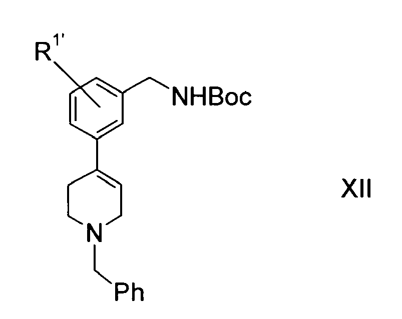 wobei R^{1'} für H, F, CF₃, OCF₃, (C₁-C₈)-Alkyl, (C₃-C₁₀)-Cycloalkyl, dreigliedriges bis zehngliedriges Heterocycloalkyl mit 1, 2 oder 3 gleichen oder verschiedenen Ringheteroatomen ausgewählt aus Stickstoff, Sauerstoff und Schwefel, (C₆-C₁₄)-Aryl, (C₁-C₈)-Alkoxy, (C₃-C₁₀)-Cycloalkoxy, (C₆-C₁₄)-Aryloxy, Di((C₁-C₈)-alkyl)amino, Di((C₃-C₁₀)-cycloalkyl)amino oder Di((C₆-C₁₄)-aryl)amino steht, oder ein geschütztes Derivat davon oder eine Vorstufengruppe davon; Boc für tert-Butoxycarbonyl steht; und Ph für Phenyl steht, oder deren Salz.

27. Verbindung nach Anspruch 26, bei der es sich um 3-(1-Benzyl-1,2,3,6-tetrahydropyridin-4-yl)benzylcarbaminsäure-tert-butylester oder dessen Salz handelt.

28. Verbindung der Formel XIV, 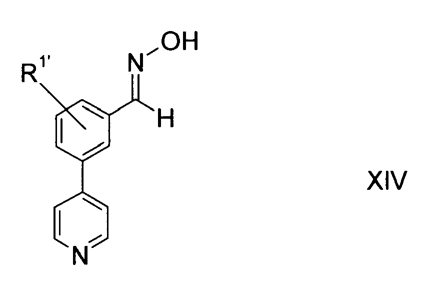 wobei R^{1'} für H, F, CF₃, OCF₃, (C₁-C₈)-Alkyl, (C₃-C₁₀)-Cycloalkyl, dreigliedriges bis zehngliedriges Heterocycloalkyl mit 1, 2 oder 3 gleichen oder verschiedenen Ringheteroatomen ausgewählt aus Stickstoff, Sauerstoff und Schwefel, (C₆-C₁₄)-Aryl, (C₁-C₈)-Alkoxy, (C₃-C₁₀)-Cycloalkoxy, (C₆-C₁₄)-Aryloxy, Di((C₁-C₈)-alkyl)amino, Di((C₃-C₁₀)-cycloalkyl)amino oder Di((C₆-C₁₄)-aryl)amino steht, oder ein geschütztes Derivat davon oder eine Vorstufengruppe davon; oder deren Salz.

29. Verbindung nach Anspruch 28, bei der es sich um 3-(4-Pyridyl)benzaldehydoxim oder dessen Salz handelt.

30. Verbindung der Formel XV, 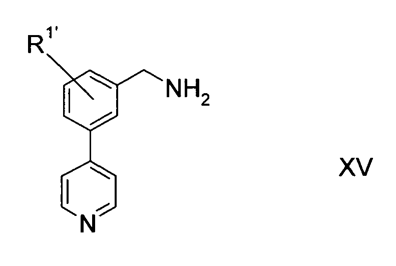 wobei R^{1'} für H, F, CF₃, OCF₃, (C₁-C₈)-Alkyl, (C₃-C₁₀)-Cycloalkyl, dreigliedriges bis zehngliedriges Heterocycloalkyl mit 1, 2 oder 3 gleichen oder verschiedenen Ringheteroatomen ausgewählt aus Stickstoff, Sauerstoff und Schwefel, (C₆-C₁₄)-Aryl, (C₁-C₈)-Alkoxy, (C₃-C₁₀)-Cycloalkoxy, (C₆-C₁₄)-Aryloxy, Di((C₁-C₈)-alkyl)amino, Di((C₃-C₁₀)-cycloalkyl)amino oder Di((C₆-C₁₄)-aryl)amino steht, oder ein geschütztes Derivat davon oder eine Vorstufengruppe davon; oder deren Salz.

31. Verbindung nach Anspruch 30, bei der es sich um 3-(4-Pyridyl)benzylamin oder dessen Salz handelt.

32. Verbindung der Formel XVI, 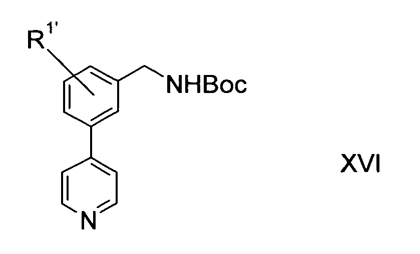 wobei R^{1'} für H, F, CF₃, OCF₃, (C₁-C₈)-Alkyl, (C₃-C₁₀)-Cycloalkyl, dreigliedriges bis zehngliedriges Heterocycloalkyl mit 1, 2 oder 3 gleichen oder verschiedenen Ringheteroatomen ausgewählt aus Stickstoff, Sauerstoff und Schwefel, (C₆-C₁₄)-Aryl, (C₁-C₈)-Alkoxy, (C₃-C₁₀)-Cycloalkoxy, (C₆-C₁₄)-Aryloxy, Di((C₁-C₈)-alkyl)amino, Di((C₃-C₁₀)-cycloalkyl)amino oder Di((C₆-C₁₄)-aryl)amino steht, oder ein geschütztes Derivat davon oder eine Vorstufengruppe davon; und Boc für tert-Butoxycarbonyl steht, oder deren Salz.

33. Verbindung nach Anspruch 32, bei der es sich um 3-(4-Pyridyl)benzylcarbaminsäure-tert-butylester oder dessen Salz handelt.

34. Verbindung der Formel IVb, 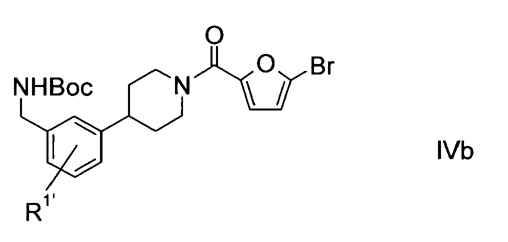
wobei R^{1'} für H, F, CF₃, OCF₃, (C₁-C₈)-Alkyl, (C₃-C₁₀)-Cycloalkyl, dreigliedriges bis zehngliedriges Heterocycloalkyl mit 1, 2 oder 3 gleichen oder verschiedenen Ringheteroatomen ausgewählt aus Stickstoff, Sauerstoff und Schwefel, (C₆-C₁₄)-Aryl, (C₁-C₈)-Alkoxy, (C₃-C₁₀)-Cycloalkoxy, (C₆-C₁₄)-Aryloxy, Di((C₁-C₈)-alkyl)amino, Di((C₃-C₁₀)-cycloalkyl)amino oder Di((C₆-C₁₄)-aryl)amino steht, oder ein geschütztes Derivat davon oder eine Vorstufengruppe davon;
und Boc für tert-Butoxycarbonyl steht,
oder deren Salz.

35. Verbindung nach Anspruch 34, bei der es sich um 4-[3-(tert-Butoxycarbonylaminomethyl)phenyl]-1-[5-brom-2-furanoyl]piperidin handelt.

36. Verwendung einer Verbindung nach einem der Ansprüche 18 bis 35 als Zwischenprodukt für die Herstellung einer Verbindung der Formel I, 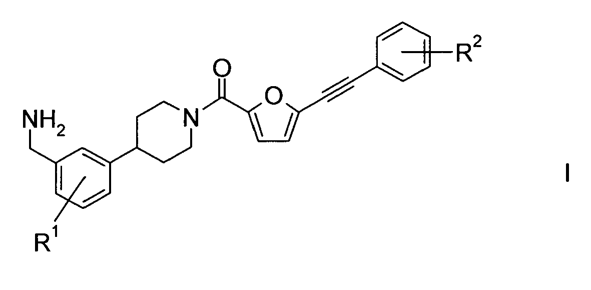 wobei
R¹ für H, F, CF₃, OCF₃, (C₁-C₈)-Alkyl, (C₃-C₁₀)-Cycloalkyl, dreigliedriges bis zehngliedriges Heterocycloalkyl mit 1, 2 oder 3 gleichen oder verschiedenen Ringheteroatomen ausgewählt aus Stickstoff, Sauerstoff und Schwefel, (C₆-C₁₄)-Aryl, (C₁-C₈)-Alkoxy, (C₃-C₁₀)-Cycloalkoxy, (C₆-C₁₄)-Aryloxy, Di((C₁-C₈)-alkyl)amino, Di((C₃-C₁₀)-cycloalkyl)amino oder Di((C₆-C₁₄)-aryl)amino steht; und
R² für H, F, CF₃, OCF₃, (C₁-C₈)-Alkyl, (C₃-C₁₀)-Cycloalkyl, dreigliedriges bis zehngliedriges Heterocycloalkyl mit 1, 2 oder 3 gleichen oder verschiedenen Ringheteroatomen ausgewählt aus Stickstoff, Sauerstoff und Schwefel, (C₆-C₁₄)-Aryl, fünfgliedriges bis zehngliedriges Heteroaryl mit 1, 2 oder 3 gleichen oder verschiedenen Ringheteroatomen ausgewählt aus Stickstoff, Sauerstoff und Schwefel, (C₁-C₈)-Alkoxy, (C₃-C₁₀)-Cycloalkoxy, (C₆-C₁₄)-Aryloxy, fünfgliedriges bis zehngliedriges Heteroaryloxy mit 1, 2 oder 3 gleichen oder verschiedenen Ringheteroatomen ausgewählt aus Stickstoff, Sauerstoff und Schwefel, Di((C₁-C₈)-alkyl)amino, Di((C₃-C₁₀)-cycloalkyl)amino oder Di((C₆-C₁₄)-aryl)amino steht;
oder deren Salz.

## Revendications

1. Procédé pour la préparation d'un composé de formule I, 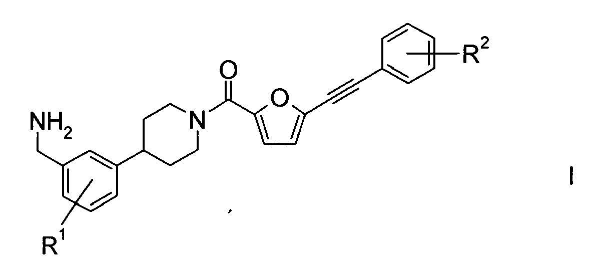
dans laquelle R¹ est H, F, CF₃, OCF₃, un alkyle en C₁-C₈, un cycloalkyle en C₃-C₁₀, un hétérocycloalkyle de 3 chaînons à 10 chaînons comprenant 1, 2 ou 3 hétéroatomes cycliques identiques ou différents choisis parmi l'azote, l'oxygène et le soufre, un aryle en C₆-C₁₄, un alcoxy en C₁-C₈, un cycloalcoxy en C₃-C₁₀, un aryloxy en C₆-C₁₄, un di(alkyle en C₁-C₈)amino, un di(cycloalkyle en C₃-C₁₀)amino ou un di(aryle en C₆-C₁₄)amino ; et
R² est H, F, CF₃, OCF₃, un alkyle en C₁-C₈, un cycloalkyle en C₃-C₁₀, un hétérocycloalkyle de 3 chaînons à 10 chaînons comprenant 1, 2 ou 3 hétéroatomes cycliques identiques ou différents choisis parmi l'azote, l'oxygène et le soufre, un aryle en C₆-C₁₄, un hétéroaryle de 5 chaînons à 10 chaînons comprenant 1, 2 ou 3 hétéroatomes cycliques identiques ou différents choisis parmi l'azote, l'oxygène et le soufre, un alcoxy en C₁-C₈, un cycloalcoxy en C₃-C₁₀, un aryloxy en C₆-C₁₄, un hétéroaryloxy de 5 chaînons à 10 chaînons comprenant 1, 2 ou 3 hétéroatomes cycliques identiques ou différents choisis parmi l'azote, l'oxygène et le soufre, un di(alkyle en C₁-C₈)amino, un di(cycloalkyle en C₃-C₁₀)amino ou un di(aryle en C₆-C₁₄)amino ;
ou son sel,
comprenant
a) le traitement d'un composé phényléthynylsilane de formule II, 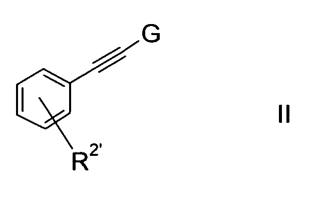 dans laquelle G est un triméthylsilyle, un triéthylsilyle, un triisopropylsilyle, ou un diméthyl-tert-butylsilyle, et R^{2'} est R² comme défini ci-dessus ou est un dérivé protégé de celui-ci ou un groupe précurseur de celui-ci, dans un solvant avec un hydroxyde, carbonate ou alcoolate de métal alcalin ou un alcoolate de métal alcalino-terreux, pour former une solution d'un phényléthyne de formule III ; 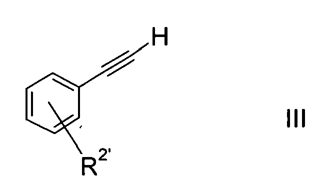 et
b) la combinaison de la solution résultante du phényléthyne avec un composé de formule IV, 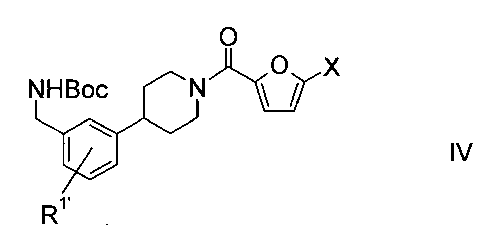 dans laquelle X est un bromo ou un iodo, R^{1'} est R¹ comme défini ci-dessus ou est un dérivé protégé de celui-ci ou un groupe précurseur de celui-ci, et Boc est le tert-butoxycarbonyle, en présence de catalyseur au palladium homogène, d'un sel cuivreux, et d'une amine encombrée dans un solvant pour former un composé protégé de formule V, 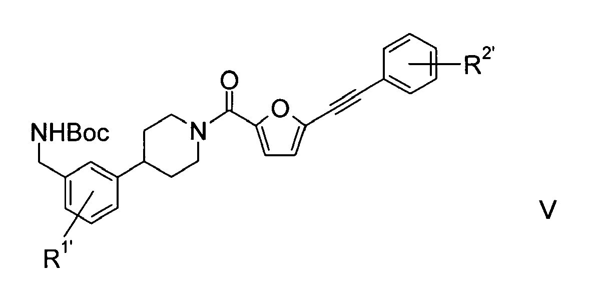
dans laquelle R^{1'} et R^{2'} sont R¹ et R², respectivement, comme défini ci-dessus ou sont un dérivé protégé de ceux-ci ou un groupe précurseur de ceux-ci, et Boc est le tert-butoxycarbonyle, ou son sel.

2. Procédé selon la revendication 1 pour la préparation de 4-[3-(aminométhyl)phényl]-1-[5-(2-fluorophényléthynyl)-2-furanoyl]pipéridine de formule la, 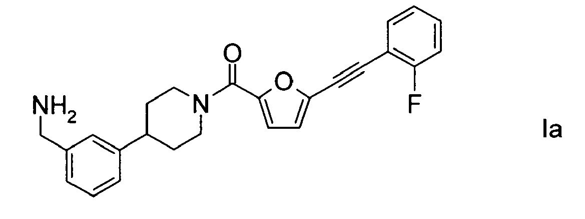 ou son sel, comprenant
a) le traitement d'un composé (2-fluorophényléthynyl)silyle de formule IIa, 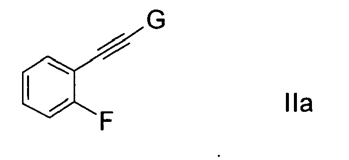 dans laquelle G est un triméthylsilyle, un triéthylsilyle, un triisopropylsilyle, ou un diméthyl-tert-butylsilyle, dans un solvant avec un hydroxyde, carbonate ou alcoolate de métal alcalin ou un alcoolate de métal alcalino-terreux, pour former une solution de 2-fluorophényléthyne ; et
b) la combinaison de la solution résultante de 2-fluorophényléthyne avec un composé de formule IVa, 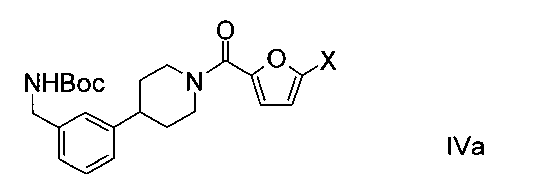 dans laquelle X est un bromo ou un iodo, et Boc est le tert-butoxycarbonyle, en présence de catalyseur au palladium homogène, d'un sel cuivreux et d'une amine encombrée dans un solvant pour former la 4-[3-(tert-butoxycarbonylaminométhyl)phényl]-1-[5-(2-fluorophényléthynyl)-2-furanoyl]pipéridine de formule Va, 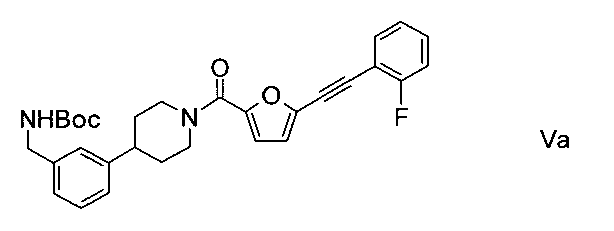
dans laquelle Boc est le tert-butoxycarbonyle.

3. Procédé selon la revendication 1 ou 2 **caractérisé en ce que** G est un triméthylsilyle.

4. Procédé selon l'une quelconque des revendications 1 à 3 **caractérisé en ce que** X est un bromo.

5. Procédé selon l'une quelconque des revendications 1 à 4, comprenant en outre l'élimination du groupe tert-butoxycarbonyle et d'autres groupes protecteurs et/ou la conversion des groupes précurseurs présents dans un composé de formule V ou Va pour obtenir un composé de formule I ou la ou son sel.

6. Procédé selon l'une quelconque des revendications 1 à 5, comprenant en outre l'élimination du groupe tert-butoxycarbonyle d'un composé de formule V ou Va en présence d'un acide pour obtenir un composé de formule I ou la ou son sel.

7. Procédé selon l'une quelconque des revendications 1 à 6, comprenant en outre l'élimination du groupe tert-butoxycarbonyle de la 4-[3-(tert-butoxycarbonylaminométhyl)phényl]-1-[5-(2-fluorophényléthynyl)-2-furanoyl]pipéridine en présence d'acide méthanesulfonique pour obtenir le sel d'acide méthanesulfonique de 4-[3-(aminométhyl)phényl]-1-[5-(2-fluorophényléthynyl)-2-furanoyl]pipérid ine.

8. Procédé pour la préparation d'un composé de formule IV, 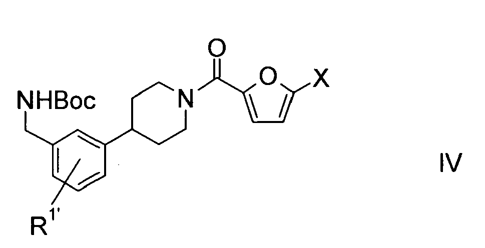
dans laquelle R^{1'} est H, F, CF₃, OCF₃, un alkyle en C₁-C₈, un cycloalkyle en C₃-C₁₀, un hétérocycloalkyle de 3 chaînons à 10 chaînons comprenant 1, 2 ou 3 hétéroatomes cycliques identiques ou différents choisis parmi l'azote, l'oxygène et le soufre, un aryle en C₆-C₁₄, un alcoxy en C₁-C₈, un cycloalcoxy en C₃-C₁₀, un aryloxy en C₆-C₁₄, un di(alkyle en C₁-C₈)amino, un di(cycloalkyle en C₃-C₁₀)amino ou un di(aryle en C₆-C₁₄)amino, ou un dérivé protégé de ceux-ci ou un groupe précurseur de ceux-ci ; et
X est un bromo ou un iodo,
comprenant
a) l'activation de l'acide 5-bromo-2-furoïque ou l'acide 5-iodo-2-furoïque pour obtenir une forme activée d'acide 5-bromo-2-furoïque ou d'acide 5-iodo-2-furoïque ; et
b) la combinaison de la forme activée d'acide 5-bromo-2-furoïque ou d'acide 5-iodo-2-furoïque avec un composé de formule VI, 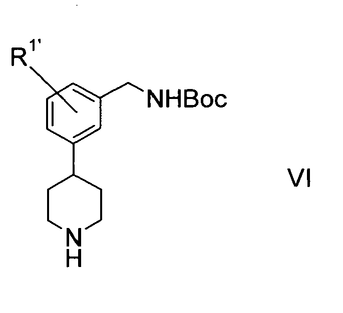
dans laquelle R^{1'} est comme défini pour la formule IV et Boc est le tert-butoxycarbonyle, dans un solvant.

9. Procédé selon la revendication 8 **caractérisé en ce que** R^{1'} est un hydrogène.

10. Procédé selon la revendication 8 ou 9 pour la préparation du composé de formule IVc, 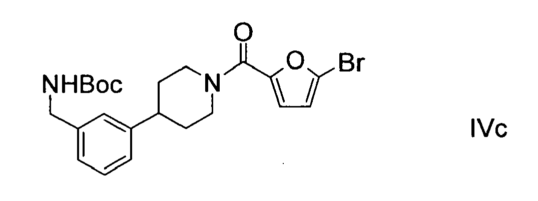 dans laquelle Boc est le tert-butoxycarbonyle, comprenant
a) le traitement de l'acide 5-bromo-2-furoïque avec le chlorure de thionyle pour obtenir le chlorure de 5-bromo-2-furoyle, et
b) la combinaison du chlorure de 5-bromo-2-furoyle avec le composé de formule VIa 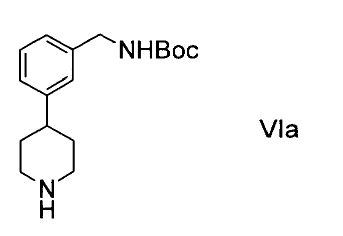
dans laquelle Boc est le tert-butoxycarbonyle, dans un solvant en présence d'une base.

11. Procédé pour la préparation d'un composé de formule VI 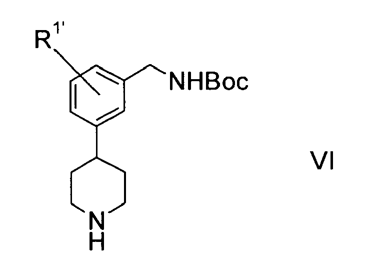
dans laquelle R^{1'} est H, F, CF₃, OCF₃, un alkyle en C₁-C₈, un cycloalkyle en C₃-C₁₀, un hétérocycloalkyle de 3 chaînons à 10 chaînons comprenant 1, 2 ou 3 hétéroatomes cycliques identiques ou différents choisis parmi l'azote, l'oxygène et le soufre, un aryle en C₆-C₁₄, un alcoxy en C₁-C₈, un cycloalcoxy en C₃-C₁₀, un aryloxy en C₆-C₁₄, un di(alkyle en C₁-C₈)amino, un di(cycloalkyle en C₃-C₁₀)amino ou un di(aryle en C₆-C₁₄)amino, ou un dérivé protégé de ceux-ci ou un groupe précurseur de ceux-ci ; et
Boc est le tert-butoxycarbonyle,
ou son sel,
comprenant
a) le traitement d'un sel d'une 3-halogénobenzylamine de formule VII, 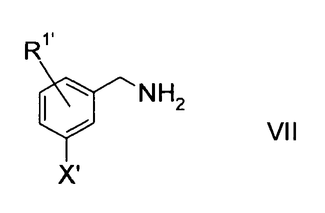 avec le 1,2-bis(chlorodiméthylsilyl)éthane dans un solvant en présence d'une base pour obtenir un 1-(3-halogénobenzyl)-2,2,5,5-tétraméthyl-1-aza-2,5-disilacyclopentane de formule VIII ; 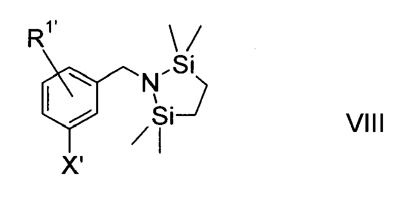
b) le traitement du 1-(3-halogénobenzyl)-2,2,5,5-tétraméthyl-1-aza-2,5-disilacyclopentane de formule VIII dans un solvant avec un composé alkyl-lithium et la 1-benzyl-4-pipéridone à une température d'environ -80 °C à environ -40 °C pour obtenir un alcool de formule IX ; 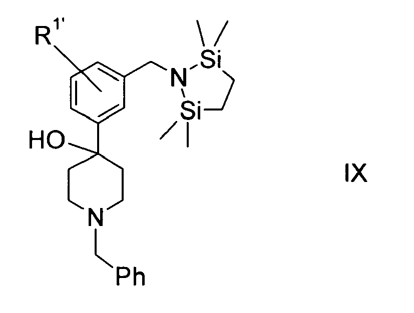
c) le traitement de l'alcool de formule IX avec un acide dans un solvant pour obtenir une hydroxypipéridinylbenzylamine de formule X 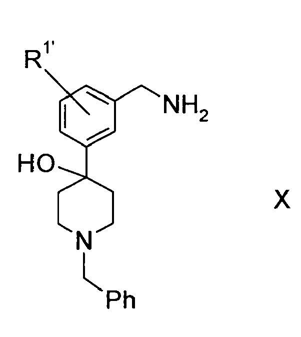 sous la forme du sel de l'acide ;
d) le traitement de l'hydroxypipéridinylbenzylamine de formule X ou son sel avec un acide concentré, non oxydant à une température d'environ 70 °C à environ 150 °C, suivi par une alcalinisation pour obtenir une oléfine de formule XI ; 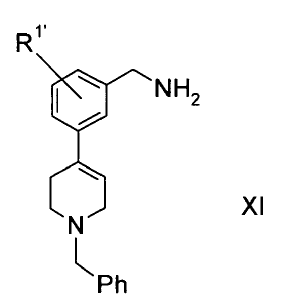
e) le traitement de l'oléfine de formule XI avec le dicarbonate de di-tert-butyle dans un solvant en présence d'une base pour obtenir une amine protégée de formule XII 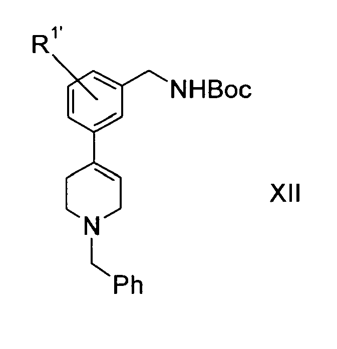
dans laquelle Boc est le tert-butoxycarbonyle, et où Ph dans les formules IX, X, XI et XII est un phényle, X' dans les formules VII et VIII est un bromo ou un iodo, et R^{1'} dans les formules VII, VIII, IX, X, XI et XII est comme défini pour la formule VI.

12. Procédé selon la revendication 11 pour la préparation de 4-[3-(tert-butoxycarbonylaminométhyl)phényl]pipéridine ou son sel,
comprenant
a) le traitement du chlorhydrate de 3-bromobenzylamine ou du chlorhydrate de 3-iodobenzylamine avec le 1,2-bis(chlorodiméthylsilyl)éthane dans un hydrocarbure aliphatique halogéné en présence d'une amine tertiaire pour obtenir le 1-(3-halogénobenzyl)-2,2,5,5-tétraméthyl-1-aza-2,5-disilacyclopentane correspondant, où l'halogéno est un bromo ou un iodo ;
b) le traitement du 1-(3-halogénobenzyl)-2,2,5,5-tétraméthyl-1-aza-2,5-disilacyclopentane dans un éther avec un composé alkyl-lithium et la 1-benzyl-4-pipéridone à une température d'environ -80 °C à environ -40 °C pour obtenir le 1-[3-(1-benzyl-4-hydroxypipéridin-4-yl)benzyl]-2,2,5,5-tétraméthyl-1-aza-2,5-disilacyclopentane ;
c) le traitement du 1-[3-(1-benzyl-4-hydroxypipéridin-4-yl)benzyl]-2,2,5,5-tétraméthyl-1-aza-2,5-disilacyclopentane avec un acide inorganique dans un hydrocarbure halogéné pour obtenir la 3-(1-benzyl-4-hydroxypipéridin-4-yl)benzylamine sous forme de sel de l'acide inorganique ;
d) le-traitement de la 3-(1-benzyl-4-hydroxypipéridin-4-yl)benzylamine avec un acide concentré, non oxydant à une température d'environ 70 °C à environ 150 °C, suivi par une alcalinisation pour obtenir la 3-(1-benzyl-1,2,3,6-tétrahydropyridin-4-yl)benzylamine ; et
e) le traitement de la 3-(1-benzyl-1,2,3,6-tétrahydropyridin-4-yl)benzylamine avec le dicarbonate de di-tert-butyle dans un alcool aliphatique, l'acétate d'éthyle, un éther, un hydrocarbure halogéné, un mélange de deux ou plus de tels solvants ou un mélange d'un ou plusieurs de tels solvants avec l'eau, en présence d'un hydroxyde, carbonate ou alcoolate de métal alcalin ou d'un amine tertiaire pour obtenir l'ester tert-butylique d'acide 3-(1-benzyl-1,2,3,6-tétrahydropyridin-4-yl)benzylcarbamique.

13. Procédé selon les revendications 11 ou 12, comprenant en outre le traitement de l'amine de formule XII avec l'hydrogène dans un solvant en présence d'un catalyseur au palladium et en présence d'un acide pour obtenir un composé de formule VI ou son sel.

14. Procédé pour la préparation d'un composé de formule VI, 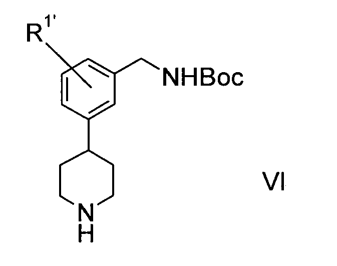 dans laquelle R^{1'} est H, F, CF₃, OCF₃, un alkyle en C₁-C₈, un cycloalkyle en C₃-C₁₀, un hétérocycloalkyle de 3 chaînons à 10 chaînons comprenant 1, 2 ou 3 hétéroatomes cycliques identiques ou différents choisis parmi l'azote, l'oxygène et le soufre, un aryle en C₆-C₁₄, un alcoxy en C₁-C₈, un cycloalcoxy en C₃-C₁₀, un aryloxy en C₆-C₁₄, un di(alkyle en C₁-C₈)amino, un di(cycloalkyle en C₃-C₁₀)amino ou un di(aryle en C₆-C₁₄)amino, ou un dérivé protégé de ceux-ci ou un groupe précurseur de ceux-ci ; et Boc est le tert-butoxycarbonyle,
ou son sel,
comprenant
a) le traitement d'un 3-(4-pyridyl)benzaldéhyde de formule XIII, 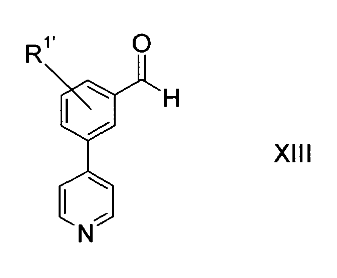 avec l'hydroxylamine ou un sel d'hydroxylamine dans un solvant pour obtenir un oxime de formule XIV ou son sel ; 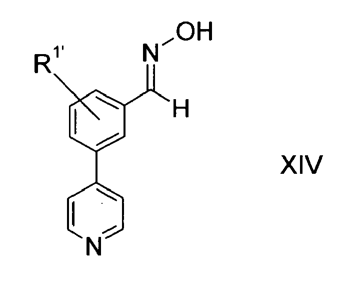
b) le traitement de l'oxime de formule XIV ou son sel avec l'hydrogène dans un solvant en présence d'un catalyseur au palladium pour obtenir une 3-(4-pyridyl)benzylamine de formule XV ou son sel ; 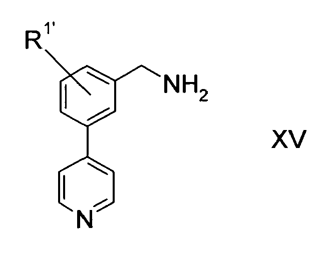
c) le traitement de la 3-(4-pyridyl)benzylamine de formule XV ou son sel avec le dicarbonate de di-tert-butyle dans un solvant en présence d'une base pour obtenir une 3-(4-pyridyl)benzylamine protégée par Boc de formule XVI, 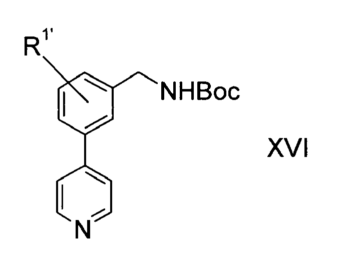 dans laquelle Boc est le tert-butoxycarbonyle ; et
d) le traitement de la 3-(4-pyridyl)benzylamine protégée par Boc de formule XVI avec l'hydrogène dans un solvant en présence d'un catalyseur au platine et en présence d'un acide pour obtenir un composé de formule VI ou son sel ;
où R^{1'} dans les formules XIII, XIV, XV et XVI est comme défini ci-dessus pour la formule VI.

15. Procédé selon la revendication 14 pour la préparation de la 4-[3-(tert-butoxycarbonylaminométhyl)phényl]pipéridine ou son sel, comprenant
a) le traitement de 3-(4-pyridyl)benzaldéhyde avec l'hydroxylamine ou un sel de celle-ci dans un alcool aliphatique à une température d'environ 0 °C à environ 40 °C pour obtenir l'oxime de 3-(4-pyridyl)benzaldéhyde ou son sel ;
b) le traitement de l'oxime de 3-(4-pyridyl)benzaldéhyde ou son sel avec l'hydrogène à une pression d'environ 300 kPa à environ 1500 kPa dans un alcool aliphatique en présence d'un catalyseur au palladium à une température d'environ 20 °C à environ 50 °C pour obtenir la 3-(4-pyridyl)benzylamine ou son sel ;
c) le traitement de la 3-(4-pyridyl)benzylamine ou son sel avec le dicarbonate de di-tert-butyle dans un alcool aliphatique, l'acétate d'éthyle, un éther, un hydrocarbure halogéné, un mélange de deux ou plus de tels solvants ou un mélange d'un ou plusieurs de tels solvants avec l'eau, en présence d'un hydroxyde, carbonate ou alcoolate de métal alcalin ou d'une amine tertiaire pour obtenir l'ester tert-butylique d'acide 3-(4-pyridyl)benzylcarbamique ; et
d) le traitement de l'ester tert-butylique d'acide 3-(4-pyridyl)benzylcarbamique avec l'hydrogène dans un alcool aliphatique à une pression d'environ 2000 kPa à environ 6000 kPa en présence d'un catalyseur au platine et en présence d'un acide.

16. Procédé pour la préparation d'un composé de formule VI 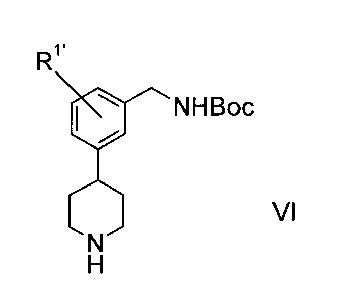
dans laquelle R^{1'} est H, F, CF₃, OCF₃, un alkyle en C₁-C₈, un cycloalkyle en C₃-C₁₀, un hétérocycloalkyle de 3 chaînons à 10 chaînons comprenant 1, 2 ou 3 hétéroatomes cycliques identiques ou différents choisis parmi l'azote, l'oxygène et le soufre, un aryle en C₆-C₁₄, un alcoxy en C₁-C₈, un cycloalcoxy en C₃-C₁₀, un aryloxy en C₆-C₁₄, un di(alkyle en C₁-C₈)amino, un di(cycloalkyle en C₃-C₁₀)amino ou un di(aryle en C₆-C₁₄)amino, ou un dérivé protégé de ceux-ci ou un groupe précurseur de ceux-ci ; et
Boc est le tert-butoxycarbonyle,
ou son sel,
comprenant
a) le traitement d'un 3-(4-pyridyl)benzonitrile de formule XVII, 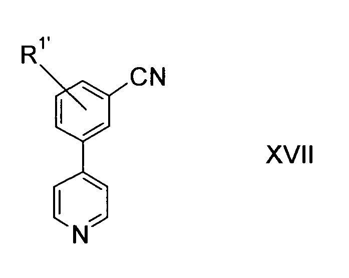 avec l'hydrogène en présence d'un catalyseur au palladium et en présence d'un acide, ou avec un hydrure complexe, dans un solvant pour obtenir une 3-(4-pyridyl)benzylamine de formule XV ou son sel ; 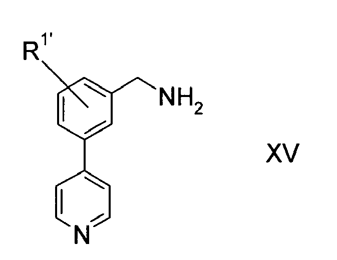
b) le traitement de la 3-(4-pyridyl)benzylamine de formule XV ou son sel avec le dicarbonate de di-tert-butyle dans un solvant en présence d'une base pour obtenir une 3-(4-pyridyl)benzylamine protégée par Boc de formule XVI, 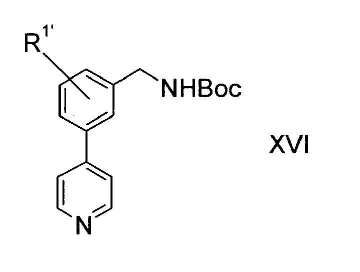 dans laquelle Boc est le tert-butoxycarbonyle ; et
c) le traitement de la 3-(4-pyridyl)benzylamine protégée par Boc de formule XVI avec l'hydrogène dans un solvant en présence d'un catalyseur au platine et en présence d'un acide pour obtenir un composé de formule VI ou son sel ;
où R^{1'} dans les formules XV, XVI et XVII est comme défini ci-dessus pour la formule VI.

17. Procédé selon la revendication 16 pour la préparation de 4-[3-(tert-butoxycarbonylaminométhyl)phényl]pipéridine ou son sel, comprenant
a) le traitement du 3-(4-pyridyl)benzonitrile avec l'hydrogène en présence d'un catalyseur au palladium à une pression d'environ 2000 kPa à environ 6000 kPa dans un alcool aliphatique ou un mélange d'un alcool aliphatique et d'eau, ou avec un hydrure complexe dans un alcool aliphatique ou un éther, à une température d'environ 0 °C à environ 50 °C, pour obtenir la 3-(4-pyridyl)benzylamine ou son sel ;
b) le traitement de la 3-(4-pyridyl)benzylamine ou son sel avec le dicarbonate de di-tert-butyle dans un alcool aliphatique, l'acétate d'éthyle, un éther, un hydrocarbure halogéné, un mélange de deux ou plus de tels solvants ou un mélange d'un ou plusieurs de tels solvants avec l'eau, en présence d'un hydroxyde, carbonate ou alcoolate de métal alcalin ou d'une amine tertiaire, pour obtenir l'ester tert-butylique d'acide 3-(4-pyridyl)benzylcarbamique ; et
c) le traitement de l'ester tert-butylique d'acide 3-(4-pyridyl)benzylcarbamique avec l'hydrogène dans un alcool aliphatique à une pression d'environ 2000 kPa à environ 6000 kPa en présence d'un catalyseur au platine et en présence d'un acide.

18. Composé de formule VIII, 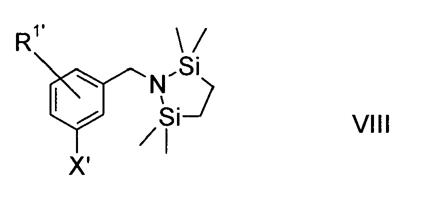 dans laquelle R^{1'} est H, F, CF₃, OCF₃, un alkyle en C₁-C₈, un cycloalkyle en C₃-C₁₀, un hétérocycloalkyle de 3 chaînons à 10 chaînons comprenant 1, 2 ou 3 hétéroatomes cycliques identiques ou différents choisis parmi l'azote, l'oxygène et le soufre, un aryle en C₆-C₁₄, un alcoxy en C₁-C₈, un cycloalcoxy en C₃-C₁₀, un aryloxy en C₆-C₁₄, un di(alkyle en C₁-C₈)amino, un di(cycloalkyle en C₃-C₁₀)amino ou un di(aryle en C₆-C₁₄)amino, ou un dérivé protégé de ceux-ci ou un groupe précurseur de ceux-ci ; et X' est un bromo ou un iodo ; le composé 1-(3-bromobenzyl)-2,2,5,5-tétraméthyl-1-aza-2,5-disilacyclopentane étant exclu.

19. Composé selon la revendication 18 qui est le 1-(3-iodobenzyl)-2,2,5,5-tétraméthyl-1-aza-2,5-disilacyclopentane.

20. Composé de formule IX, 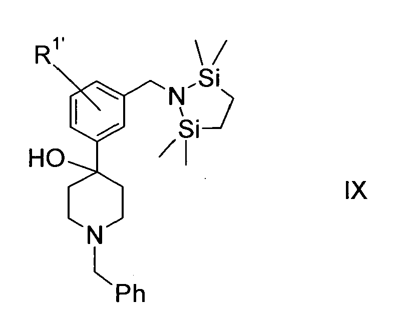 dans laquelle R^{1'} est H, F, CF₃, OCF₃, un alkyle en C₁-C₈, un cycloalkyle en C₃-C₁₀, un hétérocycloalkyle de 3 chaînons à 10 chaînons comprenant 1, 2 ou 3 hétéroatomes cycliques identiques ou différents choisis parmi l'azote, l'oxygène et le soufre, un aryle en C₆-C₁₄, un alcoxy en C₁-C₈, un cycloalcoxy en C₃-C₁₀, un aryloxy en C₆-C₁₄, un di(alkyle en C₁-C₈)amino, un di(cycloalkyle en C₃-C₁₀)amino ou un di(aryle en C₆-C₁₄)amino, ou un dérivé protégé de ceux-ci ou un groupe précurseur de ceux-ci ; et Ph est le phényle.

21. Composé selon la revendication 20 qui est le 1-[3-(1-benzyl-4-hydroxypipéridin-4-yl)benzyl]-2,2,5,5-tétraméthyl-1-aza-2,5-disilacyclopentane.

22. Composé de formule X, 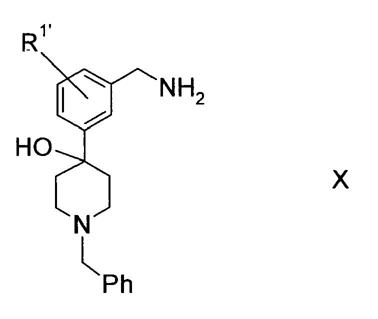 dans laquelle R^{1'} est H, F, CF₃, OCF₃, un alkyle en C₁-C₈, un cycloalkyle en C₃-C₁₀, un hétérocycloalkyle de 3 chaînons à 10 chaînons comprenant 1, 2 ou 3 hétéroatomes cycliques identiques ou différents choisis parmi l'azote, l'oxygène et le soufre, un aryle en C₆-C₁₄, un alcoxy en C₁-C₈, un cycloalcoxy en C₃-C₁₀, un aryloxy en C₆-C₁₄, un di(alkyle en C₁-C₈)amino, un di(cycloalkyle en C₃-C₁₀)amino ou un di(aryle en C₆-C₁₄)amino, ou un dérivé protégé de ceux-ci ou un groupe précurseur de ceux-ci ; et Ph est le phényle, ou son sel.

23. Composé selon la revendication 22 qui est la 3-(1-benzyl-4-hydroxypipéridin-4-yl)benzylamine ou son sel.

24. Composé de formule XI, 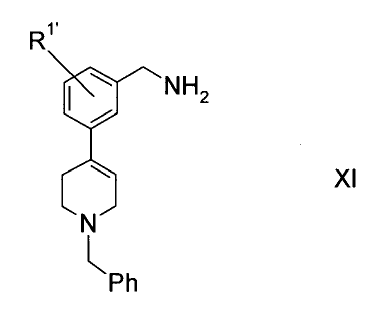 dans laquelle R^{1'} est H, F, CF₃, OCF₃, un alkyle en C₁-C₈, un cycloalkyle en C₃-C₁₀, un hétérocycloalkyle de 3 chaînons à 10 chaînons comprenant 1, 2 ou 3 hétéroatomes cycliques identiques ou différents choisis parmi l'azote, l'oxygène et le soufre, un aryle en C₆-C₁₄, un alcoxy en C₁-C₈, un cycloalcoxy en C₃-C₁₀, un aryloxy en C₆-C₁₄, un di(alkyle en C₁-C₈)amino, un di(cycloalkyle en C₃-C₁₀)amino ou un di(aryle en C₆-C₁₄)amino, ou un dérivé protégé de ceux-ci ou un groupe précurseur de ceux-ci ; et Ph est le phényle, ou son sel.

25. Composé selon la revendication 24 qui est la 3-(1-benzyl-1,2,3,6-tétrahydropyridin-4-yl)benzylamine ou son sel.

26. Composé de formule XII, 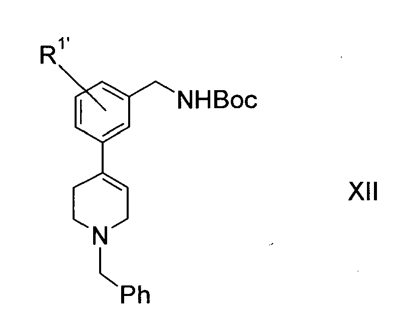 dans laquelle R^{1'} est H, F, CF₃, OCF₃, un alkyle en C₁-C₈, un cycloalkyle en C₃-C₁₀, un hétérocycloalkyle de 3 chaînons à 10 chaînons comprenant 1, 2 ou 3 hétéroatomes cycliques identiques ou différents choisis parmi l'azote, l'oxygène et le soufre, un aryle en C₆-C₁₄, un alcoxy en C₁-C₈, un cycloalcoxy en C₃-C₁₀, un aryloxy en C₆-C₁₄, un di(alkyle en C₁-C₈)amino, un di(cycloalkyle en C₃-C₁₀)amino ou un di(aryle en C₆-C₁₄)amino, ou un dérivé protégé de ceux-ci ou un groupe précurseur de ceux-ci ; Boc est le tert-butoxycarbonyle ; et Ph est le phényle, ou son sel.

27. Composé selon la revendication 26 qui est l'ester tert-butylique d'acide 3-(1-benzyl-1,2,3,6-tétrahydropyridin-4-yl)benzylcarbamique ou son sel.

28. Composé de formule XIV, 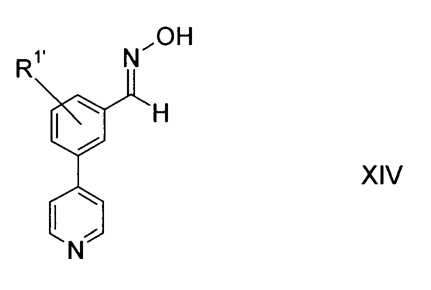 dans laquelle R^{1'} est H, F, CF₃, OCF₃, un alkyle en C₁-C₈, un cycloalkyle en C₃-C₁₀, un hétérocycloalkyle de 3 chaînons à 10 chaînons comprenant 1, 2 ou 3 hétéroatomes cycliques identiques ou différents choisis parmi l'azote, l'oxygène et le soufre, un aryle en C₆-C₁₄, un alcoxy en C₁-C₈, un cycloalcoxy en C₃-C₁₀, un aryloxy en C₆-C₁₄, un di(alkyle en C₁-C₈)amino, un di(cycloalkyle en C₃-C₁₀)amino ou un di(aryle en C₆-C₁₄)amino, ou un dérivé protégé de ceux-ci ou un groupe précurseur de ceux-ci, ou son sel.

29. Composé selon la revendication 28 qui est l'oxime de 3-(4-pyridyl)benzaldéhyde ou son sel.

30. Composé de formule XV, 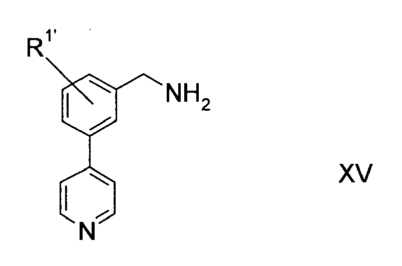 dans laquelle R^{1'} est H, F, CF₃, OCF₃, un alkyle en C₁-C₈, un cycloalkyle en C₃-C₁₀, un hétérocycloalkyle de 3 chaînons à 10 chaînons comprenant 1, 2 ou 3 hétéroatomes cycliques identiques ou différents choisis parmi l'azote, l'oxygène et le soufre, un aryle en C₆-C₁₄, un alcoxy en C₁-C₈, un cycloalcoxy en C₃-C₁₀, un aryloxy en C₆-C₁₄, un di(alkyle en C₁-C₈)amino, un di(cycloalkyle en C₃-C₁₀)amino ou un di(aryle en C₆-C₁₄)amino, ou un dérivé protégé de ceux-ci ou un groupe précurseur de ceux-ci, ou son sel.

31. Composé selon la revendication 30 qui est la 3-(4-pyridyl)benzylamine ou son sel.

32. Composé de formule XVI, 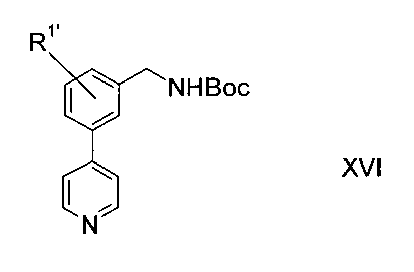 dans laquelle R^{1'} est H, F, CF₃, OCF₃, un alkyle en C₁-C₈, un cycloalkyle en C₃-C₁₀, un hétérocycloalkyle de 3 chaînons à 10 chaînons comprenant 1, 2 ou 3 hétéroatomes cycliques identiques ou différents choisis parmi l'azote, l'oxygène et le soufre, un aryle en C₆-C₁₄, un alcoxy en C₁-C₈, un cycloalcoxy en C₃-C₁₀, un aryloxy en C₆-C₁₄, un di(alkyle en C₁-C₈)amino, un di(cycloalkyle en C₃-C₁₀)amino ou un di(aryle en C₆-C₁₄)amino, ou un dérivé protégé de ceux-ci ou un groupe précurseur de ceux-ci ; et Boc est le tert-butoxycarbonyle, ou son sel.

33. Composé selon la revendication 32 qui est l'ester tert-butylique d'acide 3-(4-pyridyl)benzylcarbamique ou son sel.

34. Composé de formule IVb, 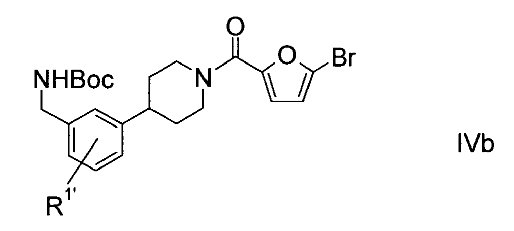
dans laquelle R^{1'} est H, F, CF₃, OCF₃, un alkyle en C₁-C₈, un cycloalkyle en C₃-C₁₀, un hétérocycloalkyle de 3 chaînons à 10 chaînons comprenant 1, 2 ou 3 hétéroatomes cycliques identiques ou différents choisis parmi l'azote, l'oxygène et le soufre, un aryle en C₆-C₁₄, un alcoxy en C₁-C₈, un cycloalcoxy en C₃-C₁₀, un aryloxy en C₆-C₁₄, un di(alkyle en C₁-C₈)amino, un di(cycloalkyle en C₃-C₁₀)amino ou un di(aryle en C₆-C₁₄)amino, ou un dérivé protégé de ceux-ci ou un groupe précurseur de ceux-ci ;
et Boc est le tert-butoxycarbonyle,
ou son sel.

35. Composé selon la revendication 34 qui est la 4-[3-(tert-butoxycarbonylaminométhyl)phényl]-1-[5-bromo-2-furanoyl]pipéridine.

36. Utilisation d'un composé selon l'une quelconque des revendications 18 à 35 en tant qu'intermédiaire pour la préparation d'un composé de formule I, 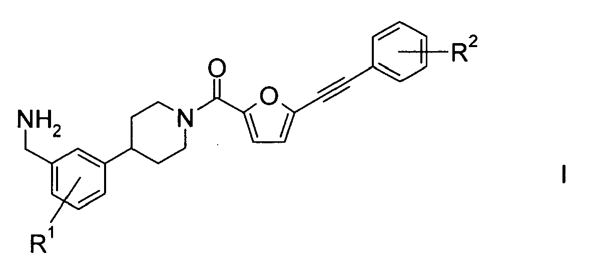
dans laquelle R¹ est H, F, CF₃, OCF₃, un alkyle en C₁-C₈, un cycloalkyle en C₃-C₁₀, un hétérocycloalkyle de 3 chaînons à 10 chaînons comprenant 1, 2 ou 3 hétéroatomes cycliques identiques ou différents choisis parmi l'azote, l'oxygène et le soufre, un aryle en C₆-C₁₄, un alcoxy en C₁-C₈, un cycloalcoxy en C₃-C₁₀, un aryloxy en C₆-C₁₄, un di(alkyle en C₁-C₈)amino, un di(cycloalkyle en C₃-C₁₀)amino ou un di(aryle en C₆-C₁₄)amino ; et
R² est H, F, CF₃, OCF₃, un alkyle en C₁-C₈, un cycloalkyle en C₃-C₁₀, un hétérocycloalkyle de 3 chaînons à 10 chaînons comprenant 1, 2 ou 3 hétéroatomes cycliques identiques ou différents choisis parmi l'azote, l'oxygène et le soufre, un aryle en C₆-C₁₄, un hétéroaryle de 5 chaînons à 10 chaînons comprenant 1, 2 ou 3 hétéroatomes cycliques identiques ou différents choisis parmi l'azote, l'oxygène et le soufre, un alcoxy en C₁-C₈, un cycloalcoxy en C₃-C₁₀, un aryloxy en C₆-C₁₄, un hétéroaryloxy de 5 chaînons à 10 chaînons comprenant 1, 2 ou 3 hétéroatomes cycliques identiques ou différents choisis parmi l'azote, l'oxygène et le soufre, un di(alkyle en C₁-C₈)amino, un di(cycloalkyle en C₃-C₁₀)amino ou un di(aryle en C₆-C₁₄)amino ; ou son sel.
